# EUROPÄISCHE PATENTSCHRIFT

(11) **EP 4 629 846 B1**
(45) Veröffentlichungstag und Bekanntmachung des Hinweises auf die Patenterteilung: **08.07.2026**
(21) Anmeldenummer: 23710775.0
(22) Anmeldetag: 15.03.2023
(51) Int. Cl.: A23L 33/125, A23L 33/115, A61K 8/02, A61K 8/37, A61K 8/73, A61Q 19/00, C08B 37/00

(54) **PULVERFÖRMIGE GETRÄGERTE MKT-ZUSAMMENSETZUNGEN UND DEREN VERWENDUNGEN**
POWDERY SUPPORTED MCT-COMPOSITIONS AND THEIR USE
COMPOSITIONS DE TCM SUPPORTÉES EN POUDRE ET LEUR UTILISATION

(30) Priorität: 24.02.2023 WO PCT/EP2023/054759; 09.03.2023 WO PCT/EP2023/055982
(43) Veröffentlichungstag der Anmeldung: 15.10.2025
(73) Patentinhaber: IOI Oleo GmbH, 20459 Hamburg (DE)
(72) Erfinder: REYER, Sebastian, 58453 Witten (DE); STEPAN, Sven, 58453 Witten (DE)
(74) Vertreter: Strehlke, Ingo Kurt
(86) Internationale Anmeldenummer: PCT/EP2023/056610
(87) Internationale Veröffentlichungsnummer: WO 2024/175213

(56) Entgegenhaltungen:
- EP-A1- 1 666 502
- WO-A1-2017/017248
- WO-A1-2021/041005
- AL-ASSAF ET AL: "Characterization and properties of Acacia senegal (L.) Willd. var. senegal with enhanced properties (Acacia (sen) SUPER GUM(TM)): Part 1-Controlled maturation of Acacia senegal var. senegal to increase viscoelasticity, produce a hydrogel form and convert a poor into a good emulsifier", FOOD HYDROCOLLOIDS, ELSEVIER BV, NL, vol. 21, no. 3, 16 November 2006 (2006-11-16), pages 319 - 328, XP005770872, ISSN: 0268-005X, DOI: 10.1016/J.FOODHYD.2006.04.011

## Beschreibung

Die vorliegende Erfindung betrifft das technische Gebiet der mittelkettigen Triglyceride (synonym auch als MKTs bzw. MCTs bezeichnet) und insbesondere die Bereitstellung derartiger Triglyceride in speziellen Zusammensetzungen.

Insbesondere betrifft die vorliegende Erfindung eine Zusammensetzung, welche mittelkettige Triglyceride (MKTs) enthält und welche pulverförmig ausgebildet ist, insbesondere zur Verwendung in Lebensmittel- bzw. Nahrungsmittel-, kosmetischen oder pharmazeutischen Erzeugnissen, wobei die Zusammensetzung eine Vielzahl einzelner Partikel umfasst oder hieraus besteht und wobei die Partikel mindestens ein polysaccharidbasiertes Trägermaterial auf Basis von Gummi arabicum umfassen.

Weiterhin betrifft die vorliegende Erfindung auch diesbezügliche Nahrungsmittel-, kosmetische oder pharmazeutische Erzeugnisse, welche jeweils die Zusammensetzung nach der Erfindung enthalten.

Darüber hinaus betrifft die vorliegende Erfindung auch die Verwendung der erfindungsgemäßen Zusammensetzung in einem Nahrungsmittel-, kosmetischen oder pharmazeutischen Erzeugnis bzw. zur Herstellung eines Nahrungsmittel-, kosmetischen oder pharmazeutischen Erzeugnisses.

Des Weiteren betrifft die vorliegende Erfindung auch ein Verfahren zur Herstellung der Zusammensetzung nach der Erfindung.

Die vorliegende Erfindung betrifft gleichermaßen auch ein Verfahren zur Steuerung bzw. Einstellung der Beladung eines Gummi arabicum umfassenden oder hieraus gebildeten polysaccharidbasierten Trägermaterials mit mindestens einem mittelkettigen Triglycerid (MKT), und zwar insbesondere zur Herstellung einer mittelkettige Triglyceride (MKTs) enthaltenden partikulären bzw. pulverförmigen Zusammensetzung, wie erfindungsgemäß definiert.

Schließlich betrifft die vorliegende Erfindung auch die Verwendung des Glykoproteingehalts eines Gummi arabicum umfassenden bzw. hieraus gebildeten polysaccharidbasierten Trägermaterials zur Steuerung bzw. Einstellung der Beladung des polysaccharidbasierten Trägermaterials mit mindestens einem mittelkettigen Triglycerid (MKT).

Im Stand der Technik werden insbesondere im Bereich der Nahrungsmittel sowie auch im kosmetischen und pharmazeutischen Bereich für diesbezügliche Produkte bzw. Erzeugnisse oftmals Öle und Fettphasen bzw. Fette (z. B. Triglyceride oder dergleichen) eingesetzt, um die Produkte bzw. Erzeugnisse mit speziellen Eigenschaften auszustatten. Dies erfolgt zumeist auf Grundlage der physikalischen bzw. physikochemischen Eigenschaften der herangezogenen Substanzen sowie deren ernährungsphysiologischen bzw. metabolischen Relevanz, insbesondere im Fall von Nahrungsmitteln. Jedoch besteht oftmals die Problematik, dass sich Öle bzw. Fettphasen in ihrer reinen Form mitunter nicht zufriedenstellend in entsprechende Produkte bzw. Erzeugnisse einarbeiten lassen.

In diesem Zusammenhang ist es häufig problematisch, Öle bzw. Fettphasen in entsprechende Erzeugnissen bzw. Produkten effektiv zu inkorporieren, um hohe Gehalte einerseits und stabile bzw. homogene Emulsionen andererseits zu erhalten. Oftmals ist es nicht möglich, Öle bzw. Fettphasen homogen und stabil zu emulgieren. Insbesondere kann in der Folge auch eine Komponenten- bzw. Systementmischung oder dergleichen auftreten, einhergehend mit einer unzureichenden (Lagerungs-)Stabilität und letztlich eingeschränkten Produktqualität. Insofern ist es nicht in ausreichendem Maße möglich, entsprechende Produkteigenschaften gezielt vorzugeben bzw. dauerhaft beizubehalten. Hinzu kommt, dass oftmals auch eine geringere Bioverfügbarkeit der zugrundeliegenden Komponenten vorliegt.

Vor diesem Hintergrund besteht im Stand der Technik somit insgesamt ein großer Bedarf an der Bereitstellung diesbezüglicher Produkte bzw. Erzeugnisse, insbesondere in Form homogener und stabiler Dispersionen, insbesondere Emulsionen, bei welchen eine stabile, homogene und dauerhafte Inkorporation der Öle bzw. Fettphasen gegeben ist, wobei auch hohe Gehalte bzw. Konzentrationen an Ölen bzw. Fetten realisiert werden können.

Vor diesem Hintergrund werden im Stand der Technik Ansätze verfolgt, die in entsprechenden Produkten bzw. Erzeugnissen einzusetzenden Öle bzw. Fettphasen zu Zwecken einer verbesserten Handhabung und verbesserten Einarbeitung auf Träger bzw. Trägermaterialien aufzubringen, zu fixieren bzw. zu verkapseln. Derartige geträgerte bzw. verkapselte Systeme zeichnen sich im Vergleich zu den freien, insbesondere nicht geträgerten bzw. nicht verkapselten Ölen bzw. Fettphasen grundsätzlich durch eine bessere Handhabbarkeit und Verarbeitung auch in Bezug auf herzustellende Dispersionen bzw. Emulsionen aus.

Was die geträgerten bzw. verkapselten Öl- bzw. Fettphasensysteme anbelangt, so kommt hierzu im Allgemeinen eine Vielzahl einsetzbarer Öle bzw. Fettphasen sowie Substanzen zur Ausbildung von Trägersystemen bzw. Verkapselungen zum Einsatz.

Als Trägermaterial wird im Stand der Technik - neben zahlreichen anderen Materialien - auf polysaccharidbasierte Trägermaterialen zurückgegriffen, wobei in diesem Zusammenhang oftmals ein Fokus auf den Einsatz von modifizierter Stärke sowie Maltodextrin oder dergleichen liegt. Auf Basis derartiger Trägersysteme kann eine Fixierung bzw. Anlagerung entsprechender Öle bzw. Fettphasen jedoch oftmals nur bis zu einem gewissen Maß bzw. Grad erfolgen. Derartige Trägersysteme sind dabei oftmals teilchenförmig ausgebildet. Die so resultierenden Zusammensetzungen auf Basis der beladenen Trägersysteme weisen nicht immer optimale Beladungsgrade mit den Ölen bzw. Fettphasen auf. Zudem liegen oftmals nicht immer optimale Anwendungs- bzw. Verarbeitungseigenschaften derartiger Zusammensetzungen auf Basis von aus modifizierter Stärke bzw. Maltodextrin gebildeten Trägermaterialien vor. Zudem liegt nicht immer eine gute Stabilität vor, wobei oftmals auch die Fließfähigkeit bzw. Rieselfähigkeit weniger gut ausgebildet ist. Zudem können derartige Zusammensetzungen auch zur Verklumpung bzw. Makroagglomeratbildung neigen, und zwar insbesondere auch für den Fall höherer Beladungsgrade mit dem Öl bzw. der Fettphase, da mitunter eine gewisse Klebrigkeit der zugrundeliegenden partikulären Strukturen vorliegt.

Im Allgemeinen ist bei Trägersystemen des Standes der Technik auch die Lagerstabilität oftmals nicht zufriedenstellend. Darüber hinaus verhält es sich geträgerte bzw. verkapselte Systeme des Standes der Technik auch infolge der zuvor genannten Nachteile oftmals auch derart, dass eine optimale Einarbeitung bzw. Verarbeitung zu Produkten bzw. Erzeugnissen, beispielsweise Nahrungsmittel, kosmetischen sowie pharmazeutischen Produkten bzw. Erzeugnissen, unter dem Aspekt der Ausbildung stabiler und homogener Dispersionen, insbesondere stabiler und homogener Emulsionen, oftmals nicht gegeben ist. Dies führt dazu, dass für die resultierenden Produkte bzw. Erzeugnisse schlechtere Produkteigenschaften vorliegen, und zwar auch was deren Stabilität, Gehalte an Ölen bzw. Fetten, Bioverfügbarkeit der Inhaltsstoffe, Anwendungseigenschaften oder dergleichen anbelangt.

Darüber hinaus ist insbesondere für den Bereich der Nahrungsmittel bzw. entsprechender Produkte bzw. Erzeugnisse auch anzuführen, dass Trägermaterialien auf Basis von modifizierter Stärke bzw. Maltodextrin ernährungsphysiologisch nicht neutral sind und in diesem Zusammenhang auch eine eigene kalorische Einflussnahme aufweisen kann, was beispielsweise für diätische Nahrungsmittelprodukte problematisch bzw. unerwünscht ist. Auch sind die Löslichkeitseigenschaften bei Einbringen in eine Flüssigkeit, insbesondere Wasser, oftmals nicht optimal, was der Ausbildung stabiler bzw. homogener Emulsionen insgesamt abträglich ist.

Darüber hinaus kann grundsätzlich auch Gummi arabicum als polysaccharidbasierter Träger bzw. Trägermaterial eingesetzt werden. Insbesondere weist Gummi arabicum im Hinblick auf die Emulgierbarkeit Vorteile auf. Jedoch können bei herkömmlichen, im Stand der Technik eingesetzten Gummi arabicum im Allgemeinen nicht die gewünschten hohen Beladungsmengen in Bezug auf Öle bzw. Fettphasen realisiert werden, da die diesbezügliche Beladungskapazität im Allgemeinen nicht ausreichend ist. Dies bedeutet, dass auch in auf derartige Zusammensetzungen basierenden Produkte bzw. Erzeugnisse, in welche derartige Zusammensetzungen eingearbeitet sind, gleichermaßen nicht die gewünschte hohe Menge bzw. den gewünschten Gehalt an Ölen bzw. Fetten aufweisen. Hierzu ist versucht worden, Gummi arabicum mit weiteren Materialien für das Trägersystem zu kombinieren bzw. weitere Trägersysteme einzusetzen, hierunter beispielsweise auch modifizierte Stärke bzw. Maltodextrin als weitere Trägermaterialien, was wiederum mit den vorgenannten Nachteilen, welche mit diesen einhergehen, verknüpft ist.

Die wissenschaftliche Publikation gemäß Savihta Krishnan et al. "Microencapsulation of cardamom oleoresin: Evaluation of blends of gum arabic, maltodextrin and a modified starch as wall materials ", Carbohydrate Polymers, Vol. 61, Issue 1, 4. Juli 2005, Seiten 95 bis 102*,* betrifft die Mikroverkapselung von Kardamom-Oleoresin und die diesbezügliche Analyse von Mischungen aus Gummi arabicum, Maltodextrin und einer modifizierten Stärke. Die Mikroverkapselung wird dabei insbesondere auf Basis von binären und ternären Mischungen der vorgenannten Komponenten ausgebildet. Somit wird auf Mischsysteme zur Ausbildung von Verkapselungen abgestellt. Die Verwendung binärer bzw. ternärer Mischungen ist dabei mit bestimmten Nachteilen verbunden, wie auch zuvor in Bezug auf die jeweiligen Substanzen angeführt.

Die WO 2017/017248 A1 betrifft ein Verfahren zur Herstellung von Gummi arabicum, welches die Schritte des Bereitstellens eines Gummi arabicums aus Akazien-Syal und des Auswählens von Gummi arabicum mit einem Tanningehalt von > 700 ppm umfasst, wobei das hergestellte Gummi arabicum eine verbesserte Emulgierleistung aufweist.

Weiterhin betrifft der wissenschaftliche Publikation gemäß Al-Assaf et al. "Characterization and properties of Acacia Senegal (L.) Willd. var. Senegal with enhanced properties (Acacia (sen) SUPER GUM™): Part 1-Controlled maturation of Acacia Senegal var. Senegal to increase viscoelasticity, produce a hydrogel form and convert a poor into a good emulsifier", Food Hydrocolloids, Elsevier BV, NL, Bd. 21, Nr. 3, 16. November 2006, Seiten 319-328, die Charakterisierung und Untersuchung der Eigenschaften von Acacia senegal, wobei die kontrollierte Reifung von Acacia senegal zur Erhöhung der Viskoelastizität zur Herstellung einer Hydrogelform und Umwandlung eines schlechten in einen guten Emulgator durchgeführt wird.

Zudem betrifft die WO 2021/041005 A1 ein Verfahren zur Herstellung von modifiziertem Gummi arabicum, wobei das Gummi arabicum erhitzt wird, wodurch wärmebehandeltes Gummi arabicum entsteht, wobei dieses wärmebehandelte Gummi arabicum in einer Lösung aufgelöst wird, optional gefiltert wird und wobei die Gummi arabicum enthaltene Lösung anschließend einer Sprühtrocknung unterzogen wird.

Darüber hinaus betrifft die EP 1 666 502 A1 ein Verfahren zur Verbesserung der Emulgierfähigkeit von Gummi arabicum, wobei Gummi arabicum unter trockenen Bedingungen erhitzt wird derart, dass der Trocknungsverlust nicht mehr als 3 % beträgt.

Wie zuvor angeführt, werden im Stand der Technik zahlreiche mögliche Trägermaterialien für Öle bzw. Fettphasen verwendet.

In diesem Zusammenhang betrifft die US 10 463 626 B2 eine trockene Zusammensetzung, welche dreidimensionale poröse Mikropartikel umfasst. Die Mikropartikel umfassen einen Wirkstoff, polymere Nanopartikel, Lipidtröpfchen sowie ein Nanopartikel-Stabilisierungsmittel und ein Kälteschutzmittel, wobei der Wirkstoff von den Nanopartikeln bzw. Lipidtröpfchen getragen wird. Die als Trägermaterial fungierenden Nanopartikel basieren dabei auf einem PLGA-Polymer (Poly(lactid-co-glycolid)).

Weiterhin betrifft die wissenschaftliche Publikation gemäß Vita Paramita et al. "High-Oil-Load Encapsulation of Medium-Chain Triglycerides and d-Limonene Mixture in Modified Starch by Spray Drying", Journal of Food Science, Vol. 77, Band 2, Seiten E38 bis E44*,* die Verkapselung eines Gemischs von mittelkettigen Triglyceriden und d-Limonen in modifizierter Stärke. Diesbezüglich wird somit modifizierte Stärke als Trägermaterial eingesetzt. Wie zuvor angeführt, ist der Einsatz modifizierter Stärke mit bestimmten Nachteilen verbunden.

Zusammenfassend ist somit für Trägermaterialien des Standes der Technik, welche für die Aufnahme bzw. Fixierung von Ölen bzw. Fettphasen verwendet werden, sowie für Zusammensetzungen auf Basis derartiger Trägersysteme anzuführen, dass nicht immer hohe Beladungsgrade mit den Ölen bzw. Fettphasen realisiert werden können und dass derartige Systeme bzw. Zusammensetzungen nicht immer optimale Anwendungs- und Verarbeitungseigenschaften, insbesondere im Hinblick auf die Bereitstellung entsprechender Produkte bzw. Erzeugnisse, wie Nahrungsmittel, Pharmazeutika oder Kosmetika, aufweisen, und zwar auch was die Ausbildung stabiler bzw. homogener Emulsionen oder dergleichen angelangt. Zudem liegt nicht immer eine optimale Dosierbarkeit der in Rede stehenden Systeme bzw. Zusammensetzungen vor.

Mittelkettigen Triglyceriden (MKTs bzw. *Medium-Chain Triglycerides,* MCTs) kommt aufgrund ihrer speziellen Eigenschaften eine hohe Bedeutung im Bereich von Nahrungsmitteln, kosmetischen sowie pharmazeutischen Produkten zu. So werden mittelkettige Triglyceride oftmals im Rahmen der Produktion von Kosmetika und Pharmazeutika eingesetzt, beispielsweise als Trägerstoff für weitere Inhaltsstoffe bzw. Komponenten, Lösungsvermittler, Trennmittel, Gleitmittel oder dergleichen. Zudem finden mittelkettige Triglyceride (MKTs) oftmals Verwendung im Bereich der Nahrungsmittelindustrie, beispielsweise aufgrund ihrer metabolischen Eigenschaften bzw. Besonderheiten, und zwar auch in diätischen Lebensmitteln oder dergleichen. Auch von daher besteht für mittelkettige Triglyceride ein hoher Bedarf an der Bereitstellung entsprechender Erzeugnisse bzw. Produkte, welche in Form von homogenen bzw. stabilen Dispersionen, insbesondere Emulsionen, vorliegen, wobei für die mittelkettigen Triglyceride als solche gleichermaßen eine homogene Verteilung bzw. hohe Gehalte bzw. Konzentrationen sowie eine hohe Bioverfügbarkeit, insbesondere in Bezug auf Nahrungsmittel, gegeben sein soll, und dies bei gleichzeitig hoher Lagerstabilität. Mit den im Stand der Technik vorliegenden Trägersystemen sowie diesbezüglichen Zusammensetzungen, welche derartige Trägersysteme enthalten, ist aber eine zufriedenstellende Ausrüstung mit mittelkettigen Triglyceriden aufgrund der vorgenannten Unzulänglichkeiten nicht immer gegeben.

Vor diesem Hintergrund besteht eine Aufgabe der vorliegenden Erfindung darin, Trägersysteme bzw. derartige Trägersysteme enthaltende Zusammensetzungen zur Aufnahme bzw. Fixierung von Ölen bzw. Fettphasen, insbesondere von mittelkettigen Triglyceriden (MKTs), bereitzustellen, welche die zuvor geschilderten Nachteile des Standes der Technik zumindest weitgehend vermeiden oder aber wenigstens abschwächen.

Insbesondere sollen diesbezügliche Trägersysteme bzw. Zusammensetzungen bereitgestellt werden, welche eine effiziente Aufnahme bzw. Fixierung von Ölen und Fettphasen ermöglichen, und zwar insbesondere im Hinblick auf mittelkettige Triglyceride (MKTs), wobei insgesamt hohe Beladungsgrade der Trägersysteme bzw. der Zusammensetzungen mit den mittelkettigen Triglyceriden (MKTs) ermöglicht werden sollen. Dabei sollen diesbezügliche Trägersysteme bzw. Zusammensetzungen auch verbesserte Handhabungs- und Verwendungs- bzw. Verarbeitungseigenschaften aufweisen, insbesondere im Hinblick auf deren Verwendung und Einsatz in weiterführenden Produkten bzw. Erzeugnissen, welche in Form stabiler und homogener Dispersionen, insbesondere Emulsionen, oder aber auch in fester bzw. pulverförmiger Form ausgebildet sein sollen.

In vollkommen überraschender Weise hat die Anmelderin im Rahmen der vorliegenden Erfindung herausgefunden, dass eine Zusammensetzung mit einem hohen Gehalt an mittelkettigen Triglyceriden (MKTs) ausgerüstet bzw. bereitgestellt werden kann, wenn für die Zusammensetzung Partikel auf Basis eines speziellen Trägermaterials insbesondere zur Bindung bzw. Fixierung des mittelkettigen Triglycerids (MKT) eingesetzt werden, nämlich in Form eines polysaccharidbasierten Trägermaterials, welches in spezieller Weise Gummi arabicum umfasst bzw. hieraus gebildet ist, wobei das Gummi arabicum einen hohen Glykoproteingehalt aufweist, wie nachfolgend im Einzelnen angeführt.

Denn aufgrund der ziel- und zweckgerichteten Verwendung eines Trägermaterials auf Basis eines speziellen Gummi arabicum kann die Ausrüstung bzw. Beaufschlagung des Trägermaterials mit dem mittelkettigen Triglycerid (MKT) in völlig überraschender Weise dahingehend verbessert werden, dass insgesamt große Mengen an mittelkettigem Triglycerid (MKT) durch die Zusammensetzung aufgenommen bzw. an den Partikeln fixiert werden können. Erfindungsgemäß wird somit eine spezielle Zusammensetzung mit einem hohen Anteil bzw. Gehalt an mittelkettigem Triglycerid (MKT) bereitgestellt, was zu insgesamt verbesserten Produkt- und Anwendungseigenschaften der Zusammensetzung führt, wie gleichermaßen nachfolgend dargelegt. Auch vor diesem Hintergrund eignet sich die erfindungsgemäße Zusammensetzung zur Verwendung in einer Vielzahl von Produkten im Bereich der Lebensmittel bzw. Nahrungsmittel sowie im Bereich der Kosmetik bzw. Pharmazie.

Zur Lösung der zuvor geschilderten Aufgabe schlägt die vorliegende Erfindung somit - gemäß einem **ersten** Aspekt der vorliegenden Erfindung - eine mittelkettige Triglyceride (MKTs) enthaltende pulverförmige Zusammensetzung gemäß Anspruch 1 vor; weitere, insbesondere besondere und/oder vorteilhafte Ausgestaltungen der erfindungsgemäßen Zusammensetzung nach der Erfindung sind Gegenstand der die Zusammensetzung betreffenden Unteransprüche.

Weiterhin betrifft die vorliegende Erfindung - gemäß einem **zweiten** Aspekt der vorliegenden Erfindung - ein Nahrungsmittel-, kosmetisches oder pharmazeutisches Erzeugnis, welches die Zusammensetzung nach der Erfindung enthält, gemäß dem das erfindungsgemäße Erzeugnis betreffenden unabhängigen Anspruch.

Gleichermaßen betrifft die vorliegende Erfindung - gemäß einem **dritten** Aspekt der vorliegenden Erfindung - auch die Verwendung der Zusammensetzung nach der Erfindung in einem Nahrungsmittel-, kosmetischen oder pharmazeutischen Erzeugnis bzw. zur Herstellung eines Nahrungsmittel-, kosmetischen oder pharmazeutischen Erzeugnisses gemäß dem die erfindungsgemäße Verwendung der Zusammensetzung betreffenden unabhängigen Anspruch.

Darüber hinaus betrifft die vorliegende Erfindung - gemäß einem **vierten** Aspekt der vorliegenden Erfindung - das erfindungsgemäße Verfahren zur Herstellung der diesbezüglichen mittelkettige Triglyceride (MKCs) enthaltenden pulverförmigen Zusammensetzung gemäß dem das erfindungsgemäße Verfahren betreffenden unabhängigen Anspruch.

Des Weiteren betrifft die vorliegende Erfindung - gemäß einem **fünften** Aspekt der vorliegenden Erfindung - das erfindungsgemäße Verfahren zur Steuerung bzw. Einstellung der Beladung, insbesondere Beladungsmenge, eines Gummi arabicum umfassenden bzw. hieraus gebildeten polysaccharidbasierten Trägermaterials mit mindestens einem mittelkettigen Triglycerid (MKT) gemäß dem diesbezüglichen unabhängigen Anspruch.

Darüber hinaus betrifft die vorliegende Erfindung - gemäß einem **sechsten** Aspekt der vorliegenden Erfindung - die Verwendung des Glykoproteingehalts eines Gummi arabicum umfassenden bzw. hieraus gebildeten polysaccharidbasierten Trägermaterials zur Steuerung bzw. Einstellung der Beladung, insbesondere Beladungsmenge, des polysaccharidbasierten Trägermaterials mit mindestens einem mittelkettigen Triglycerid (MKT) gemäß dem die erfindungsgemäße Verwendung betreffenden unabhängigen Anspruch.

Es versteht sich bei den nachfolgenden Ausführungen von selbst, dass Ausgestaltungen, Ausführungsformen, Vorteile und dergleichen, welche nachfolgend zu Zwecken der Vermeidung von Wiederholungen nur zu einem Erfindungsaspekt ausgeführt sind, selbstverständlich auch in Bezug auf die übrigen Erfindungsaspekte entsprechend gelten, ohne dass dies einer gesonderten Erwähnung bedarf.

Des Weiteren versteht es sich von selbst, dass einzelne Aspekte und Ausführungsformen der vorliegenden Erfindung auch in beliebiger Kombination mit anderen Aspekten und Ausführungsformen der vorliegenden Erfindung als offenbart gelten und insbesondere auch eine beliebige Kombination von Merkmalen und Ausführungsformen, wie sie sich aus den Rückbezügen aller Patentansprüche ergibt, umfangreich als offenbart gilt, und zwar im Hinblick auf sämtliche sich ergebenden Kombinationsmöglichkeiten.

Insbesondere gilt für die die Erfindung charakterisierenden Merkmale, dass auch beliebige Kombinationen dieser Merkmale als offenbart gelten, wobei Ausführungsformen gleicher Präferenz der verschiedenen Merkmale in ihrer Kombination bevorzugt sind (z.B. Mengen bzw. Mengenbereiche der betreffenden Inhaltsstoffe gleicher Präferenz oder dergleichen). Ebenfalls sind auch sämtliche anderweitige Kombinationen (d.h. Kombinationen auf Basis unterschiedlicher Präferenzen bzw. unterschiedlicher Bevorzugungsebenen) mitoffenbart.

Bei allen nachstehend genannten relativen bzw. prozentualen gewichtsbezogenen Angaben, insbesondere relativen Mengen- oder Gewichtsangaben, ist weiterhin zu beachten, dass diese im Rahmen der vorliegenden Erfindung vom Fachmann derart auszuwählen sind, dass sie sich in der Summe unter Einbeziehung aller Komponenten bzw. Inhaltsstoffe, insbesondere wie nachfolgend definiert, stets zu 100 % bzw. 100 Gew.-% ergänzen bzw. addieren; dies versteht sich aber für den Fachmann von selbst.

Im Übrigen gilt, dass der Fachmann - anwendungsbezogen oder aber einzelfallbedingt - von den nachfolgend angeführten Werte- bzw. Bereichsangaben der vorliegenden Mengen bzw. Gehalte der Inhaltsstoffe bzw. Komponenten erforderlichenfalls abweichen kann, ohne dass er den Rahmen der vorliegenden Erfindung verlässt.

Zudem gilt, dass alle im Folgenden genannten Werte- bzw. Parameterangaben oder dergleichen grundsätzlich mit genormten bzw. standardisierten oder explizit angegebenen Bestimmungsverfahren oder andernfalls mit dem Fachmann auf diesem Gebiet an sich geläufigen Bestimmungs- bzw. Messmethoden ermittelt bzw. bestimmt werden können.

Dies vorausgeschickt, wird die vorliegende Erfindung nunmehr nachfolgend im Detail erläutert:
Gegenstand der vorliegenden Erfindung - gemäß einem **ersten** Aspekt der vorliegenden Erfindung - ist somit die erfindungsgemäße mittelkettige Triglyceride (MKTs) enthaltende pulverförmige Zusammensetzung, insbesondere zur Verwendung in kosmetischen, pharmazeutischen oder Nahrungsmittelerzeugnissen, wobei die Zusammensetzung eine Vielzahl einzelner Partikel umfasst oder hieraus besteht, wobei die Partikel jeweils mindestens ein polysaccharidbasiertes Trägermaterial umfassen, wobei das polysaccharidbasierte Trägermaterial mit mindestens einem mittelkettigen Triglycerid (MKT), insbesondere mit mindestens einem Triglycerid mindestens einer linearen gesättigten C₆-, C₈-, C₁₀- oder C₁₂-Carbonsäure, versehen und/oder beaufschlagt ist und wobei die Partikel jeweils mindestens ein polysaccharidbasiertes Trägermaterial und mindestens ein an das polysaccharidbasierte Trägermaterial gebundenes und/oder fixiertes mittelkettiges Triglycerid (MKT), insbesondere mindestens ein an das polysaccharidbasierte Trägermaterial gebundenes und/oder fixiertes Triglycerid mindestens einer linearen gesättigten C₆-, C₈-, C₁₀- oder C₁₂-Carbonsäure, umfassen;
wobei das polysaccharidbasierte Trägermaterial Gummi arabicum umfasst oder hieraus gebildet aus, wobei das Gummi arabicum einen Glykoproteingehalt, bezogen auf das Gummi arabicum, im Bereich von 4 Gew-% bis 25 Gew.-% aufweist, wobei sich der Glykoproteingehalt auf den Gesamtgehalt aller im Gummi arabicum vorhandener Glykoproteine bezieht, und
wobei die Zusammensetzung, bezogen auf die Zusammensetzung, einen Triglyceridgehalt im Bereich von 50 Gew.-% bis 90 Gew.-% aufweist.

Überraschenderweise hat die Anmelderin eine effiziente wie wirksame Möglichkeit gefunden, eine Zusammensetzung mit einer hohen Menge bzw. einem hohen Gehalt an mittelkettigem Triglycerid auszurüsten bzw. zu versehen, nämlich durch die ziel- und zweckgerichtete Bereitstellung einer partikulären bzw. pulverförmigen Zusammensetzung unter Verwendung eines speziellen polysaccharidbasierten Trägermaterials, welches ein wiederum spezielles Gummi arabicum umfasst oder hieraus gebildet ist, wonach nämlich im Rahmen der vorliegenden Erfindung das Gummi arabicum einen hohen Glykoproteingehalt aufweist.

In diesem Zusammenhang ist es im Rahmen der vorliegenden Erfindung gleichermaßen unerwartet und überraschend, dass durch den Einsatz von Gummi arabicum mit hohem Glykoproteingehalt eine hohe Menge an mittelkettigem Triglycerid an das polysaccharidbasierte Trägermaterial gebunden bzw. fixiert werden kann, wobei gleichzeitig auch eine hervorragende Dispergierbarkeit, insbesondere Emulgierbarkeit der erfindungsgemäßen Zusammensetzung, in Flüssigkeiten, insbesondere in Wasser, vorliegt, so dass auf dieser Basis Erzeugnisse bzw. (End-)Produkte (wie insbesondere Nahrungsmittel-, kosmetische bzw. pharmazeutische Produkte) in Form von homogenen und stabilen Dispositionen, insbesondere Emulsionen, erhalten werden, welche zudem gleichermaßen einen hohen MKT-Gehalt aufweisen. Die hervorragenden Eigenschaften hinsichtlich der Dispergierbarkeit, insbesondere Emulgierbarkeit, der erfindungsgemäßen Zusammensetzung sind dabei auch insofern überraschend, als ein hoher Gehalt an mittelkettigem Triglycerid den diesbezüglichen Eigenschaften grundsätzlich abträglich ist. Auch ist die erfindungsgemäße Zusammensetzung riesel- und fließfähig ausgebildet und lässt sich ohne Weiteres, insbesondere homogen und ohne Anwendung großer Misch- und/oder Scherenergie, in pulverförmige Zusammensetzungen einarbeiten bzw. zu pulverförmigen Produkten verarbeiten.

In diesem Zusammenhang kommt dem erfindungsgemäß eingesetztem polysaccharidbasierten Trägermaterial auf Basis eines speziellen Gummi arabicum mit hohem Glykoproteingehalt eine besondere Bedeutung zu. Ohne sich auf diese Theorie beschränken oder berufen zu wollen, fungiert das erfindungsgemäß eingesetzte Gummi arabicum im Hinblick auf die mittelkettigen Triglyceride nämlich insbesondere als intrinsischer Dispergator, insbesondere Emulgator, und zwar in mehrfacher Hinsicht, nämlich sowohl bei Beladung der Zusammensetzung bzw. dem diesbezüglichen Trägermaterial mit den mittelkettigen Triglyceriden einerseits und bei der Ausbildung der Dispersion, insbesondere Emulsion, im Rahmen der Anwendung bzw. Verwendung der erfindungsgemäßen Zusammensetzung unter entsprechender Einbringung der Zusammensetzung in eine Flüssigkeit, insbesondere Wasser, zur Ausbildung entsprechender Erzeugnisse bzw. Produkte andererseits. Dabei liegt - gleichermaßen ohne sich auf diese Theorie berufen oder beschränken zu wollen - sowohl für die Anbindung bzw. Fixierung der mittelkettigen Triglyceride (MKTs) an das polysaccharidbasierte Trägermaterials als auch für die nachfolgende Ausbildung einer Dispersion, insbesondere Emulsion, eine optimale Wechselwirkung zwischen den Bestandteilen des Gummi arabicums, nämlich insbesondere auf Basis der diesbezüglichen Glykoproteine einerseits und den mittelkettigen Triglyceriden (MKTs) andererseits vor. Dies führt insgesamt zu verbesserten Anbindungs- bzw. Beladungseigenschaften und zudem auch zu guten Freisetzungseigenschaften im Hinblick auf die Ausbildung von Dispersionen, insbesondere Emulsionen, bei Einbringen der Zusammensetzung in Flüssigkeiten, wie Wasser.

Darüber hinaus weist die erfindungsgemäße partikel- bzw. pulverförmige Zusammensetzung auch eine ausgezeichnete Rieselfähigkeit und Fließfähigkeit auf. Dies geht auch mit einer guten Handhabbarkeit bei der Herstellung der Zusammensetzung nach der Erfindung sowie mit einer guten Handhabbarkeit der erfindungsgemäßen Zusammensetzung bei ihrer Anwendung bzw. Verarbeitung einher, insbesondere auch was eine hervorragende Dosierbarkeit und das Vermeiden von Klumpen- bzw. Makroagglomeratbildungen oder dergleichen anbelangt. Auf dieser Basis können homogene (End-)Produkte bzw. Erzeugnisse aufgrund stabiler und homogener Dispersionen, insbesondere Emulsionen auf Basis der erfindungsgemäßen Zusammensetzung bereitgestellt werden. Insbesondere weist die erfindungsgemäße Zusammensetzung in überraschender Weise und trotz der hohen Beladung bzw. des hohen Gehalts an mittelkettigen Triglyceriden (MKTs) keine der Fließfähigkeit bzw. Rieselfähigkeit entgegenstehende Klebrigkeit bzw. Adhäsivität der Partikel oder dergleichen auf. Folglich lässt sich die erfindungsgemäße Zusammensetzung insbesondere auch ohne Weiteres, insbesondere homogen und ohne Anwendung großer Misch- und/oder Scherenergie, in pulverförmige Zusammensetzungen einarbeiten bzw. zu pulverförmigen Produkten verarbeiten.

Insbesondere können auf Basis der erfindungsgemäßen Zusammensetzung entsprechende (End-)Produkte, wie Nahrungsmittel-, kosmetische bzw. pharmazeutische Erzeugnisse, mit einem hohen Gehalt an mittelkettigen Triglyceriden (MKTs) versehen werden, was mit weiteren zentralen Vorteilen einhergeht, nämlich insbesondere infolge der positiven Eigenschaften von mittelkettigen Triglyceriden (MKTs), beispielsweise als Trägerstoff, Lösungsvermittler, Trennmittel, Gleitmittel, Hydrophobiermittel, Film- und Schutzbildner sowie Viskositätsregulator oder dergleichen. Auf dieser Basis können entsprechende Produkte mit diesbezüglich optimierten Eigenschaften bereitgestellt werden.

Zudem weisen mittelkettige Triglyceride hervorragende ernährungsphysiologische bzw. metabolische Eigenschaften auf, so dass auch die diesbezüglichen Eigenschaften entsprechender (Nahrungsmittel-)Erzeugnisse verbessert werden können.

Insgesamt kann auf Basis der erfindungsgemäßen Zusammensetzung die Einarbeitung von mittelkettigen Triglyceriden (MKTs) in Formulierungen zum Erhalt entsprechender Produkte bzw. Erzeugnisse erleichtert bzw. verbessert werden.

Infolge der Bereitstellung entsprechender Produkte bzw. Erzeugnisse mit einem hohen Gehalt an mittelkettigen Triglyceriden (MKTs) können beispielsweise im Hinblick auf Kosmetika gute Pflegeeigenschaften und Anwendungseigenschaften (z. B. gutes Auftragen von Cremes oder dergleichen auf die Haut) bereitgestellt werden.

Auch in diesem Zusammenhang liegt für die mittelkettigen Triglyceride (MKTs) eine hohe Bioverfügbarkeit vor, insbesondere da diese in den anwendungsfertigen Produkten homogen verteilt und in stabilen Dispersionen, insbesondere Emulsionen, inkorporiert vorliegen.

Die erfindungsgemäße Zusammensetzung ist zudem mit dem Vorteil verbunden, dass diese insbesondere vegan ausgebildet sein kann. Darüber hinaus kann die Zusammensetzung nach der Erfindung zumindest im Wesentlichen geschmacks- und/oder geruchsneutral bzw. zumindest im Wesentlichen farblos ausgebildet sein. Insbesondere weist die erfindungsgemäße Zusammensetzung auch eine hohe Stabilität, insbesondere Lagerstabilität, auf. In diesem Zusammenhang können die erfindungsgemäßen Zusammensetzungen eine Lagerstabilität von mindestens 36 Monaten bei einer Lagertemperatur unterhalb von 25° C in trockener Atmosphäre (d.h. relative Luftfeuchtigkeit kleiner 60 %) aufweisen, insbesondere bei Lagerung bzw. Aufbewahrung in einem luft- und/oder feuchtigkeitsdichten und/oder lichtundurchlässigen Behälter.

Zudem verhält es sich im Rahmen der vorliegenden Erfindung aber auch derart, dass die erfindungsgemäße Zusammensetzung infolge des hohen Gehalts an mittelkettigen Triglyceriden (MKTs) auch einen damit einhergehenden hohen Ketokörper-Index (ketogener Index) aufweist, so dass auf dieser Basis auch entsprechende Produkte bzw. Erzeugnisse mit gleichermaßen hohem Ketokörper-Index bereitgestellt werden können. Vor diesem Hintergrund kommt der erfindungsgemäßen Zusammensetzung auch eine entsprechend hohe ernährungsphysiologische bzw. diätische Relevanz zu, beispielsweise was die Verwendung der Zusammensetzung nach der Erfindung in Nahrungsmitteln bzw. diätischen Lebensmitteln oder dergleichen anbelangt. Denn bei physiologischer Verstoffwechselung von mittelkettigen Triglyceriden (MKTs) im menschlichen Körper werden Keto- bzw. Ketonkörper gebildet, was von entsprechender ernährungsphysiologischer Relevanz beispielsweise im Hinblick auf Diäten, Spezialernährung oder dergleichen ist. Insbesondere kann auf dieser Basis auch eine hohe Energiedichte diesbezüglicher Zusammensetzungen und Produkte bzw. Erzeugnisse bereitgestellt werden.

Die erfindungsgemäße Zusammensetzung kann dabei beispielsweise Verwendung in Salben, Cremes, Pasten, Lotionen, Gelen, Sprays bzw. Flüssigkeiten finden, wie nachfolgend noch angeführt. Die erfindungsgemäße Zusammensetzung kann insbesondere auch für Produkte bzw. Erzeugnisse auf Basis von Trockenformulierungen, insbesondere pulverförmigen Formulierungen, eingesetzt werden, zumal auch eine gute Vermischbarkeit vorliegt. Insbesondere kann eine Verwendung für trocken- bzw. pulverförmige kosmetische Produkte, wie Shampoos, Duschgels, Hautpflegeprodukte oder dergleichen, zweckmäßig sein. Aber auch für den Bereich der Nahrungsmittel und Nahrungsmittelerzeugnisse sowie der Pharmazeutika kommen pulverförmige Ausgestaltungen bzw. Erzeugnisse auf Basis von Trockenformulierungen, insbesondere pulverförmigen Formulierungen, in Betracht.

Hierbei kann, sofern erforderlich oder gewünscht, die Zugabe von Flüssigkeit, insbesondere Wasser, erst durch den Anwender erfolgen. Aufgrund der hervorragenden Dispergierbarkeit, insbesondere Emulgierbarkeit, resultiert dann im Rahmen der Anwendung unter Zugabe einer Flüssigkeit, insbesondere Wasser, unmittelbar die Ausbildung einer entsprechend anwendungsfertigen homogenen Dispersion, insbesondere Emulsion.

**In** diesem Zusammenhang weist die erfindungsgemäße Zusammensetzung auch den Vorteil auf, dass nach Zugabe einer Flüssigkeit, insbesondere Wasser (oder aber Lösemitteln), sofort eine Emulsion erhalten wird, und zwar auch ohne weiterführendes Erhitzen. In diesem Zusammenhang handelt es sich bei der erfindungsgemäßen Zusammensetzung somit insbesondere auch um eine effektive Transformationszusammensetzung bzw. -pulver, welche(s) nach Zugabe von Flüssigkeit, insbesondere Wasser, zu einer stabilen und homogenen Emulsion führt ("vom Pulver zur Emulsion"). Auf dieser Basis wird im Rahmen der vorliegenden Erfindung die Einarbeitung bzw. Inkorporation von MKTs in Erzeugnissen bzw. Produkten erleichtert, und zwar auch für tensidhaltige Formulierungen ("vom Pulver zum Tensid"). Die erfindungsgemäße Zusammensetzung kann auch insbesondere pulverförmigen Formulierungen, wie beispielsweise partikel- bzw. pulverförmigen und/oder im Wesentlichen wasserfreien Haut- und Haarreinigungsprodukten, insbesondere (Trocken-)Shampoos bzw. (Trocken-)Duschgelen, zugesetzt werden. Insbesondere kann die erfindungsgemäße Zusammensetzung auch für Formulierungen auf Basis von Balsamen, auf Basis von sprühgetrockneten Emulsionen oder Mischungen verschiedener Öle zugesetzt werden. Dabei kann auch bei derartigen Formulierungen ein hoher Gehalt an MKTs eingestellt werden, was bei kosmetischen Produkten auch zu verbesserten Pflegeeigenschaften führt, und zwar auch im Hinblick auf Pulverformulierungen. In diesem Zusammenhang kann im Rahmen der vorliegenden Erfindung der Nachteil überkommen werden, dass insbesondere bei partikel- bzw. pulverförmigen bzw. zumindest im Wesentlichen wasserfreien Formulierungen bzw. Erzeugnissen oftmals kein hoher Öl- bzw. Fettphasengehalt eingestellt werden kann. Vor diesem Hintergrund stellt die vorliegende Erfindung auch einen wertvollen Beitrag zur Entwicklung bzw. Bereitstellung von Produkten bzw. Erzeugnissen, wie Hautpflegeprodukten oder dergleichen, dar, bei welchen in der Herstellung zumindest im Wesentlichen kein Wasser verwendet wird und wobei erst im Rahmen der Anwendung bzw. Verwendung durch den Verbraucher Wasser zur Ausbildung einer Emulsion hinzugefügt wird, was aufgrund der hervorragenden Eigenschaften bereits auch bei Raumtemperatur erfolgen kann. Dies ist auch der Nachhaltigkeit zuträglich. Auf dieser Basis können entsprechende Emulsionen gebildet werden, die beispielsweise auf die Haut aufgetragen werden können und diesbezüglich gute Pflegeeigenschaften aufweisen. Insbesondere können in diesem Zusammenhang hohe Gehalte an MKTs realisiert werden, wie zuvor angeführt.

Zusammenfassend ist somit herauszustellen, dass die erfindungsgemäße Konzeption mit der Verwendung eines speziellen Gummi arabicum zur Bereitstellung eines diesbezüglichen polysaccharidhaltigen Trägermaterials für mittelkettige Triglyceride (MKTs) in überraschender Weise zu einer hohen Beladung mit mittelkettigen Triglyceriden (MKTs), nämlich bei den erfindungsgemäßen Zusammensetzungen zu einem Triglyceridgehalt von mindestens 50 Gew.-% führt und dies bei gleichzeitig hervorragender Riesel- bzw. Fließfähigkeit und Ausbildung stabiler Dispersionen bzw. Emulsionen im Rahmen der Anwendung der Zusammensetzung nach der Erfindung. Das erfindungsgemäß eingesetzte Gummi arabicum weist dabei einen hohen Gehalt an Glykoproteinen im Bereich von 4 Gew.-% bis 25 Gew.-%, bezogen auf das Gummi arabicum, auf.

Was die Partikel auf Basis des polysaccharidbasierten Trägermaterials und dem mindestens einen mittelkettigen Triglycerid (MKT) anbelangt, so können diese in beliebiger Form bzw. beliebiger körperlicher Ausbildung vorliegen. Insbesondere können die Partikel als Matrixpartikel ausgebildet sein, insbesondere wobei das Trägermaterial die Matrix zur Aufnahme der mittelkettigen Triglyceride (MKTs) bildet. Diesbezüglich können die mittelkettigen Triglyceride (MKTs) in das Matrix- bzw. Trägermaterial eingelagert bzw. inkorporiert und/oder auf das Matrix- bzw. Trägermaterial aufgelagert sein; grundsätzlich kann die Matrix porös oder aber kontinuierlich bzw. dicht ausgebildet sein. Darüber hinaus können die Partikel auf Basis des polysaccharidbasierten Trägermaterials und des mindestens einen mittelkettigen Triglycerids (MKT) auch als Kapselpartikel, insbesondere Kern/Hülle-Kapseln (Core/Shell-Aufbau bzw. Core/Shell-Aufbau), ausgebildet sein; diesbezüglich kann es insbesondere vorgesehen sein, dass das polysaccharidbasierte Trägermaterial die Kapselhülle ausbildet, welche die mittelkettigen Triglyceride (MKTs) umschließen (wobei zusätzlich mittelkettige Triglyceride auch auf bzw. an der äußeren Kapseloberfläche vorhanden sein können).

Gemäß einer besonderen Ausführungsform der vorliegenden Erfindung kann es vorgesehen sein, dass die Partikel der erfindungsgemäßen Zusammensetzung jeweils eine Mehrzahl von Primärpartikeln (= Basispartikeln) umfassen und/oder hieraus gebildet sind, insbesondere wobei die Primärpartikel miteinander verbunden und/oder miteinander agglomeriert sind und/oder insbesondere wobei die Primärpartikel jeweils das mindestens eine mittelkettige Triglycerid und das Gummi arabicum umfassen oder hieraus gebildet sind, vorzugsweise jeweils mit Kern/Hülle-Aufbau und/oder vorzugsweise jeweils als Kern/Hülle-Primärpartikel, vorzugsweise wobei der Kern das mindestens eine mittelkettige Triglycerid umfasst oder hieraus gebildet ist und die Hülle das Gummi arabicum umfasst oder hieraus gebildet ist. Gemäß einer besonderen Ausgestaltung, insbesondere bei hohen MKT-Beladungen, kann es dabei insbesondere gegebenenfalls vorgesehen sein, dass das mindestens eine mittelkettige Triglycerid zusätzlich auf bzw. an der Oberfläche des Kern/Hülle-Aufbaus und/oder der Kern/Hülle-Primärpartikel, insbesondere auf der Hülle, vorhanden ist (d. h. als sogenanntes "freies" Oberflächenöl, dessen Anteil bzw. Gehalt analytisch bestimmt werden kann, insbesondere mittels Lösungsmittelextraktion mit einem im Allgemeinen lipophilen Lösemittel, wie z. B. Diethylether); in diesem Zusammenhang kann der Gehalt von auf bzw. an der Oberfläche des Kern/Hülle-Aufbaus und/oder der Kern/Hülle-Primärpartikel, insbesondere auf der Hülle, vorhandenem/n mittelkettigem/n Triglycerid(en), bezogen auf die Partikel, insbesondere 5 Gew.-% bis 60 Gew.-%, vorzugsweise 10 Gew.-% bis 55 Gew.-%, bevorzugt 15 Gew.-% bis 50 Gew.-%, besonders bevorzugt 20 Gew.-% bis 45 Gew.-%, betragen.

Gemäß einer besonderen Ausführungsform der vorliegenden Erfindung kann es weiterhin vorgesehen sein, dass die Partikel der erfindungsgemäßen Zusammensetzung jeweils eine Mehrzahl von miteinander verbundenen und/oder miteinander agglomerierten Primärpartikeln (= Basispartikeln) umfassen und/oder hieraus gebildet sind, insbesondere wobei die Primärpartikel jeweils das mindestens eine mittelkettige Triglycerid und das Gummi arabicum umfassen oder hieraus gebildet sind, vorzugsweise jeweils mit Kern/Hülle-Aufbau und/oder vorzugsweise jeweils als Kern/Hülle-Primärpartikel, vorzugsweise wobei der Kern das mindestens eine mittelkettige Triglycerid umfasst oder hieraus gebildet ist und die Hülle das Gummi arabicum umfasst oder hieraus gebildet ist. Gemäß einer besonderen Ausgestaltung, insbesondere bei hohen MKT-Beladungen, kann es dabei insbesondere gegebenenfalls vorgesehen sein, dass das mindestens eine mittelkettige Triglycerid zusätzlich auf bzw. an der Oberfläche des Kern/Hülle-Aufbaus und/oder der Kern/Hülle-Primärpartikel, insbesondere auf der Hülle, vorhanden ist (d. h. als sogenanntes "freies" Oberflächenöl, dessen Anteil bzw. Gehalt analytisch bestimmt werden kann, insbesondere mittels Lösungsmittelextraktion mit einem im Allgemeinen lipophilen Lösemittel, wie z. B. Diethylether); in diesem Zusammenhang kann der Gehalt von auf bzw. an der Oberfläche des Kern/Hülle-Aufbaus und/oder der Kern/Hülle-Primärpartikel, insbesondere auf der Hülle, vorhandenem/n mittelkettigem/n Triglycerid(en), bezogen auf die Partikel, insbesondere 5 Gew.-% bis 50 Gew.-%, vorzugsweise 10 Gew.-% bis 40 Gew.-%, bevorzugt 15 Gew.-% bis 30 Gew.-%, betragen.

Was zudem das erfindungsgemäß eingesetzte Gummi arabicum anbelangt, so weist dies weiterführend verbesserte Eigenschaften auch als Emulgator und Stabilisator auf, und zwar auch in Bezug auf die mittelkettigen Triglyceride (MKTs), wie sie erfindungsgemäß eingesetzt werden. Weiterhin weist Gummi arabicum im Allgemeinen eine gute Verträglichkeit auf, da es nicht toxisch und nicht allergen ist. Zudem ist Gummi arabicum physiologisch inert, wobei zumindest im Wesentlichen keine Verstoffwechselung bei Aufnahme in den Körper vorliegt, so dass beispielsweise im Rahmen der Verwendung für Nahrungsmittelerzeugnisse oder dergleichen keine zusätzlichen Kalorien aufgenommen werden und zudem der mit den MKTs zusammenhängende Ketostoffwechsel nicht gestört wird. Insbesondere ist dies förderlich in Bezug auf die Ketokörperbildung. Zudem handelt es sich bei Gummi arabicum, wie es erfindungsgemäß eingesetzt wird, um eine vegane Substanz bzw. um einen naturbasierten Rohstoff mit definierter Zusammensetzung.

Im Allgemeinen bezeichnet Gummi arabicum (synonym auch als Gummiarabikum bzw. Arabisches Gummi genannt) das Gummi aus dem Exsudat von verschiedenen, insbesondere in Afrika verbreiteten Akazien-Bäumen.

Im Allgemeinen enthält Gummi arabicum Polysaccharide, und zwar insbesondere auf Basis von sauren Alkali- bzw. Erdalkalisalzen der Arabinsäure bzw. Polyarabinsäure. Zudem enthält Gummi arabicum daneben auch Glykoproteine (insbesondere Arabinogalactanproteine und darüber hinaus aber auch andere bzw. weitere, d. h. von Arabinogalactanproteine verschiedene Glykoproteine).

Im Allgemeinen wird Gummi arabicum, wie zuvor angeführt, aus dem Exsudat bzw. Wundsaft der Bäume gewonnen. Dabei kann insbesondere derart vorgegangen werden, dass die geschälte Baumrinde in einem nach unten gerichteten Winkel eingeschnitten wird. Aus dem heraussickernden Gummi können sich im Laufe von 20 bis 30 Tagen eine oberflächlich harte Blase oder wurm- bzw. hornförmige Stücke bilden, welche dann entfernt bzw. geerntet werden können. Der Baum kann aber auch von sich aus Gummi abscheiden, insbesondere wenn die Rinde durch Austrocknung aufreißt. Das erhaltene Material kann weiter getrocknet und weiteren Bearbeitungsschritten, wie Aufreinigungsschritten oder dergleichen, unterzogen werden.

Wie nachfolgend noch im Detail angeführt, stellt die vorliegende Erfindung gemäß einer erfindungsgemäßen Ausführungsform insbesondere auf die Verwendung eines sehr speziellen Gummi arabicum ab, welches nämlich im Speziellen aus dem Gummiarabicum-Baum (*Senegalia senegal* bzw. synonym *Acacia senegal* oder Senegal-Akazie), gewonnen ist bzw. hiervon stammt. Denn in diesem Zusammenhang hat die Anmelderin überraschend gefunden, dass ein derartig spezielles Gummi arabicum einen entsprechend hohen Gehalt an Glykoproteinen aufweist und sich von daher in besonderem Maße als polysaccharidbasiertes Trägermaterial für die erfindungsgemäße Zusammensetzung eignet.

Gummi arabicum ist insbesondere durch die CAS-Nummer (CAS-Registrierungsnummer) 9000-01-5 beschrieben bzw. gekennzeichnet.

In der Europäischen Union ist Gummi arabicum als Lebensmittelzusatzstoff mit der E-Nummer E 414 zugelassen.

Für weitergehende Einzelheiten zu Gummi arabicum kann auch verwiesen werden auf Römpp Chemie Lexikon, 10. Auflage, Band 2, Georg Thieme Verlag, Stuttgart/New York, 1997, Seiten 1625-1626, Stichwort: "Gummi arabicum", sowie die dort jeweils referierte Literatur.

Bei den angeführten Glykoproteinen, wie sie durch Gummi arabicum bereitgestellt werden bzw. wie sie im Gummi arabicum (insbesondere natürlich) vorhanden sind, handelt es sich im Allgemeinen um (Makro-)Moleküle, welche auf einem Protein bzw. Proteinrest oder -gruppe mit einer oder mehreren hieran gebundenen, insbesondere kovalent gebundenen, Kohlenhydratgruppe(n) (d. h. Zuckergruppen) basieren. Mit anderen Worten handelt es sich bei den Glykoproteinen, wie sie durch Gummi arabicum bereitgestellt werden, im Allgemeinen um glykosylierte Proteine, d. h. um an Kohlenhydratgruppen bzw. -resten (d. h. Zuckergruppen bzw. -resten) gebundene Proteine bzw. Proteinreste oder -gruppen. Dabei können die Kohlenhydratgruppen bzw. -reste grundsätzlich stark variieren und von Mono- über Di- und Oligosaccharide bis hin zu Polysacchariden reichen, wobei im Rahmen der vorliegenden Erfindung insbesondere Oligosaccharid- und vor allem Polysaccharidgruppen als Kohlenhydratgruppen der jeweiligen Glykoproteine vorliegen.

Insbesondere bezeichnet der Begriff der Glykoproteine, wie er im Rahmen der vorliegenden Erfindung verwendet wird, die Gesamtheit aller vorhandenen Glykoproteine. Mit anderen Worten wird der Begriff der Glykoproteine, wie er im Rahmen der vorliegenden Erfindung verwendet wird, als Ober- oder Sammelbezeichnung für sämtliche Glykoproteine, welche im erfindungsgemäß eingesetzten Gummi arabicum vorhanden sind, verwendet. Dies umfasst somit insbesondere Arabinogalactanproteine als spezielle im erfindungsgemäß eingesetzten Gummi arabicum vorhandene Glykoproteine, aber auch sämtliche weiteren im erfindungsgemäß eingesetzten Gummi arabicum vorhandenen Glykoproteine (d. h. auch von Arabinogalactanproteinen verschiedene Glykoproteine).

Folglich bezieht sich der Begriff des Glykoproteingehalts des erfindungsgemäß eingesetzten Gummi arabicum (d. h. des Gehalts an Glykoproteinen im erfindungsgemäß eingesetzten Gummi arabicum), wie er im Rahmen der vorliegenden Erfindung verwendet wird, auf den Gesamtgehalt aller im erfindungsgemäß eingesetzten Gummi arabicum vorhandener Glykoproteine.

Der Glykoproteingehalt des erfindungsgemäß eingesetzten Gummi arabicum bezieht sich zudem auf das jeweilige Glykoprotein-Molekül als Ganzes bzw. also solches (d. h. nicht nur auf dessen reinen Gehalt an Protein(en) bzw. Proteingruppen).

Der Glykoproteingehalt des erfindungsgemäß eingesetzten Gummi arabicum kann mit herkömmlichen und dem Fachmann an sich bekannten bzw. geläufigen Analyse- bzw. Bestimmungsmethoden bestimmt werden, insbesondere chromatographisch, vorzugsweise mittels HPLC-Chromatographie oder SEC-Methode, insbesondere SEC-MALLS-Methode (Size-Exclusion Chromatography coupled to Multi-Angle Laser-light Scattering). Dies ist dem Fachmann hinlänglich bekannt. Diesbezüglich kann auf einschlägige Fachliteratur und Fachpublikationen verwiesen werden, insbesondere L. Lopez-Torrez et al. "Acacia senegal vs. Acacia seyal gums - Part 1: Composition and structure of hyperbranched plant exudates", Food Hydrokolloids, Volume 51, Oktober 2015, Seiten 41 bis 53 (vgl. hierzu auch URL bzw. Link: https://doi.org/10.1016/j.foodhyd.2015.04.019)*,* sowie die dort referierte Literatur (vgl. insbesondere L. Picton et al. "Analysis of a complex polysaccharide (gum arabic) by multi-angle laser light scattering coupled on-line to size exclusion chromatography and flow field flow fractionation", Carbohydrate Polymers, Volume 42, Issue 1, Mai 2000, Seiten 23 bis 31; vgl. hierzu auch URL bzw. Link: https://doi.org/10.1016/S0144-8617(99)00139-3)*,* wobei die vorgenannten Literaturstellen und deren gesamte Offenbarung bezüglich der Bestimmung des Glykoproteingehalts hiermit durch Bezugnahme vollumfänglich eingeschlossen sind.

Nur des Verständnisses und der Klarheit wegen wird in diesem Zusammenhang auch noch auf Folgendes hingewiesen: Von der erfindungsgemäß verwendeten charakterisierenden Kenngröße des Glykoproteingehalts zu unterscheiden ist die Kenngröße des Proteingehalts (wobei der Proteingehalt insbesondere im Nahrungsmittelbereich über die Bestimmung des Gesamtstickstoffgehalts mit anschließender Umrechnung über einen Stickstoff-Umrechnungsfaktor [typischerweise 6,25 bzw. 6,38] ermittelt wird, wobei im Allgemeinen dieser Proteingehalt signifikant geringer als die erfindungsgemäß verwendete Kenngröße des Glykoproteingehalts ausfällt, da die Glykoproteinmoleküle nur zu einem und mitunter nur relativ geringem Molekülanteil aus Proteingruppen gebildet sind, wohingegen der übrige und oftmals überwiegende Molekülanteil durch Kohlenhydrat- bzw. Saccharidreste oder -gruppen gebildet wird). Der Bestimmung des Proteingehalts liegt auch eine eigenständige Bestimmungsmethode zugrunde: Für weitergehende diesbezügliche Einzelheiten zur Bestimmung des Proteingehalts kann insbesondere verwiesen werden auf: DIN EN ISO 8968-2, Ausgabe Juni 2002, sowie auf die amtliche Sammlung von Verfahren zur Probenahme und zur Untersuchung von Lebensmitteln, Bedarfsgegenständen, kosmetischen Mitteln, Tabakerzeugnissen und Futtermitteln (ASU): BVL L 01.00-10/2:2016-03, Untersuchung von Lebensmitteln - Bestimmung des Stickstoffgehaltes in Milch - Teil 2: Blockaufschluss-Verfahren (Makroverfahren) (Übernahme der gleichnamigen Deutschen Norm DIN EN ISO 8968-2, Ausgabe Juni 2002); siehe auch "IDF-Faktenblatt (IDF Fact Sheet) zur Proteinbestimmung" (Verband der Deutschen Milchwirtschaft e.V. - VDM), Ausgabe Mai 2013.

Im Allgemeinen weisen die im erfindungsgemäß eingesetzten Gummi arabicum vorhandenen Glykoproteine ein gewichtsmittleres Molekulargewicht M̅_{w} im Bereich von 10⁴ g/mol bis 10⁷ g/mol, insbesondere im Bereich von 1 x 10⁵ g/mol bis 9 x 10⁶ g/mol, vorzugsweise 1,5 x 10⁵ g/mol bis 6 x 10⁶ g/mol, auf. Hierdurch lassen sich besonders gute Eigenschaften, insbesondere Anwendungseigenschaften, der erfindungsgemäßen Zusammensetzung und hohe MKT-Beladungen erzielen.

Im Allgemeinen kann es erfindungsgemäß vorgesehen sein, dass der Glykoproteingehalt des Gummi arabicum zumindest teilweise, insbesondere überwiegend, durch Arabinogalactanproteine (synonym auch als "AGPs" bezeichnet) bereitgestellt wird und/oder gebildet wird. Auf diese Weise können sich besonders gute Eigenschaften, insbesondere Anwendungseigenschaften, der erfindungsgemäßen Zusammensetzung und hohe MKT-Beladungen erzielen lassen.

Insbesondere ist es erfindungsgemäß bevorzugt, wenn der Glykoproteingehalt des Gummi arabicum zu wenigstens 50 %, insbesondere zu wenigstens 60 %, vorzugsweise zu wenigstens 65 %, durch Arabinogalactanproteine bereitgestellt wird und/oder gebildet wird, bezogen auf den Glykoproteingehalt.

Weiterhin ist es erfindungsgemäß insbesondere bevorzugt, wenn der Glykoproteingehalt des Gummi arabicum zu 50 % bis 99 %, insbesondere zu 60 % bis 98 %, vorzugsweise zu 65 % bis 95 %, durch Arabinogalactanproteine (synonym auch als "AGPs" bezeichnet) bereitgestellt wird und/oder gebildet wird, bezogen auf den Glykoproteingehalt.

Die mittelkettigen Triglyceride (MKTs bzw. *Medium-Chain Triglycerides* bzw. MCTs), stellen im Allgemeinen Triglyceride dar, welche mittelkettige Fettsäuren enthalten. Zu den mittelkettigen Fettsäuren zählen Capron-, Capryl-, Caprin- und Laurinsäure gesättigte Fettsäuren. Bei den vorgenannten Säuren handelt es sich um gesättigte geradkettige bzw. unverzweigte C₆-, C₈-, C₁₀- bzw. C₁₂- Carbonsäuren. Mittelkettige Triglyceride können im industriellen Maßstab z. B. durch Hydrolyse von pflanzlichen Fetten, wie z. B. Kokosfett und Palmkernöl, Fraktionierung der mittelkettigen Fettsäuren und anschließender Veresterung mit Glycerin gewonnen werden. Die erfindungsgemäß eingesetzten mittelkettigen Triglyceride (MKTs) sind im Allgemeinen insbesondere zumindest im Wesentlichen farblos und zudem geruchs- und geschmacksneutral.

Für weitere Informationen zu mittelkettigen Trigyceriden (MKTs bzw. MCTs) kann auch verwiesen werden auf Römpp Chemie Lexikon, 10. Auflage, Band 4, Georg Thieme Verlag, Stuttgart/New York, 1998, Seite 1560, Stichwort "MCT (MKT)", sowie die dort jeweils referierte Literatur.

Nachfolgend wird die erfindungsgemäße Zusammensetzung anhand von weiteren Ausgestaltungen und Ausführungsformen der vorliegenden Erfindung weiterführend beschrieben:
Wie zuvor angeführt, wird im Rahmen der vorliegenden Erfindung ein spezielles Gummi arabicum mit hohem Glykoproteingehalt eingesetzt. Wie zuvor angeführt, kann hierdurch eine besonders hohe Beladung des zugrundeliegenden polysaccharidbasierten Trägermaterials mit mittelkettigen Triglyceriden (MKTs) realisiert werden.

Im Rahmen der vorliegenden Erfindung kann es sich in Bezug auf den Glykoproteingehalt insbesondere wie folgt verhalten:
- So kann das Gummi arabicum einen Glykoproteingehalt, bezogen auf das Gummi arabicum, von mindestens 4,5 Gew.-%, insbesondere mindestens 5 Gew.-%, vorzugsweise mindestens 5,5 Gew.-%, bevorzugt mindestens 6 Gew.-%, aufweisen.
- Zudem weist das Gummi arabicum einen Glykoproteingehalt, bezogen auf das Gummi arabicum, von höchstens 25 Gew.-% auf.
- Weiterhin kann das Gummi arabicum einen Glykoproteingehalt, bezogen auf das Gummi arabicum, von höchstens 22 Gew.-%, insbesondere höchstens 20 Gew.-%, vorzugsweise höchstens 18 Gew.-%, bevorzugt höchstens 16 Gew.-%, aufweisen.
- Erfindungsgemäß kann es insbesondere vorgesehen sein, dass das Gummi arabicum einen Glykoproteingehalt, bezogen auf das Gummi arabicum, im Bereich von 4,5 Gew.-% bis 22 Gew.-%, insbesondere im Bereich von 5 Gew.-% bis 20 Gew.-%, vorzugsweise im Bereich von 5,5 Gew.-% bis 18 Gew.-%, bevorzugt im Bereich von 6 Gew.-% bis 16 Gew.-%, aufweist.

Wie zuvor angeführt, können insbesondere infolge des hohen Glykoproteingehalts erfindungsgemäß besonders hohe Beladungen der Zusammensetzung bzw. der Partikel mit dem zugrundeliegenden polysaccharidbasierten Trägermaterial erhalten werden.

Der erfindungsgemäß angeführte Triglyceridgehalt, wie er für die Zusammensetzung nach der Erfindung vorliegend angeführt wird, bezieht sich vorliegend auf den Gehalt an dem mindestens einen mittelkettigen Triglycerid, insbesondere den Gehalt an dem mindestens einen Triglycerid mindestens einer linearen gesättigten C₆-, C₈-, C₁₀- oder C₁₂- Carbonsäure.

Diesbezüglich kann es sich erfindungsgemäß insbesondere wie folgt verhalten:
- So kann die Zusammensetzung, bezogen auf die Zusammensetzung, einen Triglyceridgehalt von mindestens 55 Gew.-%, insbesondere mindestens 57,5 Gew.-%, vorzugsweise mindestens 60 Gew.-%, bevorzugt mindestens 65 Gew.-%, aufweisen.
- Erfindungsgemäß weist die Zusammensetzung, bezogen auf die Zusammensetzung, einen Triglyceridgehalt von höchstens 90 Gew.-% auf.
- Weiterführend kann die Zusammensetzung nach der Erfindung, bezogen auf die Zusammensetzung, einen Triglyceridgehalt von höchstens 87,5 Gew.-%, insbesondere höchstens 85 Gew.-%, vorzugsweise höchstens 82,5 Gew.-%, bevorzugt höchstens 80 Gew.-%, aufweisen.
- Insbesondere kann es erfindungsgemäß vorgesehen sein, dass die Zusammensetzung, bezogen auf die Zusammensetzung, einen Triglyceridgehalt im Bereich von 55 Gew.-% bis 87,5 Gew.-%, insbesondere im Bereich von 57,5 Gew.-% bis 85 Gew.-%, vorzugsweise im Bereich von 60 Gew.-% bis 82,5 Gew.-%, bevorzugt im Bereich von 65 Gew.-% bis 80 Gew.-%, aufweist.

Darüber hinaus kann die die erfindungsgemäße Zusammensetzung die nachfolgenden Gehalte an Gummi arabicum aufweisen:
- So kann es erfindungsgemäß vorgesehen sein, dass die Zusammensetzung, bezogen auf die Zusammensetzung, einen Gehalt an Gummi arabicum von höchstens 50 Gew.-%, vorzugsweise im Bereich von 10 Gew.-% bis 50 Gew.-%, aufweist.
- Zudem kann es im Rahmen der vorliegenden Erfindung auch vorgesehen sein, dass die Zusammensetzung, bezogen auf die Zusammensetzung, einen Gehalt an Gummi arabicum von höchstens 45 Gew.-%, insbesondere höchstens 42,5 Gew.-%, vorzugsweise höchstens 40 Gew.-%, bevorzugt höchstens 35 Gew.-%, aufweist.
- Insbesondere kann es erfindungsgemäß vorgesehen sein, dass die Zusammensetzung, bezogen auf die Zusammensetzung, einen Gehalt an Gummi arabicum im Bereich von 12,5 Gew.-% bis 45 Gew.-%, insbesondere im Bereich von 15 Gew.-% bis 42,5 Gew.-%, vorzugsweise im Bereich von 17,5 Gew.-% bis 40 Gew.-%, bevorzugt im Bereich von 20 Gew.-% bis 35 Gew.-%, aufweist.

Weiterführend kommt auch den nachfolgend angeführten Kombinationen an Gehalten bzw. Mengen der jeweiligen Inhaltsstoffe bzw. Komponenten der erfindungsgemäßen Zusammensetzung eine große Bedeutung zu. Insbesondere kann die erfindungsgemäße Zusammensetzung mit zunehmendem Glykoproteingehalt des Gummi arabicum auch den folgenden Gehalt bzw. Menge an mittleren Triglyceriden (MKTs) aufweisen:
So kann es erfindungsgemäß vorgesehen sein,
- dass das Gummi arabicum einen Glykoproteingehalt, bezogen auf das Gummi arabicum, von mindestens 4,5 Gew.-% aufweist und wobei die Zusammensetzung, bezogen auf die Zusammensetzung, einen Triglyceridgehalt von mindestens 55 Gew.-% aufweist; oder aber
- dass das Gummi arabicum einen Glykoproteingehalt, bezogen auf das Gummi arabicum, von mindestens 5 Gew.-% aufweist und dass die Zusammensetzung, bezogen auf die Zusammensetzung, einen Triglyceridgehalt von mindestens 57,5 Gew.-% aufweist; oder aber
- dass das Gummi arabicum einen Glykoproteingehalt, bezogen auf das Gummi arabicum, von mindestens 5,5 Gew.-% aufweist und dass die Zusammensetzung, bezogen auf die Zusammensetzung, einen Triglyceridgehalt von mindestens 60 Gew.-% aufweist; oder aber
- dass das Gummi arabicum einen Glykoproteingehalt, bezogen auf das Gummi arabicum, von mindestens 6 Gew.-% aufweist und dass die Zusammensetzung, bezogen auf die Zusammensetzung, einen Triglyceridgehalt von mindestens 65 Gew.-% aufweist.

Erfindungsgemäß ist es vorgesehen,
- dass das Gummi arabicum einen Glykoproteingehalt, bezogen auf das Gummi arabicum, von höchstens 25 Gew.-% aufweist und wobei die Zusammensetzung, bezogen auf die Zusammensetzung, einen Triglyceridgehalt von höchstens 90 Gew.-% aufweist.

Zudem kann es erfindungsgemäß es vorgesehen sein,
- dass das Gummi arabicum einen Glykoproteingehalt, bezogen auf das Gummi arabicum, von höchstens 22 Gew.-% aufweist und wobei die Zusammensetzung, bezogen auf die Zusammensetzung, einen Triglyceridgehalt von höchstens 87,5 Gew.-% aufweist; oder aber
- dass das Gummi arabicum einen Glykoproteingehalt, bezogen auf das Gummi arabicum, von höchstens 20 Gew.-% aufweist und wobei die Zusammensetzung, bezogen auf die Zusammensetzung, einen Triglyceridgehalt von höchstens 85 Gew.-% aufweist; oder aber
- dass das Gummi arabicum einen Glykoproteingehalt, bezogen auf das Gummi arabicum, von höchstens 18 Gew.-% aufweist und wobei die Zusammensetzung, bezogen auf die Zusammensetzung, einen Triglyceridgehalt von höchstens 82,5 Gew.-% aufweist; oder aber
- dass das Gummi arabicum einen Glykoproteingehalt, bezogen auf das Gummi arabicum, von höchstens 16 Gew.-% aufweist und wobei die Zusammensetzung, bezogen auf die Zusammensetzung, einen Triglyceridgehalt von höchstens 80 Gew.-% aufweist.

Im Rahmen der vorliegenden Erfindung ist es vorgesehen,
- dass das Gummi arabicum einen Glykoproteingehalt, bezogen auf das Gummi arabicum, im Bereich von 4 Gew.-% bis 25 Gew.-% aufweist und wobei die Zusammensetzung, bezogen auf die Zusammensetzung, einen Triglyceridgehalt im Bereich von 50 Gew.-% bis 90 Gew.-% aufweist.

Weiterführend kann es im Rahmen der vorliegenden Erfindung auch vorgesehen sein,
- dass das Gummi arabicum einen Glykoproteingehalt, bezogen auf das Gummi arabicum, im Bereich von 4,5 Gew.-% bis 22 Gew.-% aufweist und wobei die Zusammensetzung, bezogen auf die Zusammensetzung, einen Triglyceridgehalt im Bereich von 55 Gew.-% bis 87,5 Gew.-% aufweist; oder aber
- dass das Gummi arabicum einen Glykoproteingehalt, bezogen auf das Gummi arabicum, im Bereich von 5 Gew.-% bis 20 Gew.-% aufweist und wobei die Zusammensetzung, bezogen auf die Zusammensetzung, einen Triglyceridgehalt im Bereich von 57,5 Gew.-% bis 85 Gew.-% aufweist; oder aber
- dass das Gummi arabicum einen Glykoproteingehalt, bezogen auf das Gummi arabicum, im Bereich von 5,5 Gew.-% bis 18 Gew.-% aufweist und wobei die Zusammensetzung, bezogen auf die Zusammensetzung, einen Triglyceridgehalt im Bereich von 60 Gew.-% bis 82,5 Gew.-% aufweist; oder aber
- dass das Gummi arabicum einen Glykoproteingehalt, bezogen auf das Gummi arabicum, im Bereich von 6 Gew.-% bis 16 Gew.-% aufweist und wobei die Zusammensetzung, bezogen auf die Zusammensetzung, einen Triglyceridgehalt im Bereich von 65 Gew.-% bis 80 Gew.-% aufweist.

Was das erfindungsgemäß in bevorzugter Weise eingesetzte Gummi arabicum anbelangt, so ist hierzu auch Folgendes anzuführen:
Insbesondere kann das Gummi arabicum naturbasiert und/oder natürlichen Ursprungs, insbesondere pflanzlichen Ursprungs, sein.

Weiterhin kann das Gummi arabicum auf einem pflanzlichen Exsudat basieren bzw. aus einem pflanzliches Exsudat gewonnen sein, insbesondere auf Basis bzw. aus einem pflanzlichen Exsudat von Akazienbäumen, insbesondere Gummiarabicum-Baum (*Senegalia senegal* bzw. synonym *Acacia senegal* oder Senegal-Akazie).

Erfindungsgemäß kann es insbesondere vorgesehen sein, dass das Gummi arabicum aus Akazienbäumen, insbesondere Gummiarabicum-Baum (*Senegalia senegal* bzw. synonym *Acacia senegal* oder Senegal-Akazie), gewonnen ist bzw. hiervon stammt. Erfindungsgemäß kann es zudem vorgesehen sein, dass das Gummi arabicum aus einem Exsudat von Akazienbäumen, insbesondere Gummiarabicum-Baum (*Senegalia senegal* bzw. synonym *Acacia senegal* oder Senegal-Akazie), gewonnen ist bzw. hierauf basiert.

Denn in diesem Zusammenhang hat die Anmelderin gleichermaßen in überraschender Weise gefunden, dass ein spezielles Gummi arabicum, welches auf dem zuvor angeführten Gummiarabicum-Baum (*Senegalia senegal* bzw. synonym *Acacia senegal* oder Senegal-Akazie) zurückgeht, sich in besonderer Weise für das bzw. als polysaccharidbasierte(s) Trägermaterial eignet, und zwar insbesondere auch vor dem Hintergrund, dass das in Rede stehende spezielle Gummi arabicum einen hohen Glykoproteingehalt aufweist.

Gemäß einer erfindungsgemäßen Ausführungsform kann es insbesondere vorgesehen sein, dass der Glykoproteingehalt des Gummi arabicum durch Auswahl des Gummi arabicum, insbesondere durch Auswahl des bzw. der für die Gewinnung des Gummi arabicum, vorzugsweise auf Basis eines Exsudats, eingesetzten (Pflanzen-)Materials bzw. (Pflanzen-)Spezies, gegebenenfalls unter zusätzlicher Anreicherung, insbesondere mittels Fraktionierung, kontrolliert und/oder eingestellt ist. In diesem Zusammenhang kann das (Pflanzen-)Material bzw. die (Pflanzen-)-Spezies insbesondere ausgewählt sein aus der Gruppe von Akazienbäumen, insbesondere Gummiarabicum-Baum (*Senegalia senegal* bzw. synonym *Acacia senegal* oder Senegal-Akazie).

Gemäß einer weiteren Ausgestaltung der vorliegenden Erfindung kann es gleichermaßen vorgesehen sein, dass der Glykoproteingehalt des erfindungsgemäß im Allgemeinen eingesetzten Gummi arabicum durch Anreicherung, insbesondere mittels Fraktionierung, kontrolliert bzw. eingestellt ist.

Die Einstellung des Glykoproteingehalts kann dabei auch kumulativ bzw. in Ergänzung zu der gezielten Auswahl von Gummi arabicum, wie zuvor angeführt, erfolgt sein.

Das im Rahmen der vorliegenden Erfindung eingesetzte Gummi arabicum zeichnet sich auch dadurch aus, dass das Gummi arabicum, insbesondere als Hauptbestandteil, mindestens ein Polysaccharid, vorzugsweise mindestens ein bevorzugt saure(s) Alkali- und/oder Erdalkalisalz(e) der (Poly-)Arabinsäure umfassendes Polysaccharid, besonders bevorzugt mindestens ein bevorzugt verzweigtes Polysaccharid aus L-Arabinose, D-Galaktose, L-Rhamnose und D-Glucuronsäure, insbesondere in einem (Mol-)Verhältnis im Bereich von 2,5 - 4,5 : 0,5 - 5 : 0,1 - 2 : 0,5 - 2,5, vorzugsweise im Bereich von 2,7 - 4 : 1 - 4 : 0,5 - 1,5 : 0,7 - 2, bevorzugt im Bereich von 2,9 - 3,7 : 1,5 - 3,5 : 0,8 - 1,2 : 1 - 1,7, besonders bevorzugt etwa 3,5 : etwa 2,9 : etwa 1,1 : etwa 1,6, weiter bevorzugt 3,5 : 2,9 : 1,1 : 1,6 oder weiter bevorzugt 3 : 3 : 1 : 1, enthält.

Folglich wird erfindungsgemäß insbesondere ein Gummi arabicum eingesetzt, welches einen definierten Polysaccharidanteil aufweist, und zwar auch was dessen konkrete Ausbildung bzw. Beschaffenheit anbelangt. Hierdurch werden die Eigenschaften gegenüber den mittelkettigen Triglyceriden (MKTs) nochmals verbessert, und dies auch im Hinblick auf die Fixierung bzw. Beaufschlagung mit den MKTs. Insbesondere gehen die Polysaccharide auch mit einem definierten Verhalten der erfindungsgemäßen Zusammensetzung bzw. der Partikel auf Basis des polysaccharidbasierten Trägermaterials bei Dispergierung bzw. bei Ausbildung einer Emulsion unter Einbringung der Zusammensetzung in eine Flüssigkeit, insbesondere Wasser, einher.

Darüber hinaus ist auch der Gehalt der Polysaccharide von Relevanz: So kann es erfindungsgemäß beispielsweise vorgesehen sein, dass das Gummi arabicum einen Polysaccharidgehalt, bezogen auf das Gummi arabicum, im Bereich von 75 Gew.-% bis 96 Gew.-% aufweist. Insbesondere kann das Gummi arabicum einen Polysaccharidgehalt, bezogen auf das Gummi arabicum, von mindestens 75 Gew.-% aufweisen. Zudem kann das Gummi arabicum einen Polysaccharidgehalt, bezogen auf das Gummi arabicum, von höchstens 96 Gew.-% aufweisen.

Insbesondere kann erfindungsgemäß auch Folgendes vorgesehen sein:
- So kann das Gummi arabicum einen Polysaccharidgehalt, bezogen auf das Gummi arabicum, im Bereich von 78 Gew.-% bis 95,5 Gew, insbesondere im Bereich von 80 Gew.-% bis 95 Gew.-%, vorzugsweise im Bereich von 82 Gew.-% bis 94,5 Gew.-%, bevorzugt im Bereich von 84 Gew.-% bis 94 Gew.-%, aufweisen.
- Zudem kann das Gummi arabicum einen Polysaccharidgehalt, bezogen auf das Gummi arabicum, von mindestens 78 Gew.-%, insbesondere mindestens 80 Gew.-%, vorzugsweise mindestens 82 Gew.-%, bevorzugt mindestens 84 Gew.-%, aufweisen.
- Weiterhin kann das Gummi arabicum einen Polysaccharidgehalt, bezogen auf das Gummi arabicum, von höchstens 95,5 Gew.-%, insbesondere höchstens 95 Gew.-%, vorzugsweise höchstens 94,5 Gew.-%, bevorzugt höchstens 94 Gew.-%, aufweisen.

Was die im Rahmen der vorliegenden Erfindung eingesetzten mittelkettigen Triglyceride (MKTs) anbelangt, so kann erfindungsgemäß insbesondere Folgendes vorgesehen sein:
Insbesondere kann das mittelkettige Triglycerid (MKT) ein Triglycerid einer C₈-Carbonsäure, insbesondere einer linearen gesättigten C₈-Carbonsäure, sein. In diesem Zusammenhang kann es insbesondere vorgesehen sein, dass das mittelkettige Triglycerid (MKT) Tricaprylin ist.

Weiterhin kann das das mittelkettige Triglycerid (MKT) ein gemischtes Triglycerid von C₈- und C₁₀-Carbonsäure, insbesondere ein gemischtes Triglycerid von jeweils linearer gesättigter C₈- und C₁₀-Carbonsäure, sein. Diesbezüglich kann das das mittelkettige Triglycerid (MKT) ein C₈ : C₁₀-Anteilverhältnis im Bereich von 30 % - 90 % : 70 % - 10 %, insbesondere im Bereich 50 % - 80 % : 50 % - 20 %, vorzugsweise im Bereich von 55 % - 75 % : 45 % - 25 %, aufweisen.

Die vorgenannten mittelkettigen Triglyceride (MKTs) führen zu besonders guten Anwendungs- und Handhabungseigenschaften auch in Bezug auf Produkte bzw. Erzeugnisse im kosmetischen, pharmazeutischen, bzw. Nahrungsmittelbereich.

Im Rahmen der vorliegenden Erfindung können infolge der hohen Beladung mit MTKs insgesamt Zusammensetzungen mit hohem Ketokörper-Index bereitgestellt werden. Der Ketokörper-Index kann dabei auch als Maß für die Beladung bzw. Ausrüstung der erfindungsgemäßen Zusammensetzung mit den mittelkettigen Triglyceriden (MKTs) herangezogen werden.

Die nachfolgend angeführten Werte beziehen sich insbesondere auf die Voraussetzung bzw. Gegebenheit, dass bei physiologischer Verstoffwechselung im menschlichen Körper pro Molekül eines mittelkettigen Triglycerids insgesamt 6 Moleküle Ketokörper (vorliegend berechnet als 3-Hydroxybuttersäure (3-BHB)) gebildet werden.

In diesem Zusammenhang verhält es sich physiologisch insbesondere derart - ohne sich hierauf berufen oder beschränken zu wollen -, dass mittelkettige Triglyceride insbesondere in der Leber unter Freisetzung von Ketokörpern (Ketonkörpern) oxidiert werden. Der Ketokörper-Index stellt dabei auch auf die hervorragenden ernährungsphysiologischen Eigenschaften der erfindungsgemäßen Zusammensetzung ab. Zudem geht ein hoher Ketokörper-Index auch mit hervorragenden Handhabungs- und Anwendungseigenschaften für kosmetische bzw. pharmazeutische Produkte unter Einsatz der erfindungsgemäßen Zusammensetzung einher.

Für den Ketokörper-Index gilt vor diesem Hintergrund insbesondere auch Folgendes:
- Erfindungsgemäß kann die Zusammensetzung nach der Erfindung einen Ketokörper-Index (ketogener Index), berechnet als theoretische Anzahl Millimol (mmol) 3-Hydroxybuttersäure (3-BHB) pro ein Gramm der Zusammensetzung, von mindestens 0,5 mmol/g aufweisen.
- Insbesondere kann die Zusammensetzung einen Ketokörper-Index (ketogener Index), berechnet als theoretische Anzahl Millimol (mmol) 3-Hydroxybuttersäure (3-BHB) pro ein Gramm der Zusammensetzung, von mindestens 1 mmol/g, insbesondere mindestens 2 mmol/g, vorzugsweise mindestens 3 mmol/g, bevorzugt mindestens 4 mmol/g, aufweisen.
- Erfindungsgemäß kann die Zusammensetzung nach der Erfindung einen Ketokörper-Index (ketogener Index), berechnet als theoretische Anzahl Millimol (mmol) 3-Hydroxybuttersäure (3-BHB) pro ein Gramm der Zusammensetzung, im Bereich von 0,5 mmol/g bis 50 mmol/g aufweisen.
- Insbesondere kann die Zusammensetzung einen Ketokörper-Index (ketogener Index), berechnet als theoretische Anzahl Millimol (mmol) 3-Hydroxybuttersäure (3-BHB) pro ein Gramm der Zusammensetzung, im Bereich von 1 mmol/g bis 45 mmol/g, insbesondere im Bereich von 2 mmol/g bis 40 mmol/g, vorzugsweise im Bereich von 3 mmol/g bis 30 mmol/g, bevorzugt im Bereich von 4 mmol/g bis 25 mmol/g, aufweisen.

Erfindungsgemäß können somit insgesamt hohe Werte für den Ketokörper-Index der erfindungsgemäßen Zusammensetzung bereitgestellt werden. Insbesondere liegt in Bezug auf die erfindungsgemäße Zusammensetzung auch von daher eine hohe Dichte bzw. hohe Beladung in Bezug auf die mittelkettigen Triglyceride (MKTs) vor. Auf dieser Basis können auch entsprechende Erzeugnisse bzw. Produkte, die mit der erfindungsgemäßen Zusammensetzung ausgerüstet sind, einen hohen Ketokörper-Index aufweisen. Dies ist sowohl im kosmetischen bzw. pharmazeutischen Bereich, insbesondere in Bezug auf die Handhabungs- und Anwendungseigenschaften der erfindungsgemäßen Zusammensetzung, als auch im Bereich der Nahrungsmittel von Vorteil, insbesondere in Bezug auf die ernährungsphysiologischen Eigenschaften der erfindungsgemäßen Zusammensetzung.

Im Hinblick auf die erfindungsgemäße Zusammensetzung können insbesondere folgende Wertekombinationen für den Ketokörper-Index, den Glykoproteingehalt des Gummi arabicum und den Triglyceridgehalt eingestellt bzw. vorgegeben werden:
So kann es erfindungsgemäß vorgesehen sein,
- dass die Zusammensetzung einen Ketokörper-Index (ketogener Index), berechnet als theoretische Anzahl Millimol (mmol) 3-Hydroxybuttersäure (3-BHB) pro ein Gramm der Zusammensetzung, von mindestens 0,5 mmol/g aufweist und wobei das Gummi arabicum einen Glykoproteingehalt, bezogen auf das Gummi arabicum, von mindestens 4 Gew.-% aufweist und wobei die Zusammensetzung, bezogen auf die Zusammensetzung, einen Triglyceridgehalt von mindestens 50 Gew.-% aufweist; oder aber
- dass die Zusammensetzung einen Ketokörper-Index (ketogener Index), berechnet als theoretische Anzahl Millimol (mmol) 3-Hydroxybuttersäure (3-BHB) pro ein Gramm der Zusammensetzung, von mindestens 1 mmol/g aufweist und wobei das Gummi arabicum einen Glykoproteingehalt, bezogen auf das Gummi arabicum, von mindestens 4,5 Gew.-% aufweist und wobei die Zusammensetzung, bezogen auf die Zusammensetzung, einen Triglyceridgehalt von mindestens 55 Gew.-% aufweist; oder aber
- dass die Zusammensetzung einen Ketokörper-Index (ketogener Index), berechnet als theoretische Anzahl Millimol (mmol) 3-Hydroxybuttersäure (3-BHB) pro ein Gramm der Zusammensetzung, von mindestens 2 mmol/g aufweist und wobei das Gummi arabicum einen Glykoproteingehalt, bezogen auf das Gummi arabicum, von mindestens 5 Gew.-% aufweist und wobei die Zusammensetzung, bezogen auf die Zusammensetzung, einen Triglyceridgehalt von mindestens 57,5 Gew.-% aufweist; oder aber
- dass die Zusammensetzung einen Ketokörper-Index (ketogener Index), berechnet als theoretische Anzahl Millimol (mmol) 3-Hydroxybuttersäure (3-BHB) pro ein Gramm der Zusammensetzung, von mindestens 3 mmol/g aufweist und wobei das Gummi arabicum einen Glykoproteingehalt, bezogen auf das Gummi arabicum, von mindestens 5,5 Gew.-% aufweist und wobei die Zusammensetzung, bezogen auf die Zusammensetzung, einen Triglyceridgehalt von mindestens 60 Gew.-% aufweist; oder aber
- dass die Zusammensetzung einen Ketokörper-Index (ketogener Index), berechnet als theoretische Anzahl Millimol (mmol) 3-Hydroxybuttersäure (3-BHB) pro ein Gramm der Zusammensetzung, von mindestens 4 mmol/g aufweist und wobei das Gummi arabicum einen Glykoproteingehalt, bezogen auf das Gummi arabicum, von mindestens 6 Gew.-% aufweist und wobei die Zusammensetzung, bezogen auf die Zusammensetzung, einen Triglyceridgehalt von mindestens 65 Gew.-% aufweist.

Zudem kann es erfindungsgemäß auch vorgesehen sein,
- dass die Zusammensetzung einen Ketokörper-Index (ketogener Index), berechnet als theoretische Anzahl Millimol (mmol) 3-Hydroxybuttersäure (3-BHB) pro ein Gramm der Zusammensetzung, im Bereich von 0,5 mmol/g bis 50 mmol/g aufweist und wobei das Gummi arabicum einen Glykoproteingehalt, bezogen auf das Gummi arabicum, im Bereich von 4 Gew.-% bis 25 Gew.-% aufweist und wobei die Zusammensetzung, bezogen auf die Zusammensetzung, einen Triglyceridgehalt im Bereich von 50 Gew.-% bis 90 Gew.-% aufweist; oder aber
- dass die Zusammensetzung einen Ketokörper-Index (ketogener Index), berechnet als theoretische Anzahl Millimol (mmol) 3-Hydroxybuttersäure (3-BHB) pro ein Gramm der Zusammensetzung, im Bereich von 1 mmol/g bis 45 mmol/g aufweist und wobei das Gummi arabicum einen Glykoproteingehalt, bezogen auf das Gummi arabicum, im Bereich von 4,5 Gew.-% bis 22 Gew.-% aufweist und wobei die Zusammensetzung, bezogen auf die Zusammensetzung, einen Triglyceridgehalt im Bereich von 55 Gew.-% bis 87,5 Gew.-% aufweist; oder aber
- dass die Zusammensetzung einen Ketokörper-Index (ketogener Index), berechnet als theoretische Anzahl Millimol (mmol) 3-Hydroxybuttersäure (3-BHB) pro ein Gramm der Zusammensetzung, im Bereich von 2 mmol/g bis 40 mmol/g aufweist und wobei das Gummi arabicum einen Glykoproteingehalt, bezogen auf das Gummi arabicum, im Bereich von 5 Gew.-% bis 20 Gew.-% aufweist und wobei die Zusammensetzung, bezogen auf die Zusammensetzung, einen Triglyceridgehalt im Bereich von 57,5 Gew.-% bis 85 Gew.-% aufweist; oder aber
- dass die Zusammensetzung einen Ketokörper-Index (ketogener Index), berechnet als theoretische Anzahl Millimol (mmol) 3-Hydroxybuttersäure (3-BHB) pro ein Gramm der Zusammensetzung, im Bereich von 3 mmol/g bis 30 mmol/g aufweist und wobei das Gummi arabicum einen Glykoproteingehalt, bezogen auf das Gummi arabicum, im Bereich von 5,5 Gew.-% bis 18 Gew.-% aufweist und wobei die Zusammensetzung, bezogen auf die Zusammensetzung, einen Triglyceridgehalt im Bereich von 60 Gew.-% bis 82,5 Gew.-% aufweist; oder aber
- dass die Zusammensetzung einen Ketokörper-Index (ketogener Index), berechnet als theoretische Anzahl Millimol (mmol) 3-Hydroxybuttersäure (3-BHB) pro ein Gramm der Zusammensetzung, im Bereich von 4 mmol/g bis 25 mmol/g aufweist und wobei das Gummi arabicum einen Glykoproteingehalt, bezogen auf das Gummi arabicum, im Bereich von 6 Gew.-% bis 16 Gew.-% aufweist und wobei die Zusammensetzung, bezogen auf die Zusammensetzung, einen Triglyceridgehalt im Bereich von 65 Gew.-% bis 80 Gew.-% aufweist.

Darüber hinaus kann es erfindungsgemäß vorgesehen sein, dass die Zusammensetzung nach der Erfindung mindestens einen Emulgator enthält.

Die Verwendung eines Emulgators ist insbesondere mit dem Vorteil verbunden, dass zum einen die Fixierung bzw. Beaufschlagung der Partikel der Zusammensetzung mit dem mittelkettigen Triglycerid nochmals verbessert werden kann. Zum anderen wird auch das Dispergier- bzw. Emulgierverhalten der erfindungsgemäßen Zusammensetzung insbesondere bei Einbringen in eine Flüssigkeit, wie Wasser, weiterführend verbessert. Insbesondere können auf dieser Basis besonders homogene Emulsionen mit stabiler Integration auch der mittelkettigen Triglyceride (MKTs) erhalten werden.

In diesem Zusammenhang kann es insbesondere vorgesehen sein, dass die Zusammensetzung den Emulgator in einer Menge von mindestens 0,0001 Gew.-%, insbesondere mindestens 0,001 Gew.-%, vorzugsweise mindestens 0,01 Gew.-%, bezogen auf die Zusammensetzung, enthält. Zudem kann es erfindungsgemäß vorgesehen sein, dass die Zusammensetzung den Emulgator in einer Menge von höchstens 5 Gew.-%, insbesondere höchstens 2,5 Gew.-%, vorzugsweise höchstens 1 Gew.-%, bezogen auf die Zusammensetzung, enthält. Weiterführend kann es erfindungsgemäß auch vorgesehen sein, dass die Zusammensetzung den Emulgator in einer Menge im Bereich von 0,0001 Gew.-% bis 5 Gew.-%, insbesondere im Bereich von 0,001 Gew.-% bis 2,5 Gew.-%, vorzugsweise im Bereich von 0,01 Gew.-% bis 1 Gew.-%, bezogen auf die Zusammensetzung, enthält.

Die vorstehenden Mengenangaben bezüglich des optionalen Emulgators beziehen selbstverständlich nur auf die erfindungsgemäße Zusammensetzung als solche. Es versteht sich aber von selbst, dass hierauf basierende, d. h. weiterführende und die erfindungsgemäße Zusammensetzung enthaltende Produkte (wie z. B. Nahrungsmittel-, Kosmetik- und pharmazeutische Produkte etc.) darüber hinaus zusätzliche Emulgatoren bzw. Emulgatorkomponenten (wie z. B. Tenside etc.) enthalten können, so dass dann in diesen weiterführenden, die erfindungsgemäße Zusammensetzung enthaltenden Produkten deutlich höhere Gesamtemulgatormengen (z. B. von 7 Gew.-% bis 20 Gew.-% in diesen Formulierungsklassen) vorliegen können (wie z. B. in Shampoos, Duschgelen etc.).

Erfindungsgemäß kann der Emulgator ein amphoterer, anionischer, kationischer oder nichtionischer Emulgator sein.

Im Rahmen der vorliegenden Erfindung kann es insbesondere vorgesehen sein, dass der Emulgator ausgewählt ist aus der Gruppe von Fettsäureglyceriden, insbesondere Mono- und Diglyceriden von Fettsäuren, und ihren Salzen; Citronensäureestern von Fettsäureglyceriden, insbesondere Citronensäureestern von Mono- und Diglyceriden von Fettsäuren, und ihren Salzen; Weinsäureestern und Mono- und Diacetylweinsäureestern von Fettsäureglyceriden, insbesondere Weinsäureestern und Diacetylweinsäureestern von Mono- und Diglyceriden von Fettsäuren, und ihren Salzen; Essigsäureestern von Fettsäureglyceriden, insbesondere Essigsäureestern von Mono- und Diglyceriden von Fettsäuren, und ihren Salzen; Milchsäureestern von Fettsäureglyceriden, insbesondere Milchsäureestern von Mono- und Diglyceriden von Fettsäuren, und ihren Salzen; gemischten Essig- und Weinsäureestern von Fettsäureglyceriden, insbesondere gemischten Essig- und Weinsäureestern von Mono- und Diglyceriden von Fettsäuren, und ihren Salzen; Polyglycerinfettsäureestern, insbesondere Polyglycerinmono-, -di- und -poly-fettsäureestern, vorzugsweise Polyglycerin-Polyricinoleat; Sorbitanestern, insbesondere Polyoxyethylen-Sorbitanestern, vorzugsweise Polyoxyethylen-Sorbitanmonoestern, bevorzugt von Fettsäuren, insbesondere Polyoxyethylen-Sorbitanmonolaurat, Polyoxyethylen-Sorbitanmonooleat, Polyoxyethylen-Sorbitanmonopalmitat, Polyoxyethylen-Sorbitanmonostearat und Polyoxyethylen-Sorbitantristearat; ethoxylierten Fettalkoholen; ethoxylierten Fettsäureglyceriden, insbesondere ethoxylierten Mono- und Diglyceriden von Fettsäuren, und ihren Salzen; Alkylpolyglucosiden und Zuckerglyceriden, insbesondere Cetearylglucosiden; Zuckerestern von Fettsäuren, insbesondere Saccharoseestern von Fettsäuren; modifizierten Stärken; modifizierten Cellulosen; quartären Ammoniumverbindungen (Ester-Quats); Lecithinen; Phospholipiden; Alkylglucosesesquistearaten, insbesondere Methylglucosesesquistearat; Fettalkoholen; Fettsäuren und ihren Salzen und Estern; Siloxanen und Siliconen; Dimethiconen und modifizierten Dimethiconen, insbesondere PEG/PPG-Dimethiconen; Alkalimetallalkylsarcosinaten, insbesondere Natriumalkylsarcosinaten, vorzugsweise Alkalimetalllaurylsarcosinaten; Aminosäureacylestern und ihren Salzen; POE/PPG-Copolymeren; Phosphatestern (Phosphorsäureestern) und ihren Salzen, insbesondere Cetylphosphaten, vorzugsweise Alkalimetallcetylphosphaten, bevorzugt Kaliumcetylphosphat; Polyacrylaten; Castorölen und modifizierten Castorölen, insbesondere PEG-Castorölen und PEG-Castorölfettsäurestern und -difettsäureestern; ethoxylierten Alkylsulfaten bzw. Alkylethersulfaten; sowie deren Kombinationen und Mischungen.

Insbesondere kann es erfindungsgemäß vorgesehen sein, dass der Emulgator an den Partikeln der Zusammensetzung gebunden und/oder fixiert ist; und/oder dass die Zusammensetzung den Emulgator in an den Partikeln gebundener und/oder fixierter Form enthält.

Erfindungsgemäß kann es aber auch vorgesehen sein, dass der Emulgator partikelförmig ausgebildet ist und/oder in Form von Emulgatorpartikeln ausgebildet ist. Insbesondere kann die Zusammensetzung den Emulgator in Form von separaten, insbesondere ungebundenen und/oder freien, Emulgatorpartikeln enthalten.

Die erfindungsgemäße Zusammensetzung zeichnet sich auch durch die folgenden Eigenschaften aus:
So kann die Zusammensetzung in bevorzugter Weise rieselfähig bzw. fließfähig ausgebildet sein. Dies geht mit besonders guten Verarbeitungseigenschaften einher. Insbesondere kann auf dieser Basis eine gute Einarbeitung bzw. Inkorporation der Zusammensetzung in Flüssigkeiten, wie Wasser, erfolgen. Insbesondere sind auch die Dosierungseigenschaften entsprechend verbessert. Auch die vorzugsweise homogene Einarbeitung in pulverförmige Zusammensetzungen wird hierdurch verbessert.

Weiterhin kann es erfindungsgemäß vorgesehen sein, dass die Zusammensetzung nach der Erfindung zumindest im Wesentlichen frei von Verklumpungen bzw. Makroagglomeraten ausgebildet ist. Auch hierdurch wird das Verhalten bei Ausbildung von Dispersionen bzw. Emulsionen verbessert. Insbesondere tritt auf dieser Basis auch keine Klumpenbildung oder dergleichen in den resultierenden Dispersionen bzw. Emulsionen auf. Auch die vorzugsweise homogene Einarbeitung in pulverförmige Zusammensetzungen wird hierdurch verbessert.

Weiterhin kann es erfindungsgemäß insbesondere vorgesehen sein, dass die Zusammensetzung nach der vorliegenden Erfindung zumindest im Wesentlichen frei ist von Partikelverklumpungen oder -aneinanderlagerungen, insbesondere Partikelmakroagglomeraten, mit Größen oberhalb von 5 mm, insbesondere oberhalb von 4,5 mm, vorzugsweise oberhalb von 4 mm, besonders bevorzugt oberhalb von 3,5 mm. Insbesondere verhält es sich erfindungsgemäß vor diesem Hintergrund derart, dass die Partikel der Zusammensetzung zumindest im Wesentlichen nicht zu derartigen Partikelverklumpungen oder -aneinanderlagerungen, insbesondere Partikelmakroagglomeraten, zusammengelagert sind. Dies erhöht die Fließ- und Rieselfähigkeit und verbessert die Ausbildung von Dispersionen bzw. Emulsionen. Darüber hinaus wird hierdurch auch die vorzugsweise homogene Einarbeitung in pulverförmige Zusammensetzungen verbessert.

Darüber hinaus zeichnet sich die erfindungsgemäße Zusammensetzung auch durch eine definierte Restfeuchte aus: Eine definierte Restfeuchte geht insbesondere auch mit einer nochmals verbesserten Riesel- bzw. Fließfähigkeit der Zusammensetzung einher. Zudem wirkt eine definierte bzw. geringe Restfeuchtigkeit einer Klumpenbildung auch bei Lagerung der erfindungsgemäßen Zusammensetzung entgegen, so dass auch auf dieser Basis eine hohe (Lager-)Stabilität der erfindungsgemäßen Zusammensetzung vorliegt. Zudem liegt auch infolge der definierten Restfeuchtigkeit eine verbesserte Emulgierfähigkeit der erfindungsgemäßen Zusammensetzung vor.

So kann es insbesondere vorgesehen sein, dass die Zusammensetzung nach der Erfindung einen Restfeuchtegehalt von höchstens 5 Gew.-%, insbesondere höchstens 4 Gew.-%, vorzugsweise höchstens 3 Gew.-%, bezogen auf die Zusammensetzung, aufweist.

Zudem kann die Zusammensetzung einen Restfeuchtegehalt von mindestens 0,1 Gew.-%, insbesondere mindestens 0,25 Gew.-%, vorzugsweise mindestens 0,5 Gew.-%, bezogen auf die Zusammensetzung, aufweisen.

Weiterhin kann die Zusammensetzung einen Restfeuchtegehalt im Bereich von 0,1 Gew.-% bis 5 Gew.-%, insbesondere im Bereich von 0,25 Gew.-% bis 4 Gew.-%, vorzugsweise im Bereich von 0,5 Gew.-% bis 3 Gew.-%, bezogen auf die Zusammensetzung, aufweisen.

Die erfindungsgemäße Zusammensetzung zeichnet sich zudem durch definierte Partikeleigenschaften aus:
So kann es erfindungsgemäß vorgesehen sein, dass die Partikel der Zusammensetzung eine D90-Partikelgröße, insbesondere einen D90-Teilchendurchmesser, im Bereich von 0,01 mm bis 5 mm, insbesondere im Bereich von 0,05 mm bis 2 mm, vorzugsweise im Bereich von 0,1 mm bis 1 mm, besonders bevorzugt im Bereich von 0,2 mm bis 0,8 mm, aufweisen; insbesondere bestimmt mittels Lichtbeugung, vorzugsweise bestimmt mittels Laserdiffraktometrie gemäß ISO 13320:2020. Die diesbezügliche Bestimmungsmethode ist dem Fachmann als solche wohlbekannt, so dass es diesbezüglich keiner weiteren Ausführungen bedarf.

Weiterhin können die Partikel der Zusammensetzung eine D50-Partikelgröße, insbesondere einen D50-Teilchendurchmesser, im Bereich von 0,01 mm bis 3 mm, insbesondere im Bereich von 0,05 mm bis 2 mm, vorzugsweise im Bereich von 0,1 mm bis 1 mm, besonders bevorzugt im Bereich von 0,15 mm bis 0,75 mm, aufweisen; insbesondere bestimmt mittels Lichtbeugung, vorzugsweise bestimmt mittels Laserdiffraktometrie gemäß ISO 13320:2020. Die diesbezügliche Bestimmungsmethode ist dem Fachmann als solche wohlbekannt, so dass es diesbezüglich keiner weiteren Ausführungen bedarf.

Gemäß einer erfindungsgemäßen Ausführungsform ist es insbesondere vorgesehen, dass die Zusammensetzung nach der Erfindung zumindest im Wesentlichen frei ist von einem weiteren und/oder zusätzlichen Trägermaterial, insbesondere zumindest im Wesentlichen frei ist von einem weiteren und/oder zusätzlichen Trägermaterial für die mittelkettigen Triglyceride (MKTs).

Insbesondere kann es erfindungsgemäß vorgesehen sein, dass die Zusammensetzung zumindest im Wesentlichen kein weiteres und/oder zusätzliches Trägermaterial, insbesondere zumindest im Wesentlichen kein weiteres und/oder zusätzliches Trägermaterial für die mittelkettigen Triglyceride (MKTs), enthält.

Vorzugsweise kann die Zusammensetzung zumindest im Wesentlichen frei sein von einem weiteren und/oder zusätzlichen polysaccharidbasierten Trägermaterial. Erfindungsgemäß kann es vorgesehen sein, dass die Zusammensetzung zumindest im Wesentlichen kein weiteres und/oder zusätzliches polysaccharidbasiertes Trägermaterial enthält.

Insbesondere kann es erfindungsgemäß vorgesehen sein, dass die Zusammensetzung nach der Erfindung zumindest im Wesentlichen frei ist von einem weiteren und/oder zusätzlichen polysaccharidbasierten Trägermaterial auf Basis von Maltodextrin und/oder Stärke, insbesondere modifizierter Stärke.

Insbesondere kann es vorgesehen sein, dass die Zusammensetzung zumindest im Wesentlichen kein weiteres und/oder zusätzliches polysaccharidbasiertes Trägermaterial auf Basis von Maltodextrin und/oder Stärke, insbesondere modifizierter Stärke, enthält.

Diesbezüglich kann es insbesondere vorgesehen sein, dass die erfindungsgemäße Zusammensetzung zumindest im Wesentlichen frei ist von einem weiteren und/oder zusätzlichen polysaccharidbasierten Trägermaterial, welches Maltodextrin und/oder modifizierte Stärke enthält oder hieraus besteht, bzw. ein solches Trägermaterial zumindest im Wesentlichen nicht enthält.

Weiterhin kann die Zusammensetzung zumindest im Wesentlichen frei sein von einem von dem Gummi arabicum verschiedenen Trägermaterial, insbesondere zumindest im Wesentlichen frei sein von einem von dem Gummi arabicum verschiedenen polysaccharidbasierten Trägermaterial, vorzugsweise zumindest im Wesentlichen frei sein von einem von dem Gummi arabicum verschiedenen Gummi arabicum.

Insbesondere kann es erfindungsgemäß vorgesehen sein, dass die Zusammensetzung zumindest im Wesentlichen kein von dem Gummi arabicum verschiedenes Trägermaterial, insbesondere zumindest im Wesentlichen kein von dem Gummi arabicum verschiedenes polysaccharidbasiertes Trägermaterial, vorzugsweise zumindest im Wesentlichen kein von dem Gummi arabicum verschiedenes Gummi arabicum, enthält.

Erfindungsgemäß kann es insbesondere vorgesehen sein, dass die erfindungsgemäße Zusammensetzung außer dem Gummi arabicum kein weiteres polysaccharidbasiertes Trägermaterial aufweist.

Erfindungsgemäß kann es insbesondere auch vorgesehen sein, dass die erfindungsgemäße Zusammensetzung außer Glykoprotein(en) kein weiteres und/oder kein anderweitiges Protein, insbesondere kein Phosphoprotein, vorzugsweise kein Casein, enthält.

Weiterhin kann es erfindungsgemäß insbesondere auch vorgesehen sein, dass die erfindungsgemäße Zusammensetzung kein(e) Phosphoprotein(e), vorzugsweise kein Casein, enthält.

Auf dieser Basis können besonders einheitliche Eigenschaften der erfindungsgemäßen Zusammensetzung im Hinblick auf die Ausrüstung bzw. Fixierung mit den mittelkettigen Triglyceriden (MKTs), einschließlich hoher Beladungsmengen, sowie hinsichtlich der Dispergierbarkeit bzw. Emulgierbarkeit der erfindungsgemäßen Zusammensetzung bereitgestellt werden. Insbesondere werden die mit den weiteren Trägermaterialien einhergehenden Nachteile überkommen, und dies bei gleichzeitig hoher Beladungskapazität der erfindungsgemäßen Zusammensetzung in Bezug auf die mittelkettigen Triglyceride (MKTs). Zudem liegen eine hohe Kompatibilität und gute Verträglichkeit vor.

Gemäß dem vorliegenden Aspekt betrifft die vorliegende Erfindung auch die Zusammensetzung nach der Erfindung, insbesondere partikuläre Zusammensetzung, vorzugsweise pulverförmige Zusammensetzung, bevorzugt mittelkettige Triglyceride (MKTs) enthaltende pulverförmige Zusammensetzung, insbesondere zur Verwendung in kosmetischen, pharmazeutischen oder Nahrungsmittelerzeugnissen, bevorzugt eine wie zuvor definierte Zusammensetzung, wobei die Zusammensetzung nach dem nachfolgend definierten Verfahren nach der Erfindung erhältlich ist bzw. erhalten ist.

Die vorliegende Erfindung ist sowohl gemäß dem ersten Aspekt der vorliegenden Erfindung als auch gemäß den weiteren Aspekten, wie sie nachfolgend beschrieben sind, somit mit einer Vielzahl von Vorteilen und Besonderheiten verbunden, welche die erfindungsgemäße Zusammensetzung einzig und besonders, insbesondere hocheffizient, machen.

Für weitergehende Einzelheiten zu diesem Erfindungsaspekt kann auch Bezug genommen werden auf die nachfolgenden Ausführungen zu den weiteren Erfindungsaspekten, wobei diese Ausführungen gleichermaßen für den vorliegenden Erfindungsaspekt gelten.

Weiterer Gegenstand der vorliegenden Erfindung - gemäß einem **zweiten** Aspekt der vorliegenden Erfindung - ist zudem das Nahrungsmittel-, kosmetisches oder pharmazeutisches Erzeugnis, enthaltend die erfindungsgemäße Zusammensetzung, wie zuvor beschrieben.

In diesem Zusammenhang können unter Einsatz der erfindungsgemäßen Zusammensetzung entsprechende Erzeugnisse bzw. Produkte bereitgestellt werden, welche einen gleichermaßen hohen Gehalt an mittelkettigen Triglyceriden aufweisen können und welche in Form stabiler Dispersionen bzw. Emulsionen vorliegen. Die erfindungsgemäßen Erzeugnisse weisen dabei hervorragende Handhabungs- und Anwendungseigenschaften auf. Zudem weisen diesbezügliche Nahrungsmittel unter Verwendung der erfindungsgemäßen Zusammensetzung hervorragende organoleptische Eigenschaften bei gleichzeitig sehr guten ernährungsphysiologischen Eigenschaften auf.

Gegenstand der vorliegenden Erfindung - gemäß einem **dritten** Aspekt der vorliegenden Erfindung - ist zudem die erfindungsgemäße Verwendung der Zusammensetzung nach der Erfindung, wie zuvor definiert, in einem Nahrungsmittel, kosmetischen oder pharmazeutischen Erzeugnis bzw. zur Herstellung eines Nahrungsmittel-, kosmetischen oder pharmazeutischen Erzeugnisses.

Was den vorgenannten Gegenstand gemäß dem zweiten und dritten Aspekt der vorliegenden Erfindung anbelangt, so kann es sich diesbezüglich insbesondere derart verhalten:
So kann es erfindungsgemäß vorgesehen sein,
- dass das Nahrungsmittel-, kosmetische oder pharmazeutische Erzeugnis die Zusammensetzung in einer Menge von mindestens 0,5 Gew.-%, insbesondere mindestens 1 Gew.-%, vorzugsweise mindestens 3 Gew.-%, bevorzugt mindestens 5 Gew.-%, bezogen auf das Erzeugnis, enthält; bzw.
- dass das Nahrungsmittel-, kosmetische oder pharmazeutische Erzeugnis die Zusammensetzung in einer Menge von höchstens 90 Gew.-%, insbesondere höchstens 80 Gew.-%, vorzugsweise höchstens 70 Gew.-%, bevorzugt höchstens 60 Gew.-%, bezogen auf das Erzeugnis, enthält; bzw.
- dass das Nahrungsmittel-, kosmetische oder pharmazeutische Erzeugnis die Zusammensetzung in einer Menge im Bereich von 0,5 Gew.-% bis 90 Gew.-%, insbesondere im Bereich von 1 Gew.-% bis 80 Gew.-%, vorzugsweise im Bereich von 3 Gew.-% bis 70 Gew.-%, bevorzugt im Bereich von 5 Gew.-% bis 60 Gew.-%, bezogen auf das Erzeugnis, enthält.

Im Allgemeinen kann es erfindungsgemäß auch vorgesehen sein, dass das Nahrungsmittel-, kosmetische oder pharmazeutische Erzeugnis die Zusammensetzung zusammen mit mindestens einem Träger (Exzipient), insbesondere physiologisch verträglichen Träger (Exzipient), enthält. Hierdurch können die Erzeugnisse weiterführend maßgeschneidert bzw. speziell ausgebildet werden, wobei gleichzeitig eine hohe Verträglichkeit gewährleistet ist.

Darüber hinaus zeichnet sich der erfindungsgemäße Gegenstand gemäß dem zweiten und dritten Aspekt der vorliegenden Erfindung auch durch folgende Eigenschaften bzw. Ausbildungen aus:
So kann das Nahrungsmittel-, kosmetische oder pharmazeutische Erzeugnis, insbesondere Nahrungsmittelerzeugnis, ein funktionelles Nahrungsmittel (*Functional Food*)*,* ein *Novel Food,* ein Nahrungsmittelzusatzstoff, ein Nahrungszusatz, ein diätetisches Lebensmittel und/oder diätetisches Nahrungsmittel, ein Power-Snack, ein Appetitzügler oder ein Kraft- und/oder Ausdauersport-Supplement sein.

Darüber hinaus kann das Nahrungsmittel-, kosmetische oder pharmazeutische Erzeugnis, insbesondere kosmetische Erzeugnis, ein Körperpflege- und/oder Körperreinigungsprodukt, insbesondere ein Haut- und/oder Haarpflege- bzw. Haut- und/oder Haarreinigungsprodukt, vorzugsweise Duschgel, Haarshampoo, Badezusatz, Seife, Hautcreme, Lotion, Gel, Creme, Sonnenschutzmittel, Repellent, Gesichtsmaske, Rasierschaum bzw. -seife, Selbstbräunungsmittel, Haarentfernungsmittel, Babypflegeprodukt, Bartpflegeprodukt, Feuchttuch, Hygieneprodukt für den Intimbereich oder dergleichen; ein Zahn- und/oder Mundpflegeprodukt bzw. Zahn- und/oder Mundreinigungsprodukt, vorzugsweise Zahnpasta, Mundwasser, Haftmittelzusammensetzung für Zahnersatzprodukte oder dergleichen; ein Haarbehandlungsprodukt, vorzugsweise Haarfärbeprodukt, Haarfestiger, Haarspülung oder dergleichen; ein Anwendungsprodukt für optische und/oder dekorative Zwecke, vorzugsweise Farbkosmetikum, Lippenpflegeprodukt, wie Lippenstift oder Lippengloss, Augenpflegeprodukt, wie Mascara oder Lidschatten, Gesichts-Make-up bzw. Rouge, Nagellack oder dergleichen; ein Körpergeruch beeinflussendes Produkt bzw. Duftprodukt, vorzugsweise Deodorant, Parfüm, Eau de Toilette oder dergleichen, sein.

Erfindungsgemäß kann es zudem vorgesehen sein, dass das Nahrungsmittel-, kosmetische oder pharmazeutische Erzeugnis eine Flüssigkeit, Salbe, Creme, Paste, Lotion, ein Gel, Spray oder ein Feststoff, ein Pulver, eine Tablette, Pille, Kapsel, ein Dragee oder dergleichen ist und/oder als Flüssigkeit, Salbe, Creme, Paste, Lotion, Gel, Spray, Feststoff, Pulver, Tablette, Pille, Kapsel, Dragee oder dergleichen formuliert und/oder ausgebildet ist.

Gemäß einer erfindungsgemäßen Ausführungsform kann es auch vorgesehen sein, dass das Nahrungsmittel-, kosmetische oder pharmazeutische Erzeugnis als Trockenformulierung, insbesondere pulverförmige Trockenformulierung, ausgebildet ist. Dabei kann es sich erfindungsgemäß insbesondere derart verhalten, dass die Trockenformulierung, vorzugsweise im Rahmen der Anwendung und/oder Verwendung, insbesondere anwender- und/oder verbraucherseitig, in eine Flüssigkeit, insbesondere Wasser, eingebracht und/oder eingelagert bzw. inkorporiert wird. Auf diese Weise können somit anwendungsfertige (End-)Produkte, insbesondere durch den Anwender bzw. Nutzer selbst bereitgestellt werden, wobei auch auf dieser Basis stabile Dispersionen, insbesondere Emulsionen, erhalten werden.

Für weitergehende Einzelheiten zu dem vorliegend beschriebenen zweiten bzw. dritten Erfindungsaspekt kann auch Bezug genommen werden auf die Ausführungen zu den weiteren Erfindungsaspekten, wobei diese Ausführungen gleichermaßen für den vorliegenden Erfindungsaspekt gelten.

Gleichermaßen ist Gegenstand der vorliegenden Erfindung - gemäß einem **vierten** Aspekt der vorliegenden Erfindung - das erfindungsgemäße Verfahren zur Herstellung einer mittelkettige Triglyceride (MKTs) enthaltenden pulverförmigen Zusammensetzung, insbesondere Verfahren zur Herstellung einer wie zuvor definierten Zusammensetzung,
wobei das Verfahren die folgenden Verfahrensschritte umfasst, insbesondere in der nachfolgend angegebenen Reihenfolge der Verfahrensschritte:
(i) Bereitstellung und/oder Erzeugung einer vorzugsweise wässrigen homogenen Dispersion, insbesondere einer vorzugsweise wässrigen homogenen Emulsion, welche Gummi arabicum, insbesondere als polysaccharidbasiertes Trägermaterial, einerseits und ein mindestens ein mittelkettiges Triglycerid (MKT), insbesondere mindestens ein Triglycerid mindestens einer linearen gesättigten C₆-, C₈-, C₁₀- oder C₁₂-Carbonsäure, andererseits, in einem vorzugsweise wässrigen flüssigen Trägermedium enthält,
   wobei ein Gummi arabicum mit einem Glykoproteingehalt, bezogen auf das Gummi arabicum, im Bereich von 4 Gew-% bis 25 Gew.-% eingesetzt wird und wobei das Triglycerid in einer Menge im Bereich von 50 Gew.-% bis 90 Gew.-%, bezogen auf das Trockengewicht der Dispersion, insbesondere Emulsion, eingesetzt wird; nachfolgend
(ii) Trocknung, insbesondere Sprühtrocknung, der in Verfahrensschritt (i) erhaltenen Dispersion, insbesondere Emulsion, unter zumindest im Wesentlichen vollständiger Entfernung des vorzugsweise wässrigen flüssigen Trägermediums, so dass eine mittelkettige Triglyceride (MKTs) enthaltende pulverförmige Zusammensetzung, insbesondere eine wie zuvor beschriebene Zusammensetzung, resultiert.

Was das erfindungsgemäße Verfahren anbelangt, so kann Verfahrensschritt (i) unter Eintrag von Scherenergie und/oder Scherkräften durchgeführt werden. Insbesondere kann in Verfahrensschritt (i) die Homogenisierung, insbesondere die Homogenisierung der Inhaltsstoffe, unter Eintrag von Scherenergie und/oder Scherkräften erfolgen, insbesondere wobei der Eintrag von Scherenergie und/oder Scherkräften mittels Hochdruckhomogenisation und/oder mittels eines vorzugsweise Hochdruckhomogenisators erfolgt. Insbesondere kann in Verfahrensschritt (i) eine Hochdruckhomogenisation, insbesondere mittels eines Hochdruckhomogenisators, durchgeführt werden.

In diesem Zusammenhang kann es erfindungsgemäß vorgesehen sein, dass die Hochdruckhomogenisation mit einem Druckgradienten von bis zu 2.500 bar, insbesondere bis zu 2.250 bar, vorzugsweise bis zu 2.000 bar, durchgeführt wird. Insbesondere kann die Hochdruckhomogenisation mit einem Druckgradienten im Bereich von 50 bar bis 2.500 bar, insbesondere im Bereich von 75 bar bis 2.250 bar, vorzugsweise im Bereich von 100 bar 2.000 bar, durchgeführt werden.

Beispielsweise können Hochdruckhomogenisatoren auf Basis von Kolben/Spalt- bzw. Kolben/Düsen-Homogenisatoren eingesetzt werden.

Insbesondere ist im Rahmen der vorliegenden Erfindung eine kontinuierliche Verfahrens- bzw. Prozessführung mit hoher Durchsatzleistung möglich. Insbesondere kann erfindungsgemäß eine kontinuierliche Produktion der Zusammensetzung nach der Erfindung erfolgen.

Gemäß einer erfindungsgemäßen Ausführungsform kann es auch vorgesehen sein, dass in Verfahrensschritt (i) zwei- und/oder mehrstufig verfahren wird, insbesondere wobei in Verfahrensschritt (i) zunächst ein Vermischen, insbesondere Verrühren, vorzugsweise unter Eintrag von Misch- und/oder Rührenergie und/oder Rührkräften, der Inhaltsstoffe erfolgt, insbesondere zu Zwecken des Erhalts einer (Vor-)-Dispersion, insbesondere (Vor-)Emulsion, und nachfolgend eine Homogenisierung, insbesondere eine Homogenisierung der Inhaltsstoffe, unter Eintrag von Scherenergie und/oder Scherkräften, vorzugsweise Hochdruckhomogenisation der (Vor-)Dispersion, insbesondere (Vor-)Emulsion, insbesondere wie zuvor definiert, durchgeführt wird, insbesondere zum Erhalt der vorzugsweise wässrigen homogenen Dispersion, insbesondere der vorzugsweise wässrigen homogenen Emulsion der Inhaltsstoffe.

Auf Basis der erfindungsgemäßen Verfahrensführung kann in Verfahrensschritt (i) eine feinteilige homogene Dispersion, insbesondere eine feinteilige homogene Emulsion, erzeugt bzw. bereitgestellt werden. Dies ist auch im Hinblick auf die nachfolgende Trocknung, insbesondere Sprühtrocknung, von Vorteil (vgl. Verfahrensschritt (ii)). Insbesondere kann auch auf dieser Basis eine erfindungsgemäße Zusammensetzung mit definierten Partikeleigenschaften bzw. - größen bereitgestellt werden.

Im Allgemeinen kann in Verfahrensschritt (i) die homogene Dispersion, insbesondere die homogene Emulsion, auf eine Viskosität, insbesondere scheinbare Viskosität, von höchstens 2.500 mPa·s, insbesondere höchstens 1.000 mPa·s, vorzugsweise höchstens 400 mPa·s, eingestellt werden bzw. eine diesbezügliche Viskosität aufweisen, insbesondere bestimmt gemäß DIN EN ISO 2555 und insbesondere bei Raumtemperatur (20 °C) und einer Scherrate von 10 s⁻¹. Die Viskosität kann insbesondere mittels Rotations-Viskosimeter bestimmt werden. Die definierte Viskosität führt zu einem besonders guten Verhalten bei der Trocknung bzw. Sprühtrocknung zum Erhalt der erfindungsgemäßen Zusammensetzung.

Was die in Verfahrensschritt (ii) erhaltene Zusammensetzung anbelangt, so kann diese eine Vielzahl einzelner Partikel umfassen bzw. hieraus bestehen. Dabei ist es erfindungsgemäß vorgesehen, dass die Partikel jeweils Gummi arabicum als polysaccharidbasiertes Trägermaterial umfassen.

In diesem Zusammenhang kann es insbesondere vorgesehen sein, dass das Gummi arabicum und/oder das polysaccharidbasierte Trägermaterial mit dem mittelkettigen Triglycerid (MKT), insbesondere mit dem Triglycerid mindestens einer linearen gesättigten C₆-, C₈-, C₁₀- oder C₁₂-Carbonsäure, versehen und/oder beaufschlagt wird und/oder insbesondere dass die Partikel das Gummi arabicum und/oder das polysaccharidbasierte Trägermaterial und das an das polysaccharidbasierte Trägermaterial gebundene und/oder fixierte mittelkettige Triglycerid (MKT), insbesondere das an das polysaccharidbasierte Trägermaterial gebundene und/oder fixierte Triglycerid mindestens einer linearen gesättigten C₆-, C₈-, C₁₀- oder C₁₂-Carbonsäure, umfassen.

Auf Basis des erfindungsgemäßen Verfahrens kann in Verfahrensschritt (i) und/oder in Verfahrensschritt (ii) die Partikelgröße eingestellt werden.

Im Rahmen des erfindungsgemäßen Verfahrens kann die Partikelgröße insbesondere eingestellt werden derart,
- dass die Partikel der Zusammensetzung eine D90-Partikelgröße, insbesondere einen D90-Teilchendurchmesser, im Bereich von 0,01 mm bis 5 mm, insbesondere im Bereich von 0,05 mm bis 2 mm, vorzugsweise im Bereich von 0,1 mm bis 1 mm, besonders bevorzugt im Bereich von 0,2 mm bis 0,8 mm, aufweisen; insbesondere bestimmt mittels Lichtbeugung, vorzugsweise bestimmt mittels Laserdiffraktometrie gemäß ISO 13320:2020; und/oder
- dass die Partikel der Zusammensetzung eine D50-Partikelgröße, insbesondere einen D50-Teilchendurchmesser, im Bereich von 0,01 mm bis 3 mm, insbesondere im Bereich von 0,05 mm bis 2 mm, vorzugsweise im Bereich von 0,1 mm bis 1 mm, besonders bevorzugt im Bereich von 0,15 mm bis 0,75 mm, aufweisen; insbesondere bestimmt mittels Lichtbeugung, vorzugsweise bestimmt mittels Laserdiffraktometrie gemäß ISO 13320:2020;
insbesondere wobei die Einstellung der Partikelgröße in Verfahrensschritt (i) insbesondere auf Basis und/oder mittels der Homogenisierung, insbesondere Hochdruckhomogenisation, und/oder in Verfahrensschritt (ii) auf Basis und/oder mittels der Trocknung, insbesondere Sprühtrocknung, erfolgt, vorzugsweise in Verfahrensschritt (ii) auf Basis und/oder mittels der Trocknung, insbesondere Sprühtrocknung.

Zudem kann im Rahmen des erfindungsgemäßen Verfahrens in Verfahrensschritt (ii) der Restfeuchtegehalt der Zusammensetzung eingestellt werden.

Insbesondere kann in Verfahrensschritt (ii) die Zusammensetzung auf einen Restfeuchtegehalt von höchstens 5 Gew.-%, insbesondere höchstens 4 Gew.-%, vorzugsweise höchstens 3 Gew.-%, bezogen auf die Zusammensetzung, eingestellt werden.

Weiterhin kann in Verfahrensschritt (ii) die Zusammensetzung auf einen Restfeuchtegehalt von mindestens 0,1 Gew.-%, insbesondere mindestens 0,25 Gew.-%, vorzugsweise mindestens 0,5 Gew.-%, bezogen auf die Zusammensetzung, eingestellt werden.

Zudem kann in Verfahrensschritt (ii) die Zusammensetzung auf einen Restfeuchtegehalt im Bereich von 0,1 Gew.-% bis 5 Gew.-%, insbesondere im Bereich von 0,25 Gew.-% bis 4 Gew.-%, vorzugsweise im Bereich von 0,5 Gew.-% bis 3 Gew.-%, bezogen auf die Zusammensetzung, eingestellt werden.

Was das erfindungsgemäße Verfahren weiterhin anbelangt, so kann nach der in Verfahrensschritt (ii) durchgeführten Trocknung, insbesondere Sprühtrocknung, eine weitere Trocknung durchgeführt werden. Insbesondere kann nach der in Verfahrensschritt (ii) durchgeführten Trocknung, insbesondere Sprühtrocknung, eine nachgeschaltete weitere Trocknung erfolgen. In diesem Zusammenhang kann die weitere und/oder nachfolgende Trocknung zu Zwecken einer (weiterführenden) Einstellung des Restfeuchtegehalts der Zusammensetzung, insbesondere wie zuvor definiert, durchgeführt werden bzw. erfolgen.

Erfindungsgemäß kann in Verfahrensschritt (ii) die Trocknung, insbesondere Sprühtrocknung, mittels Verdüsen und/oder mittels Versprühen der in Verfahrensschritt (i) erhaltenen Dispersion, insbesondere Emulsion, durchgeführt werden.

Zudem kann in Verfahrensschritt (ii) die Trocknung, insbesondere Sprühtrocknung, mittels Durchleiten und/oder mittels Versprühen der in Verfahrensschritt (i) erhaltenen Dispersion, insbesondere Emulsion, durch mindestens eine Düse, insbesondere unter (Hoch-)Druckbeaufschlagung, durchgeführt werden.

Im Rahmen des erfindungsgemäßen Verfahrens kann in Verfahrensschritt (i) und/oder in Verfahrensschritt (ii), insbesondere in Verfahrensschritt (i), mindestens ein Emulgator zugegeben bzw. eingesetzt werden, insbesondere wie zuvor definiert.

Erfindungsgemäß kann auch derart vorgegangen werden, dass nach Durchführung von Verfahrensschritt (ii) der erhaltenen Zusammensetzung mindestens ein Emulgator zugegeben wird, insbesondere wie zuvor definiert, insbesondere mittels Aufsprühen bzw. Vermischen und/oder Einarbeiten.

Für weitergehende Einzelheiten zu den erfindungsgemäßen Verfahren gemäß diesem Erfindungsaspekt kann auch Bezug genommen werden auf die Ausführungen zu den weiteren erfindungsgemäßen Aspekten, wobei diese Ausführungen gleichermaßen für den vorliegenden Erfindungsaspekt gelten.

Darüber hinaus ist Gegenstand der vorliegenden Erfindung - gemäß einem **fünften** Aspekt der vorliegenden Erfindung - auch das Verfahren zur Steuerung und/oder Einstellung der Beladung, insbesondere Beladungsmenge, eines Gummi arabicum umfassenden oder hieraus gebildeten polysaccharidbasierten Trägermaterials mit mindestens einem mittelkettigen Triglycerid (MKT), insbesondere mit mindestens einem Triglycerid mindestens einer linearen gesättigten C₆-, C₈-, C₁₀- oder C₁₂-Carbonsäure, insbesondere zur Herstellung einer mittelkettige Triglyceride (MKTs) enthaltenden partikulären und/oder pulverförmigen Zusammensetzung, vorzugsweise zur Herstellung einer wie zuvor beschriebenen Zusammensetzung,
wobei die Beladung, insbesondere Beladungsmenge, in Abhängigkeit vom Glykoproteingehalt des polysaccharidbasierten Trägermaterials gesteuert und/oder eingestellt wird.

In diesem Zusammenhang weist das Gummi arabicum einen Glykoproteingehalt, bezogen auf das Gummi arabicum, im Bereich von 4 Gew-% bis 25 Gew.-% auf, wobei sich der Glykoproteingehalt auf den Gesamtgehalt aller im Gummi arabicum vorhandener Glykoproteine bezieht.

Zudem liegt die Beladung, insbesondere die Beladungsmenge, an Triglycerid(en) im Bereich von 50 Gew.-% bis 90 Gew.-%, bezogen auf die Gesamtheit aus polysaccharidbasiertem Trägermaterial und Triglycerid(en), insbesondere bezogen auf die Zusammensetzung.

Für weitergehende Einzelheiten zu diesem Erfindungsaspekt kann auch Bezug genommen werden auf die Ausführungen zu den weiteren erfindungsgemäßen Aspekten, wobei diese Ausführungen gleichermaßen für den vorliegenden Erfindungsaspekt gelten.

Zudem ist weiterer Gegenstand der vorliegenden Erfindung - gemäß einem **sechsten** Aspekt der vorliegenden Erfindung - die Verwendung nach der Erfindung des Glykoproteingehalts eines Gummi arabicum umfassenden oder hieraus gebildeten polysaccharidbasierten Trägermaterials zur Steuerung und/oder Einstellung der Beladung, insbesondere Beladungsmenge, des polysaccharidbasierten Trägermaterials mit mindestens einem mittelkettigen Triglycerid (MKT), insbesondere mit mindestens einem Triglycerid mindestens einer linearen gesättigten C₆-, C₈-, C₁₀- oder C₁₂-Carbonsäure, insbesondere zu Zwecken der Herstellung einer mittelkettige Triglyceride (MKTs) enthaltenden partikulären und/oder pulverförmigen Zusammensetzung, vorzugsweise zu Zwecken der Herstellung einer wie zuvor beschriebenen Zusammensetzung nach der Erfindung.

In diesem Zusammenhang weist das Gummi arabicum einen Glykoproteingehalt, bezogen auf das Gummi arabicum, im Bereich von 4 Gew-% bis 25 Gew.-% auf, wobei sich der Glykoproteingehalt auf den Gesamtgehalt aller im Gummi arabicum vorhandener Glykoproteine bezieht.

Zudem liegt die Beladung, insbesondere die Beladungsmenge, an Triglycerid(en) im Bereich von 50 Gew.-% bis 90 Gew.-%, bezogen auf die Gesamtheit aus polysaccharidbasiertem Trägermaterial und Triglycerid(en), insbesondere bezogen auf die Zusammensetzung.

Für weitergehende Einzelheiten zu diesem Erfindungsaspekt kann auch Bezug genommen werden auf die Ausführungen zu den weiteren erfindungsgemäßen Aspekten, wobei diese Ausführungen gleichermaßen für den vorliegenden Erfindungsaspekt gelten.

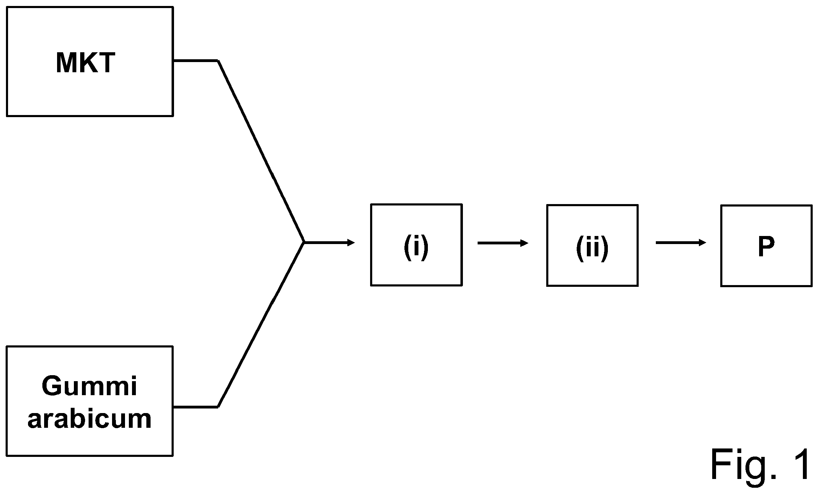

Im Folgenden wird die vorliegende Erfindung anhand einer Figurendarstellung (Figur 1) zur weiterführenden Beschreibung insbesondere des erfindungsgemäßen Verfahrens zur Herstellung der Zusammensetzung nach der Erfindung näher erläutert. Im Zusammenhang mit der nachfolgenden Erläuterung der erfindungsgemäßen Verfahrensführung, welche jedoch in Bezug auf die vorliegende Erfindung keinesfalls beschränkend ist, werden auch weitergehende Vorteile, Eigenschaften, Aspekte und Merkmale der vorliegenden Erfindung aufgezeigt.

In der einzigen Figurendarstellung zeigt Figur 1 eine schematische Darstellung bzw. Übersicht auf Basis eines Ablaufschemas einer erfindungsgemäßen Verfahrensführung zur Herstellung einer Zusammensetzung (Zusammensetzung P) nach der Erfindung, insbesondere partikulären Zusammensetzung P, vorzugsweise pulverförmigen Zusammensetzung P, bevorzugt mittelkettige Triglyceride (MKTs) enthaltenden pulverförmigen Zusammensetzung P.

Figur 1 zeigt dabei die erfindungsgemäße Verfahrensführung gemäß einer Ausführungsform der vorliegenden Erfindung, und zwar ausgehend von der Bereitstellung der Ausgangskomponenten in Form des mittelkettigen Triglycerids (MKT) und des für das Trägermaterial eingesetzten speziellen Gummi arabicum.

Figur 1 verdeutlicht weiterhin die nachfolgende Durchführung des erfindungsgemäßen Verfahrens mit der Abfolge der Verfahrensschritte (i) und (ii) zum Erhalt einer diesbezüglichen Zusammensetzung P. Dabei wird in Verfahrensschritt (i) insbesondere eine vorzugsweise wässrige homogene Dispersion, insbesondere eine vorzugsweise wässrige homogene Emulsion, bereitgestellt bzw. erzeugt, welche das Gummi arabicum, insbesondere als polysaccharidbasiertes Trägermaterial, einerseits und mindestens ein mittelkettiges Triglycerid (MKT), insbesondere mindestens ein Triglycerid mindestens einer linearen gesättigten C₆-, C₈-, C₁₀- oder C₁₂-Carbonsäure, andererseits in einem vorzugsweise wässrigen flüssigen Trägermedium enthält,
wobei im Rahmen des erfindungsgemäßen Verfahrens ein Gummi arabicum mit einem Glykoproteingehalt, bezogen auf das Gummi arabicum, im Bereich von 4 Gew-% bis 25 Gew.-% eingesetzt wird, wobei sich der Glykoproteingehalt auf den Gesamtgehalt aller im Gummi arabicum vorhandener Glykoproteine bezieht, und wobei das Triglycerid in einer Menge im Bereich von 50 Gew.-% bis 90 Gew.-%, bezogen auf das Trockengewicht der Dispersion, insbesondere Emulsion, eingesetzt wird.

In Verfahrensschritt (i) kann dabei insbesondere unter Eintrag von Scherenergie bzw. Scherkräften verfahren werden. Insbesondere kann in Verfahrensschritt (i) eine Homogenisierung der Inhaltsstoffe vorgenommen werden, beispielsweise mittels Hochdruckhomogenisation.

Figur 1 veranschaulicht auch die in Verfahrensschritt (i) nachfolgende Durchführung von Verfahrensschritt (ii), wobei in (ii) die Trocknung, insbesondere Sprühtrocknung, der in Verfahrensschritt (i) erhaltenen Dispersion, insbesondere Emulsion, unter zumindest im Wesentlichen vollständiger Entfernung des vorzugsweise wässrigen flüssigen Trägermediums erfolgt, insbesondere so dass die partikuläre Zusammensetzung P, vorzugsweise pulverförmige Zusammensetzung P, bevorzugt mittelkettige Triglyceride (MKTs) enthaltende pulverförmige Zusammensetzung P, insbesondere eine wie zuvor definierte Zusammensetzung P, resultiert.

In Verfahrensschritt (ii) kann insbesondere auch die Partikelgröße der der Zusammensetzung P nach der Erfindung zugrundeliegenden Partikel sowie die Restfeuchte der Zusammensetzung P eingestellt werden.

Auf Basis des erfindungsgemäßen Verfahrens werden somit entsprechende Zusammensetzungen P nach der Erfindung mit diesbezüglich verbesserten Eigenschaften, wie zuvor angeführt, bereitgestellt.

Weitere Ausgestaltungen, Abwandlungen und Variationen sowie Vorteile der vorliegenden Erfindung sind für den Fachmann beim Lesen der Beschreibung ohne Weiteres erkennbar und realisierbar, ohne dass er dabei den Rahmen der vorliegenden Erfindung verlässt.

Die folgenden Ausführungsbeispiele dienen lediglich der Veranschaulichung der vorliegenden Erfindung, jedoch ohne die vorliegende Erfindung hierauf zu beschränken.

### AUSFÜHRUNGSBEISPIELE:

Im Rahmen der nachfolgenden Ausführungsbeispiele bzw. Untersuchungen werden die herausragenden Eigenschaften von Zusammensetzungen nach der Erfindung verdeutlicht, und zwar auch was die Bereitstellung hoher Beladungsgrade mit mittelkettigen Triglyceriden (MKTs) anbelangt. Diesbezüglich kann die Anmelderin zeigen, dass die Verwendung eines speziellen Gummi arabicum als polysaccharidbasiertes Trägermaterial für die mittelkettigen Triglyceride (MKTs), wonach nämlich das Gummi arabicum in spezieller Weise einen hohen Glykoproteingehalt, nämlich von mindestens 4 Gew.-%, bezogen auf das Gummi arabicum, aufweist, hohe Beladungsgrade bzw. hohe Gehalte an mittelkettigem Triglycerid (MKT) ermöglicht, wonach nämlich die Zusammensetzung, bezogen auf die Zusammensetzung, einen Triglyceridgehalt von mindestens 50 Gew.-% aufweisen können. Hierzu im Einzelnen:

### a) Herstellung erfindungsgemäßer MKT-Zusammensetzungen

Die erfindungsgemäßen Zusammensetzungen können ausgehend von den die Zusammensetzung ausbildenden Inhaltsstoffen bzw. Komponenten insbesondere wie folgt hergestellt werden.

So kann insbesondere derart vorgegangen werden, dass zunächst die Bereitstellung einer vorzugsweise wässrigen homogenen Dispersion, insbesondere eine vorzugsweise wässrigen homogenen Emulsion unter Verwendung des polysaccharidbasierten Trägermaterials auf Basis des speziellen Gummi arabicum und den mittelkettigen Triglyceriden unter Verwendung von Wasser erfolgt. Hierzu kann beispielsweise ein sogenanntes "Blending" vorgenommen werden. Dabei wird zunächst Wasser vorgelegt, zu welchem das Gummi arabicum zugegeben wird, wobei eine (Rühr-)-Vermischung dieser Bestandteile vorgenommen wird. Anschließend erfolgt die Zugabe und Vermischung des mittelkettigen Triglycerids bzw. MKT-Öls zum Erhalt einer Vormischung bzw. "Pre-Blend". Diese Vormischung wird nachfolgend einer Hochdruckhomogenisation unterzogen bzw. zugeführt und auf dieser Basis homogenisiert. Die so erhaltene Emulsion wird anschließend einer Sprühtrocknung zugeführt bzw. unterzogen. Um optimale Ergebnisse zu erhalten, wird die zu versprühende Emulsion auf eine bestimmte Viskosität eingestellt, und zwar auch in Abhängigkeit von bei der Sprühtrocknung eingesetzten Sprühdüsen. Im Rahmen der Herstellung, insbesondere bei der Sprühtrocknung, wird die auf dieser Basis erhaltene partikuläre bzw. pulverförmige Zusammensetzung nach der Erfindung auch auf einen definierten Restfeuchtegehalt eingestellt, welcher im Allgemeinen 5 Gew.-%, bezogen auf die Zusammensetzung, nicht überschreiten sollte.

### b) Bereitstellung und Untersuchung von MKT-Zusammensetzungen

In Anlehnung an die in Abschnitt a) angeführten Herstellungsangaben werden erfindungsgemäße Zusammensetzungen (Zusammensetzungen C, D, E) und diesbezügliche Vergleichszusammensetzungen (Zusammensetzungen A, B) bereitgestellt und hinsichtlich ihrer Eigenschaften, wie nachfolgend angeführt, untersucht.

In den jeweiligen Zusammensetzungen wird in Bezug auf das Gummi arabicum der Gehalt an Glykoprotein sowie die korrespondierende Beladung der Zusammensetzung bzw. der Partikel mit den mittelkettigen Triglyceriden (MKT) entsprechend der nachfolgenden Tabelle eingestellt bzw. variiert. Auf Basis der entsprechenden Wertekombinationen für den Glykoprotein-Gehalt einerseits und der MKT-Beladung andererseits, wie auch in der nachfolgenden Tabelle angeführt, können grundsätzlich lagerstabile und emulgierfähige Pulver erhalten werden, wobei für die erfindungsgemäßen Zusammensetzungen trotz der höheren MKT-Beladung diesbezüglich verbesserte Eigenschaften vorliegen, wie auch nachfolgend angeführt

Die vorliegenden Zusammensetzungen werden hinsichtlich diverser Parameter getestet bzw. untersucht, nämlich was (i) die Emulgierfähigkeit in Wasser bei Raumtemperatur, (ii) die optische Qualität und Güte der jeweils erhaltenen Emulsion und (iii) die Stabilität der erhaltenen Emulsionen über einen Zeitraum von 10 bis 15 min anbelangt. Die Bewertung erfolgt zusammenfassend anhand der obigen Parameter nach dem Schulnotensystem (1 = sehr gut bis 6 = ungenügend). Die Ergebnisse sind der nachstehenden Tabelle zu entnehmen.

**Tabelle: Eigenschaften MKT-haltiger Zusammensetzungen**

| **Zusammensetzung** | **Glykoprotein-Gehalt, bezogen auf Gummi arabicum [Gew.-%]** | **MKT-Beladung, bezogen auf die Zusammensetzung bzw. Partikel [Gew.-%]** | **Bewertung** |
|---|---|---|---|
| **A** | 2 | 40 | 4 |
| **B** | 2,5 | 45 | 3 - 4 |
| **C** | 4 | 60 | 1 - 2 |
| **D** | 8 | 71 | 1 |
| **E** | 10 | 76 | 1 |

Mit den erfindungsgemäßen Zusammensetzungen C, D und E werden in Bezug auf die untersuchten Bewertungskriterien insgesamt hervorragende Eigenschaften erhalten, und dies bei gleichzeitig sehr hoher Beladung mit den mittelkettigen Triglyceriden. Auf dieser Basis können somit auch entsprechende Nahrungsmittel-, pharmazeutische bzw. kosmetische Erzeugnisse unter Verwendung der erfindungsgemäßen Zusammensetzungen mit hohem Gehalt an MKTs sowie homogener Emulsionsstruktur bei gleichzeitig hoher Lagerstabilität erhalten werden.

### c) Untersuchungen zum Restfeuchtegehalt MKT-haltiger Zusammensetzungen

Auf Basis in der in Abschnitt b) angeführten Tabelle werden entsprechende Zusammensetzungen mit jeweils variierendem Restfeuchtegehalt hergestellt. Für einen jeweiligen Restfeuchtegehalt, welcher 5 Gew.-%, bezogen auf die jeweilige Zusammensetzung, nicht überschreitet, werden dabei für die jeweiligen Zusammensetzungen insgesamt die besten Eigenschaften im Hinblick auf Lagerzeit bzw. (Lagerungs-)Stabilität und Emulsionsverhalten ermittelt. Bei einem jeweiligen Restfeuchtegehalt von mehr als 5 Gew.-% sind die diesbezüglichen Eigenschaften verschlechtert. Insbesondere ist im Allgemeinen auch eine Klumpen- bzw. Agglomeratbildung nach definierter Lagerungszeit zu beobachten, welche jedoch für die erfindungsgemäßen Zusammensetzungen C, D bzw. E deutlich geringer ausfällt als für die Vergleichszusammensetzungen A bzw. B. So übersteigt der Verklumpungsgrad für die erfindungsgemäßen Zusammensetzungen nicht den Wert von 2 %, während dieser Wert für die Vergleichszusammensetzungen jeweils oberhalb von 7 % liegt. Bei jeweiligen Restfeuchtegehalten von weniger als 0,1 Gew.-% liegt für die Zusammensetzung insgesamt eine Verschlechterung der Eigenschaften vor; insbesondere neigen die Zusammensetzungen zu einer gewissen Staubbildung, und auch die Ausbildung von Emulsionen bei Einarbeitung in Wasser ist verschlechtert. Jedoch weisen die erfindungsgemäßen Zusammensetzungen C bis E auch für sehr niedrige Restfeuchtegehalte diesbezüglich bessere Eigenschaften als die Vergleichszusammensetzung A bzw. B auf.

### d) Herstellung einer Hautpflegecreme unter Verwendung erfindungsgemäßer MKT-haltiger Zusammensetzungen

Auf Basis der erfindungsgemäßen Zusammensetzungen C bis E werden jeweilige Hautschutz- bzw. Hautpflegecremes hergestellt, und zwar auf Basis von drei Phasen, welche wie nachfolgend beschrieben zusammengeführt werden. Die erste Phase umfasst dabei die jeweilige erfindungsgemäße Zusammensetzung sowie insbesondere Öl-, Fett- bzw. Wachskomponenten. Die zweite Phase basiert auf Wasser, Glycerinmonoester sowie Zinksulfat. Die dritte Phase umfasst ein Tocopherol sowie Parfüm- bzw. Duftstoffkomponenten. Zur Herstellung der jeweiligen kosmetischen Erzeugnisse bzw. Produkte werden die erste Phase und die zweite Phase zunächst jeweils erwärmt. Anschließend wird die zweite Phase zu der ersten Phase unter Rühren zugegeben, gefolgt von einer Homogenisierung. Anschließend erfolgt eine Kühlung der so erhaltenen Phase mit nachfolgender Zugabe der dritten Phase und weiterführender Homogenisierung. Es resultieren entsprechende Hautschutz- bzw. Hautpflegecremes als kosmetische Erzeugnisse unter Verwendung der jeweiligen erfindungsgemäßen Zusammensetzungen. Die kosmetischen Erzeugnisse sind dabei lagerstabil und weisen einen hohen Gehalt an mittelkettigen Triglyceriden auf. Hieraus resultieren hervorragende Anwendungseigenschaften, und zwar auch was das Auftragen auf die Haut anbelangt. Zudem liegen hervorragende Pflegeeigenschaften vor.

Die obigen Ausführungen zeigen, dass die erfindungsgemäßen Zusammensetzungen sowie hierauf basierende Produkte bzw. Erzeugnisse mit vorteilhaften Eigenschaften einhergehen, insbesondere was die Ausbildung stabiler Emulsionen, eine hohe Lagerstabilität, hohe MKT-Gehalte sowie gute Anwendungs- und Handhabungseigenschaften anbelangt.

## Patentansprüche

1. Mittelkettige Triglyceride (MKTs) enthaltende pulverförmige Zusammensetzung, insbesondere zur Verwendung in kosmetischen, pharmazeutischen oder Nahrungsmittelerzeugnissen,
wobei die Zusammensetzung eine Vielzahl einzelner Partikel umfasst oder hieraus besteht,
wobei die Partikel jeweils mindestens ein polysaccharidbasiertes Trägermaterial umfassen, wobei das polysaccharidbasierte Trägermaterial mit mindestens einem mittelkettigen Triglycerid (MKT) versehen und/oder beaufschlagt ist und wobei die Partikel jeweils mindestens ein polysaccharidbasiertes Trägermaterial und mindestens ein an das polysaccharidbasierte Trägermaterial gebundenes und/oder fixiertes mittelkettiges Triglycerid (MKT) umfassen;
wobei das polysaccharidbasierte Trägermaterial Gummi arabicum umfasst oder hieraus gebildet ist, wobei das Gummi arabicum einen Glykoproteingehalt, bezogen auf das Gummi arabicum, im Bereich von 4 Gew-% bis 25 Gew.-% aufweist, wobei sich der Glykoproteingehalt auf den Gesamtgehalt aller im Gummi arabicum vorhandener Glykoproteine bezieht, und
wobei die Zusammensetzung, bezogen auf die Zusammensetzung, einen Triglyceridgehalt im Bereich von 50 Gew.-% bis 90 Gew.-% aufweist .

2. Zusammensetzung nach Anspruch 1,
wobei das Gummi arabicum naturbasiert und/oder natürlichen Ursprungs, insbesondere pflanzlichen Ursprungs, ist; und/oder
wobei das Gummi arabicum auf einem pflanzlichen Exsudat basiert und/oder aus einem pflanzliches Exsudat gewonnen ist, insbesondere aus einem pflanzlichen Exsudat von Akazienbäumen, insbesondere Gummiarabicum-Baum *(Senegalia senegal);* und/oder
wobei das Gummi arabicum aus Akazienbäumen, insbesondere Gummiarabicum-Baum *(Senegalia senegal*) gewonnen ist und/oder hiervon stammt; und/oder
wobei das Gummi arabicum aus einem Exsudat von Akazienbäumen, insbesondere Gummiarabicum-Baum *(Senegalia senegal)* gewonnen ist oder hierauf basiert.

3. Zusammensetzung nach Anspruch 1 oder Anspruch 2,
wobei der Glykoproteingehalt, des Gummi arabicum durch Auswahl des Gummi arabicum, insbesondere durch Auswahl des für die Gewinnung des Gummi arabicum, vorzugsweise auf Basis eines Exsudats, eingesetzten (Pflanzen-)Materials, gegebenenfalls unter zusätzlicher Anreicherung, insbesondere mittels Fraktionierung, kontrolliert und/oder eingestellt ist,
insbesondere wobei das (Pflanzen-)Material ausgewählt ist aus der Gruppe von Akazienbäumen, insbesondere Gummiarabicum-Baum *(Senegalia senegal);* und/oder
wobei der Glykoproteingehalt, des Gummi arabicum durch Anreicherung, insbesondere mittels Fraktionierung, kontrolliert und/oder eingestellt ist.

4. Zusammensetzung nach einem der vorangehenden Ansprüche,
wobei der Glykoproteingehalt durch Moleküle, insbesondere Makromoleküle, welche mindestens ein(e/n) Protein und/oder Proteinrest und/oder -gruppe mit einer oder mehreren hieran gebundenen, insbesondere kovalent gebundenen, Kohlenhydratgruppen umfassen, bereitgestellt wird und/oder gebildet wird; insbesondere wobei die Kohlenhydratgruppen aus Mono-, Di-, Oligo- und/oder Polysacchariden, vorzugsweise aus Oligosaccharid- und vor allem Polysaccharidgruppen ausgewählt sind; und/oder
wobei der Glykoproteingehalt durch Glykoproteine mit einem gewichtsmittleren Molekulargewicht M̅_{w} im Bereich von 10⁴ g/mol bis 10⁷ g/mol, insbesondere im Bereich von 1 x 10⁵ g/mol bis 9 x 10⁶ g/mol, vorzugsweise 1,5 x 10⁵ g/mol bis 6 x 10⁶ g/mol, bereitgestellt wird und/oder gebildet wird; und/oder
wobei der Glykoproteingehalt zumindest teilweise, insbesondere überwiegend, durch Arabinogalactanproteine bereitgestellt wird und/oder gebildet wird; und/oder
wobei der Glykoproteingehalt zu wenigstens 50 %, insbesondere zu wenigstens 60 %, vorzugsweise zu wenigstens 65 %, durch Arabinogalactanproteine bereitgestellt wird und/oder gebildet wird, bezogen auf den Glykoproteingehalt; und/oder
wobei der Glykoproteingehalt zu 50 % bis 99 %, insbesondere zu 60 % bis 98 %, vorzugsweise zu 65 % bis 95 %, durch Arabinogalactanproteine bereitgestellt wird und/oder gebildet wird, bezogen auf den Glykoproteingehalt.

5. Zusammensetzung nach einem der vorangehenden Ansprüche,
wobei das Gummi arabicum, insbesondere als Hauptbestandteil, mindestens ein Polysaccharid, vorzugsweise mindestens ein bevorzugt saure(s) Alkali- und/oder Erdalkalisalz(e) der (Poly-)Arabinsäure umfassendes Polysaccharid, besonders bevorzugt mindestens ein bevorzugt verzweigtes Polysaccharid aus L-Arabinose, D-Galaktose, L-Rhamnose und D-Glucuronsäure, enthält; und/oder
wobei das Gummi arabicum einen Polysaccharidgehalt, bezogen auf das Gummi arabicum, im Bereich von 75 Gew.-% bis 96 Gew.-% aufweist; und/oder
wobei das Gummi arabicum einen Polysaccharidgehalt, bezogen auf das Gummi arabicum, von mindestens 75 Gew.-% aufweist, und/oder
wobei das Gummi arabicum einen Polysaccharidgehalt, bezogen auf das Gummi arabicum, von höchstens 96 Gew.-% aufweist; und/oder
wobei das Gummi arabicum einen Polysaccharidgehalt, bezogen auf das Gummi arabicum, im Bereich von 78 Gew.-% bis 95,5 Gew, insbesondere im Bereich von 80 Gew.-% bis 95 Gew.-%, vorzugsweise im Bereich von 82 Gew.-% bis 94,5 Gew.-%, bevorzugt im Bereich von 84 Gew.-% bis 94 Gew.-%, aufweist; und/oder
wobei das Gummi arabicum einen Polysaccharidgehalt, bezogen auf das Gummi arabicum, von mindestens 78 Gew.-%, insbesondere mindestens 80 Gew.-%, vorzugsweise mindestens 82 Gew.-%, bevorzugt mindestens 84 Gew.-%, aufweist, und/oder
wobei das Gummi arabicum einen Polysaccharidgehalt, bezogen auf das Gummi arabicum, von höchstens 95,5 Gew.-%, insbesondere höchstens 95 Gew.-%, vorzugsweise höchstens 94,5 Gew.-%, bevorzugt höchstens 94 Gew.-%, aufweist.

6. Zusammensetzung nach einem der vorangehenden Ansprüche,
wobei das mittelkettige Triglycerid (MKT) ein Triglycerid einer C₈-Carbonsäure, insbesondere einer linearen gesättigten C₈-Carbonsäure, ist; und/oder
wobei das mittelkettige Triglycerid (MKT) Tricaprylin ist; und/oder
wobei das mittelkettige Triglycerid (MKT) ein gemischtes Triglycerid von C₈- und C₁₀-Carbonsäure, insbesondere ein gemischtes Triglycerid von jeweils linearer gesättigter C₈- und C₁₀-Carbonsäure, ist;
insbesondere wobei das mittelkettige Triglycerid (MKT) ein C₈ : C₁₀-Anteilverhältnis im Bereich von 30 % - 90 % : 70 % - 10 %, insbesondere im Bereich 50 % - 80 % : 50 % - 20 %, vorzugsweise im Bereich von 55 % - 75 % : 45 % - 25 %, aufweist.

7. Zusammensetzung nach einem der vorangehenden Ansprüche,
wobei die Zusammensetzung einen Ketokörper-Index (ketogener Index), berechnet als theoretische Anzahl Millimol (mmol) 3-Hydroxybuttersäure (3-BHB) pro ein Gramm der Zusammensetzung, von mindestens 0,5 mmol/g aufweist; und/oder
wobei die Zusammensetzung einen Ketokörper-Index (ketogener Index), berechnet als theoretische Anzahl Millimol (mmol) 3-Hydroxybuttersäure (3-BHB) pro ein Gramm der Zusammensetzung, von mindestens 1 mmol/g, insbesondere mindestens 2 mmol/g, vorzugsweise mindestens 3 mmol/g, bevorzugt mindestens 4 mmol/g, aufweist; und/oder
wobei die Zusammensetzung einen Ketokörper-Index (ketogener Index), berechnet als theoretische Anzahl Millimol (mmol) 3-Hydroxybuttersäure (3-BHB) pro ein Gramm der Zusammensetzung, im Bereich von 0,5 mmol/g bis 50 mmol/g aufweist; und/oder
wobei die Zusammensetzung einen Ketokörper-Index (ketogener Index), berechnet als theoretische Anzahl Millimol (mmol) 3-Hydroxybuttersäure (3-BHB) pro ein Gramm der Zusammensetzung, im Bereich von 1 mmol/g bis 45 mmol/g, insbesondere im Bereich von 2 mmol/g bis 40 mmol/g, vorzugsweise im Bereich von 3 mmol/g bis 30 mmol/g, bevorzugt im Bereich von 4 mmol/g bis 25 mmol/g, aufweist.

8. Zusammensetzung nach einem der vorangehenden Ansprüche,
wobei die Zusammensetzung mindestens einen Emulgator enthält;
insbesondere wobei die Zusammensetzung den Emulgator in einer Menge von mindestens 0,0001 Gew.-%, insbesondere mindestens 0,001 Gew.-%, vorzugsweise mindestens 0,01 Gew.-%, bezogen auf die Zusammensetzung, enthält; und/oder
insbesondere wobei die Zusammensetzung den Emulgator in einer Menge von höchstens 5 Gew.-%, insbesondere höchstens 2,5 Gew.-%, vorzugsweise höchstens 1 Gew.-%, bezogen auf die Zusammensetzung, enthält; und/oder
insbesondere wobei die Zusammensetzung den Emulgator in einer Menge im Bereich von 0,0001 Gew.-% bis 5 Gew.-%, insbesondere im Bereich von 0,001 Gew.-% bis 2,5 Gew.-%, vorzugsweise im Bereich von 0,01 Gew.-% bis 1 Gew.-%, bezogen auf die Zusammensetzung, enthält.

9. Zusammensetzung nach Anspruch 8,
wobei der Emulgator ein amphoterer, anionischer, kationischer oder nichtionischer Emulgator ist; und/oder
wobei der Emulgator ausgewählt ist aus der Gruppe von Fettsäureglyceriden, insbesondere Mono- und Diglyceriden von Fettsäuren, und ihren Salzen; Citronensäureestern von Fettsäureglyceriden, insbesondere Citronensäureestern von Mono- und Diglyceriden von Fettsäuren, und ihren Salzen; Weinsäureestern und Mono- und Diacetylweinsäureestern von Fettsäureglyceriden, insbesondere Weinsäureestern und Diacetylweinsäureestern von Mono- und Diglyceriden von Fettsäuren, und ihren Salzen; Essigsäureestern von Fettsäureglyceriden, insbesondere Essigsäureestern von Mono- und Diglyceriden von Fettsäuren, und ihren Salzen; Milchsäureestern von Fettsäureglyceriden, insbesondere Milchsäureestern von Mono- und Diglyceriden von Fettsäuren, und ihren Salzen; gemischten Essig- und Weinsäureestern von Fettsäureglyceriden, insbesondere gemischten Essig- und Weinsäureestern von Mono- und Diglyceriden von Fettsäuren, und ihren Salzen; Polyglycerinfettsäureestern, insbesondere Polyglycerinmono-, -di- und -poly-fettsäureestern, vorzugsweise Polyglycerin-Polyricinoleat; Sorbitanestern, insbesondere Polyoxyethylen-Sorbitanestern, vorzugsweise Polyoxyethylen-Sorbitanmonoestern, bevorzugt von Fettsäuren, insbesondere Polyoxyethylen-Sorbitanmonolaurat, Polyoxyethylen-Sorbitanmonooleat, Polyoxyethylen-Sorbitanmonopalmitat, Polyoxyethylen-Sorbitanmonostearat und Polyoxyethylen-Sorbitantristearat; ethoxylierten Fettalkoholen; ethoxylierten Fettsäureglyceriden, insbesondere ethoxylierten Mono- und Diglyceriden von Fettsäuren, und ihren Salzen; Alkylpolyglucosiden und Zuckerglyceriden, insbesondere Cetearylglucosiden; Zuckerestern von Fettsäuren, insbesondere Saccharoseestern von Fettsäuren; modifizierten Stärken; modifizierten Cellulosen; quartären Ammoniumverbindungen (Ester-Quats); Lecithinen; Phospholipiden; Alkylglucosesesquistearaten, insbesondere Methylglucosesesquistearat; Fettalkoholen; Fettsäuren und ihren Salzen und Estern; Siloxanen und Siliconen; Dimethiconen und modifizierten Dimethiconen, insbesondere PEG/PPG-Dimethiconen; Alkalimetallalkylsarcosinaten, insbesondere Natriumalkylsarcosinaten, vorzugsweise Alkalimetalllaurylsarcosinaten; Aminosäureacylestern und ihren Salzen; POE/PPG-Copolymeren; Phosphatestern (Phosphorsäureestern) und ihren Salzen, insbesondere Cetylphosphaten, vorzugsweise Alkalimetallcetylphosphaten, bevorzugt Kaliumcetylphosphat; Polyacrylaten; Castorölen und modifizierten Castorölen, insbesondere PEG-Castorölen und PEG-Castorölfettsäurestern und -difettsäureestern; ethoxylierten Alkylsulfaten; sowie deren Kombinationen und Mischungen; und/oder
wobei der Emulgator an den Partikeln gebunden und/oder fixiert ist; und/oder wobei die Zusammensetzung den Emulgator in an den Partikeln gebundener und/oder fixierter Form enthält; und/oder
wobei der Emulgator partikelförmig ausgebildet ist und/oder in Form von Emulgatorpartikeln ausgebildet ist; und/oder
wobei die Zusammensetzung den Emulgator in Form von separaten, insbesondere ungebundenen und/oder freien, Emulgatorpartikeln enthält.

10. Zusammensetzung nach einem der vorangehenden Ansprüche,
wobei die Zusammensetzung rieselfähig und/oder fließfähig ausgebildet ist; und/oder
wobei die Zusammensetzung zumindest im Wesentlichen frei von Verklumpungen ausgebildet ist; und/oder
wobei die Zusammensetzung zumindest im Wesentlichen frei ist von Partikelverklumpungen oder -aneinanderlagerungen, insbesondere Partikelmakroagglomeraten, mit Größen oberhalb von 5 mm, insbesondere oberhalb von 4,5 mm, vorzugsweise oberhalb von 4 mm, besonders bevorzugt oberhalb von 3,5 mm; und/oder
wobei die Zusammensetzung einen Restfeuchtegehalt von höchstens 5 Gew.-%, insbesondere höchstens 4 Gew.-%, vorzugsweise höchstens 3 Gew.-%, bezogen auf die Zusammensetzung, aufweist; und/oder
wobei die Zusammensetzung einen Restfeuchtegehalt von mindestens 0,1 Gew.-%, insbesondere mindestens 0,25 Gew.-%, vorzugsweise mindestens 0,5 Gew.-%, bezogen auf die Zusammensetzung, aufweist; und/oder
wobei die Zusammensetzung einen Restfeuchtegehalt im Bereich von 0,1 Gew.-% bis 5 Gew.-%, insbesondere im Bereich von 0,25 Gew.-% bis 4 Gew.-%, vorzugsweise im Bereich von 0,5 Gew.-% bis 3 Gew.-%, bezogen auf die Zusammensetzung, aufweist; und/oder
wobei die Partikel der Zusammensetzung eine D90-Partikelgröße, insbesondere einen D90-Teilchendurchmesser, im Bereich von 0,01 mm bis 5 mm, insbesondere im Bereich von 0,05 mm bis 2 mm, vorzugsweise im Bereich von 0,1 mm bis 1 mm, besonders bevorzugt im Bereich von 0,2 mm bis 0,8 mm, aufweisen; insbesondere bestimmt mittels Lichtbeugung, vorzugsweise bestimmt mittels Laserdiffraktometrie gemäß ISO 13320:2020; und/oder
wobei die Partikel der Zusammensetzung eine D50-Partikelgröße, insbesondere einen D50-Teilchendurchmesser, im Bereich von 0,01 mm bis 3 mm, insbesondere im Bereich von 0,05 mm bis 2 mm, vorzugsweise im Bereich von 0,1 mm bis 1 mm, besonders bevorzugt im Bereich von 0,15 mm bis 0,75 mm, aufweisen; insbesondere bestimmt mittels Lichtbeugung, vorzugsweise bestimmt mittels Laserdiffraktometrie gemäß ISO 13320:2020.

11. Nahrungsmittel-, kosmetisches oder pharmazeutisches Erzeugnis, enthaltend eine Zusammensetzung nach einem der Ansprüche 1 bis 10.

12. Verwendung einer Zusammensetzung nach einem der Ansprüche 1 bis 10 in einem Nahrungsmittel-, kosmetischen oder pharmazeutischen Erzeugnis und/oder zur Herstellung eines Nahrungsmittel-, kosmetischen oder pharmazeutischen Erzeugnisses.

13. Verfahren zur Herstellung einer mittelkettige Triglyceride (MKTs) enthaltenden pulverförmigen Zusammensetzung nach einem der Ansprüche 1 bis 10,
wobei das Verfahren die folgenden Verfahrensschritte umfasst, insbesondere in der nachfolgend angegebenen Reihenfolge der Verfahrensschritte:
(i) Bereitstellung und/oder Erzeugung einer vorzugsweise wässrigen homogenen Dispersion, insbesondere einer vorzugsweise wässrigen homogenen Emulsion, welche Gummi arabicum, insbesondere als polysaccharidbasiertes Trägermaterial, einerseits und ein mindestens ein mittelkettiges Triglycerid (MKT), insbesondere mindestens ein Triglycerid mindestens einer linearen gesättigten C₆-, C₈-, C₁₀- oder C₁₂-Carbonsäure, andererseits, in einem vorzugsweise wässrigen flüssigen Trägermedium enthält,
wobei ein Gummi arabicum mit einem Glykoproteingehalt, bezogen auf das Gummi arabicum, im Bereich von 4 Gew-% bis 25 Gew.-% eingesetzt wird und wobei das Triglycerid in einer Menge im Bereich von 50 Gew.-% bis 90 Gew.-%, bezogen auf das Trockengewicht der Dispersion, insbesondere Emulsion, eingesetzt wird; nachfolgend
(ii) Trocknung, insbesondere Sprühtrocknung, der in Verfahrensschritt (i) erhaltenen Dispersion, insbesondere Emulsion, unter zumindest im Wesentlichen vollständiger Entfernung des vorzugsweise wässrigen flüssigen Trägermediums, so dass eine mittelkettige Triglyceride (MKTs) enthaltende pulverförmige Zusammensetzung nach einem der Ansprüche 1 bis 10 resultiert.

14. Verfahren zur Steuerung und/oder Einstellung der Beladung, insbesondere Beladungsmenge, eines Gummi arabicum umfassenden oder hieraus gebildeten polysaccharidbasierten Trägermaterials mit mindestens einem mittelkettigen Triglycerid (MKT), insbesondere mit mindestens einem Triglycerid mindestens einer linearen gesättigten C₆-, C₈-, C₁₀- oder C₁₂-Carbonsäure, insbesondere zur Herstellung einer mittelkettige Triglyceride (MKTs) enthaltenden partikulären und/oder pulverförmigen Zusammensetzung, vorzugsweise zur Herstellung einer Zusammensetzung nach einem der Ansprüche 1 bis 10,
wobei die Beladung, insbesondere Beladungsmenge, in Abhängigkeit vom Glykoproteingehalt, des polysaccharidbasierten Trägermaterials gesteuert und/oder eingestellt wird,
wobei das Gummi arabicum einen Glykoproteingehalt, bezogen auf das Gummi arabicum, im Bereich von 4 Gew-% bis 25 Gew.-% aufweist, wobei sich der Glykoproteingehalt auf den Gesamtgehalt aller im Gummi arabicum vorhandener Glykoproteine bezieht, und
wobei die Beladung, insbesondere die Beladungsmenge, an Triglycerid(en) im Bereich von 50 Gew.-% bis 90 Gew.-% liegt, bezogen auf die Gesamtheit aus polysaccharidbasiertem Trägermaterial und Triglycerid(en), insbesondere bezogen auf die Zusammensetzung.

15. Verwendung des Glykoproteingehalts eines Gummi arabicum umfassenden oder hieraus gebildeten polysaccharidbasierten Trägermaterials zur Steuerung und/oder Einstellung der Beladung, insbesondere Beladungsmenge, des polysaccharidbasierten Trägermaterials mit mindestens einem mittelkettigen Triglycerid (MKT), insbesondere mit mindestens einem Triglycerid mindestens einer linearen gesättigten C₆-, C₈-, C₁₀- oder C₁₂-Carbonsäure, insbesondere zu Zwecken der Herstellung einer mittelkettige Triglyceride (MKTs) enthaltenden partikulären und/oder pulverförmigen Zusammensetzung, vorzugsweise zu Zwecken der Herstellung einer Zusammensetzung nach einem der Ansprüche 1 bis 10,
wobei das Gummi arabicum einen Glykoproteingehalt, bezogen auf das Gummi arabicum, im Bereich von 4 Gew-% bis 25 Gew.-% aufweist, wobei sich der Glykoproteingehalt auf den Gesamtgehalt aller im Gummi arabicum vorhandener Glykoproteine bezieht, und
wobei die Beladung, insbesondere die Beladungsmenge, an Triglycerid(en) im Bereich von 50 Gew.-% bis 90 Gew.-% liegt, bezogen auf die Gesamtheit aus polysaccharidbasiertem Trägermaterial und Triglycerid(en), insbesondere bezogen auf die Zusammensetzung.

## Claims

1. Composition in powder form comprising medium-chain triglycerides (MCTs), in particular for use in cosmetic, pharmaceutical or food products,
wherein the composition comprises or consists of a plurality of individual particles,
wherein each particle comprises at least one polysaccharide-based carrier material, wherein the polysaccharide-based carrier material is equipped and/or loaded with at least one medium-chain triglyceride (MCT), and wherein each particle comprises at least one polysaccharide-based carrier material and at least one medium-chain triglyceride (MCT) bound and/or fixed to the polysaccharide-based carrier material;
wherein the polysaccharide-based carrier material comprises or is formed from gum arabic, wherein the gum arabic comprises a glycoprotein content, based on the gum arabic, in the range from 4 wt.% to 25 wt.%, wherein the glycoprotein content refers to the total content of all glycoproteins present in the gum arabic, and
wherein the composition comprises, based on the composition, a triglyceride content in the range from 50 wt.% to 90 wt.%.

2. Composition according to claim 1,
wherein the gum arabic is nature-based and/or of natural origin, in particular of plant-based origin; and/or
wherein the gum arabic is based on a plant exudate and/or is obtained from a plant exudate, in particular from a plant exudate of acacia trees, in particular the gum arabic tree *(Senegalia senegal);* and/or
wherein the gum arabic is obtained from acacia trees, in particular the gum arabic tree *(Senegalia senegal),* and/or is derived therefrom; and/or
wherein the gum arabic is obtained from an exudate of acacia trees, in particular the gum arabic tree *(Senegalia senegal),* or is based thereon.

3. Composition according to claim 1 or claim 2,
wherein the glycoprotein content of the gum arabic is controlled and/or adjusted by selecting the gum arabic, in particular by selecting the (plant) material used for obtaining the gum arabic, preferably based on an exudate, optionally with additional enrichment, in particular by means of fractionation,
in particular wherein the (plant) material is selected from the group of acacia trees, in particular the gum arabic tree *(Senegalia senegal);* and/or
wherein the glycoprotein content of the gum arabic is controlled and/or adjusted by enrichment, in particular by means of fractionation.

4. Composition according to one of the preceding claims,
wherein the glycoprotein content is provided and/or formed by molecules, in particular macromolecules, comprising at least one protein and/or protein residue and/or protein group with one or more carbohydrate groups bound thereto, in particular covalently bound; in particular wherein the carbohydrate groups are selected from mono-, di-, oligo-, and/or polysaccharides, preferably from oligosaccharide and especially polysaccharide groups; and/or
wherein the glycoprotein content is provided and/or formed by glycoproteins having a weight-average molecular weight M̅_{w} in the range from 10⁴ g/mol to 10⁷ g/mol, in particular in the range from 1 x 10⁵ g/mol to 9 x 10⁶ g/mol, preferably 1.5 x 10⁵ g/mol to 6 x 10⁶ g/mol; and/or
wherein the glycoprotein content is at least partially, in particular predominantly, provided and/or formed by arabinogalactan proteins; and/or
wherein the glycoprotein content is provided and/or formed by arabinogalactan proteins to at least 50%, in particular to at least 60%, preferably to at least 65%, based on the glycoprotein content; and/or
wherein 50% to 99%, in particular 60% to 98%, preferably 65% to 95%, of the glycoprotein content is provided and/or formed by arabinogalactan proteins, based on the glycoprotein content.

5. **Composition** according to one of the preceding claims,
wherein the gum arabic, in particular as the main component, comprises at least one polysaccharide, preferably at least one polysaccharide comprising preferably acidic alkali and/or alkaline earth salts of (poly)arabinic acid, particularly preferred at least one preferably branched polysaccharide of L-arabinose, D-galactose, L-rhamnose and D-glucuronic acid; and/or
wherein the gum arabic comprises a polysaccharide content, based on the gum arabic, in the range from 75 wt.% to 96 wt.%; and/or
wherein the gum arabic comprises a polysaccharide content, based on the gum arabic, of at least 75 wt.%, and/or
wherein the gum arabic comprises a polysaccharide content, based on the gum arabic, of at most 96 wt.%; and/or
wherein the gum arabic comprises a polysaccharide content, based on the gum arabic, in the range from 78 wt.% to 95.5 wt.%, in particular in the range from 80 wt.% to 95 wt.%, preferably in the range from 82 wt.% to 94.5 wt.%, more preferably in the range from 84 wt.% to 94 wt.%; and/or
wherein the gum arabic comprises a polysaccharide content, based on the gum arabic, of at least 78 wt.%, in particular at least 80 wt.%, preferably at least 82 wt.%, more preferably at least 84 wt.%, and/or
wherein the gum arabic comprises a polysaccharide content, based on the gum arabic, of at most 95.5 wt.%, in particular at most 95 wt.%, preferably at most 94.5 wt.%, more preferably at most 94 wt.%.

6. Composition according to one of the preceding claims,
wherein the medium-chain triglyceride (MCT) is a triglyceride of a C₈ carboxylic acid, in particular a linear saturated C₈ carboxylic acid; and/or
wherein the medium-chain triglyceride (MCT) is tricaprylin; and/or
wherein the medium-chain triglyceride (MCT) is a mixed triglyceride of C₈ and C₁₀ carboxylic acids, in particular a mixed triglyceride of linear saturated C₈ and C₁₀ carboxylic acids, respectively;
in particular wherein the medium-chain triglyceride (MCT) has a C₈ : C₁₀ ratio in the range of 30% - 90% : 70% - 10%, in particular in the range of 50% - 80% : 50% - 20%, preferably in the range of 55% - 75% : 45% - 25%.

7. Composition according to one of the preceding claims,
wherein the composition has a keto body index (ketogenic index), calculated as the theoretical number of millimoles (mmol) of 3-hydroxybutyric acid (3-BHB) per one gram of the composition, of at least 0.5 mmol/g; and/or
wherein the composition has a keto body index (ketogenic index), calculated as the theoretical number of millimoles (mmol) of 3-hydroxybutyric acid (3-BHB) per one gram of the composition, of at least 1 mmol/g, in particular at least 2 mmol/g, preferably at least 3 mmol/g, more preferably at least 4 mmol/g; and/or
wherein the composition has a keto body index (ketogenic index), calculated as the theoretical number of millimoles (mmol) of 3-hydroxybutyric acid (3-BHB) per one gram of the composition, in the range from 0.5 mmol/g to 50 mmol/g; and/or
wherein the composition has a keto body index (ketogenic index), calculated as the theoretical number of millimoles (mmol) of 3-hydroxybutyric acid (3-BHB) per one gram of the composition, in the range from 1 mmol/g to 45 mmol/g, in particular in the range from 2 mmol/g to 40 mmol/g, preferably in the range from 3 mmol/g to 30 mmol/g, more preferably in the range from 4 mmol/g to 25 mmol/g.

8. Composition according to one of the preceding claims,
wherein the composition contains at least one emulsifier;
in particular wherein the composition contains the emulsifier in an amount of at least 0.0001 wt.%, in particular at least 0.001 wt.%, preferably at least 0.01 wt.%, based on the composition; and/or
in particular wherein the composition contains the emulsifier in an amount of at most 5 wt.%, in particular at most 2.5 wt.%, preferably at most 1 wt.%, based on the composition; and/or
in particular wherein the composition contains the emulsifier in an amount in the range from 0.0001 wt.% to 5 wt.%, in particular in the range from 0.001 wt.% to 2.5 wt.%, preferably in the range from 0.01 wt.% to 1 wt.%, based on the composition.

9. Composition according to claim 8,
wherein the emulsifier is an amphoteric, anionic, cationic or nonionic emulsifier; and/or
wherein the emulsifier is selected from the group consisting of fatty acid glycerides, in particular mono- and diglycerides of fatty acids, and their salts; citric acid esters of fatty acid glycerides, in particular citric acid esters of mono- and diglycerides of fatty acids, and their salts; tartaric acid esters and mono- and diacetyl tartaric acid esters of fatty acid glycerides, in particular tartaric acid esters and diacetyl tartaric acid esters of mono- and diglycerides of fatty acids, and their salts; acetic acid esters of fatty acid glycerides, in particular acetic acid esters of mono- and diglycerides of fatty acids, and their salts; lactic acid esters of fatty acid glycerides, in particular lactic acid esters of mono- and diglycerides of fatty acids, and their salts; mixed acetic and tartaric acid esters of fatty acid glycerides, in particular mixed acetic and tartaric acid esters of mono- and diglycerides of fatty acids, and their salts; polyglycerol fatty acid esters, in particular polyglycerol mono-, di-, and poly-fatty acid esters, preferably polyglycerol polyricinoleate; sorbitan esters, in particular polyoxyethylene sorbitan esters, preferably polyoxyethylene sorbitan monoesters, preferably of fatty acids, in particular polyoxyethylene sorbitan monolaurate, polyoxyethylene sorbitan monooleate, polyoxyethylene sorbitan monopalmitate, polyoxyethylene sorbitan monostearate, and polyoxyethylene sorbitan tristearate; ethoxylated fatty alcohols; ethoxylated fatty acid glycerides, in particular ethoxylated mono- and diglycerides of fatty acids, and their salts; alkyl polyglucosides and sugar glycerides, in particular cetearyl glucosides; sugar esters of fatty acids, in particular sucrose esters of fatty acids; modified starches; modified celluloses; quaternary ammonium compounds (ester quats); lecithins; phospholipids; alkylglucose sesquistearates, in particular methylglucose sesquistearate; fatty alcohols; fatty acids and their salts and esters; siloxanes and silicones; dimethicones and modified dimethicones, in particular PEG/PPG dimethicones; alkali metal alkyl sarcosinates, in particular sodium alkyl sarcosinates, preferably alkali metal lauryl sarcosinates; amino acid acyl esters and their salts; POE/PPG copolymers; phosphate esters (phosphoric acid esters) and their salts, in particular cetyl phosphates, preferably alkali metal cetyl phosphates, more preferably potassium cetyl phosphate; polyacrylates; castor oils and modified castor oils, in particular PEG-castor oils and PEG-castor oil fatty acid esters and di-fatty acid esters; ethoxylated alkyl sulfates; as well as combinations and mixtures thereof; and/or
wherein the emulsifier is bound and/or fixed to the particles; and/or wherein the composition contains the emulsifier in a form bound and/or fixed to the particles; and/or
wherein the emulsifier is in particulate form and/or is in the form of emulsifier particles; and/or
wherein the composition contains the emulsifier in the form of separate, in particular unbound and/or free, emulsifier particles.

10. Composition according to one of the preceding claims,
wherein the composition is free-flowing and/or flowable; and/or
wherein the composition is at least substantially free from clumping; and/or
wherein the composition is at least substantially free of particle clumps or agglomerates, in particular particle macroagglomerates, with sizes above 5 mm, in particular above 4.5 mm, preferably above 4 mm, particularly preferred above 3.5 mm; and/or
wherein the composition has a residual moisture content of at most 5 wt.%, in particular at most 4 wt. %, preferably at most 3 wt.%, based on the composition; and/or
wherein the composition has a residual moisture content of at least 0.1 wt.%, in particular at least 0.25 wt.%, preferably at least 0.5 wt.%, based on the composition; and/or
wherein the composition has a residual moisture content in the range from 0.1 wt.% to 5 wt.%, in particular in the range from 0.25 wt.% to 4 wt.%, preferably in the range from 0.5 wt.% to 3 wt.%, based on the composition; and/or
wherein the particles of the composition have a D90 particle size, in particular a D90 particle diameter, in the range from 0.01 mm to 5 mm, in particular in the range from 0.05 mm to 2 mm, preferably in the range from 0.1 mm to 1 mm, particularly preferred in the range from 0.2 mm to 0.8 mm; in particular determined by light diffraction, preferably determined by laser diffractometry in accordance with ISO 13320:2020; and/or
wherein the particles of the composition have a D50 particle size, in particular a D50 particle diameter, in the range from 0.01 mm to 3 mm, in particular in the range from 0.05 mm to 2 mm, preferably in the range from 0.1 mm to 1 mm, particularly preferred in the range from 0.15 mm to 0.75 mm; in particular determined by light diffraction, preferably determined by laser diffractometry in accordance with ISO 13320:2020.

11. Food, cosmetic or pharmaceutical product comprising a composition according to one of the claims 1 to 10.

12. Use of a composition according to one of the claims 1 to 10 in a food, cosmetic or pharmaceutical product and/or for producing a food, cosmetic or pharmaceutical product.

13. Method for producing a composition in powder form containing medium-chain triglycerides (MCTs) according to one of the claims 1 to 10,
wherein the method comprises the following method steps, in particular in the order of the method steps specified below:
(i) providing and/or producing a preferably aqueous homogeneous dispersion, in particular a preferably aqueous homogeneous emulsion, which comprises, on the one hand, gum arabic, in particular as a polysaccharide-based carrier material, and, on the other hand, at least one medium-chain triglyceride (MCT), in particular at least one triglyceride of at least one linear saturated C₆, C₈, C₁₀ or C₁₂ carboxylic acid, in a preferably aqueous liquid carrier medium,
wherein gum arabic with a glycoprotein content, based on the gum arabic, in the range from 4 wt.% to 25 wt.% is used, and wherein the triglyceride is used in an amount in the range from 50 wt.% to 90 wt.%, based on the dry weight of the dispersion, in particular emulsion; subsequently
(ii) drying, in particular spray drying, of the dispersion, in particular emulsion, obtained in method step (i), with at least substantially complete removal of the preferably aqueous liquid carrier medium, so that a composition in powder form comprising medium-chain triglycerides (MCTs) according to one of claims 1 to 10 results.

14. Method for controlling and/or adjusting the loading, in particular the loading amount, of a polysaccharide-based carrier material comprising gum arabic or formed therefrom with at least one medium-chain triglyceride (MCT), in particular with at least one triglyceride of at least one linear saturated C₆, C₈, C₁₀ or C₁₂ carboxylic acid, in particular for producing a particulate composition and/or a composition in powder form comprising medium-chain triglycerides (MCTs), preferably for producing a composition according to one of the claims 1 to 10,
wherein the loading, in particular the loading amount, is controlled and/or adjusted depending on the glycoprotein content of the polysaccharide-based carrier material,
wherein the gum arabic has a glycoprotein content, based on the gum arabic, in the range from 4 wt.% to 25 wt.%, wherein the glycoprotein content refers to the total content of all glycoproteins present in the gum arabic, and
wherein the loading, in particular the loading amount, of triglyceride(s) is in the range from 50 wt.% to 90 wt.%, based on the total of the polysaccharide-based carrier material and triglyceride(s), in particular based on the composition.

15. Use of the glycoprotein content of a polysaccharide-based carrier material comprising gum arabic or formed therefrom for controlling and/or adjusting the loading, in particular the loading amount, of the polysaccharide-based carrier material with at least one medium-chain triglyceride (MCT), in particular with at least one triglyceride of at least one linear saturated C₆, C₈, C₁₀ or C₁₂ carboxylic acid, in particular for the purpose of producing a particulate composition and/or a composition in powder form comprising medium-chain triglycerides (MCTs), preferably for the purpose of producing a composition according to one of the claims 1 to 10,
wherein the gum arabic has a glycoprotein content, based on the gum arabic, in the range from 4 wt.% to 25 wt.%, wherein the glycoprotein content refers to the total content of all glycoproteins present in the gum arabic, and
wherein the loading, in particular the loading amount, of triglyceride(s) is in the range from 50 wt.% to 90 wt.%, based on the total of the polysaccharide-based carrier material and triglyceride(s), in particular based on the composition.

## Revendications

1. Composition pulvérulente contenant des triglycérides à chaîne moyenne (MCT), en particulier pour une utilisation dans des produits cosmétiques, pharmaceutiques ou alimentaires,
où la composition comprend une pluralité de particules individuelles ou est constituée de celles-ci,
où les particules comprennent chacune au moins un matériau de support à base de polysaccharide, où le matériau de support à base de polysaccharide est pourvu et/ou chargé avec au moins un triglycéride à chaîne moyenne (MCT) et où les particules comprennent chacune au moins un matériau de support à base de polysaccharide et au moins un triglycéride à chaîne moyenne (MCT) lié et/ou fixé au matériau de support à base de polysaccharide;
où le matériau de support à base de polysaccharide comprend de la gomme arabique ou est formé de celle-ci, où la gomme arabique présente une teneur en glycoprotéines, par rapport à la gomme arabique, dans l'intervalle de 4 % en poids à 25 % en poids, où la teneur en glycoprotéines se rapporte à la teneur totale de toutes les glycoprotéines présentes dans la gomme arabique, et
où la composition présente, par rapport à la composition, une teneur en triglycérides dans l'intervalle de 50 % en poids à 90 % en poids.

2. Composition selon la revendication 1,
où la gomme arabique est à base naturelle et/ou d'origine naturelle, en particulier d'origine végétale; et/ou
où la gomme arabique est à base d'un exsudat végétal et/ou est dérivée d'un exsudat végétal, en particulier d'un exsudat végétal d'acacia, en particulier d'arbre de gomme arabique (Senegalia *senegal*); et/ou
où la gomme arabique est dérivée et/ou provient d'acacia, en particulier d'arbre de gomme arabique (Senegalia *senegal*); et/ou
où la gomme arabique est dérivée ou est à base d'un exsudat d'acacia, en particulier d'arbre de gomme arabique (Senegalia senegal).

3. Composition selon la revendication 1 ou la revendication 2,
où la teneur en glycoprotéines de la gomme arabique est contrôlée et/ou ajustée par sélection de la gomme arabique, en particulier par sélection du matériau (végétal) utilisé pour l'obtention de la gomme arabique, de préférence à base d'un exsudat, éventuellement avec enrichissement supplémentaire, en particulier par fractionnement,
en particulier où le matériau (végétal) est sélectionné dans le groupe d'acacia, en particulier d'arbre de gomme arabique (Senegalia *senegal*); et/ou
où la teneur en glycoprotéines de la gomme arabique est contrôlée et/ou ajustée par enrichissement, en particulier par fractionnement.

4. Composition selon l'une quelconque des revendications précédentes,
où la teneur en glycoprotéines est fournie et/ou formée par des molécules, en particulier des macromolécules, qui comprennent au moins une protéine et/ou un résidu de protéine et/ou groupe de protéine avec un ou plusieurs groupes de glucides liés à ceux-ci, en particulier liés de manière covalente; en particulier où les groupes de glucides sont sélectionnés parmi des mono-, di-, oligo- et/ou polysaccharides, de préférence parmi des groupes oligosaccharidiques et en particulier polysaccharidiques; et/ou
où la teneur en glycoprotéines est fournie et/ou formée par des glycoprotéines ayant un poids moléculaire moyen en poids M̅_{w} dans l'intervalle de 10⁴ g/mol à 10⁷ g/mol, en particulier dans l'intervalle de 1 x 10⁵ g/mol à 9 x 10⁶ g/mol, de préférence dans l'intervalle de 1,5 x 10⁵ g/mol à 6 x 10⁶ g/mol; et/ou
où la teneur en glycoprotéines est fournie et/ou formée au moins partiellement, en particulier principalement, par des protéines d'arabinogalactane; et/ou
où la teneur en glycoprotéines est fournie et/ou formée à au moins 50 %, en particulier à au moins 60 %, de préférence à au moins 65 %, par des protéines d'arabinogalactane, par rapport à la teneur en glycoprotéines; et/ou
où la teneur en glycoprotéines est fournie et/ou formée à 50 % à 99 %, en particulier à 60 % à 98 %, de préférence à 65 % à 95 %, par des protéines d'arabinogalactane, par rapport à la teneur en glycoprotéines.

5. Composition selon l'une quelconque des revendications précédentes,
où la gomme arabique contient, en particulier comme composant principal, au moins un polysaccharide, de préférence au moins un polysaccharide comprenant de préférence un/des sel(s) alcalin(s) et/ou alcalino-terreux acide(s) de l'acide (poly)arabinique, de manière particulièrement préférée au moins un polysaccharide de préférence ramifié de L-arabinose, D-galactose, L-rhamnose et acide D-glucuronique; et/ou
où la gomme arabique présente une teneur en polysaccharides, par rapport à la gomme arabique, dans l'intervalle de 75 % en poids à 96 % en poids; et/ou
où la gomme arabique présente une teneur en polysaccharides, par rapport à la gomme arabique, d'au moins 75 % en poids, et/ou
où la gomme arabique présente une teneur en polysaccharides, par rapport à la gomme arabique, d'au plus 96 % en poids; et/ou
où la gomme arabique présente une teneur en polysaccharides, par rapport à la gomme arabique, dans l'intervalle de 78 % en poids à 95,5 % en poids, en particulier dans l'intervalle de 80 % en poids à 95 % en poids, de préférence dans l'intervalle de 82 % en poids à 94,5 % en poids, de manière particulièrement préférée dans l'intervalle de 84 % en poids à 94 % en poids; et/ou
où la gomme arabique présente une teneur en polysaccharides, par rapport à la gomme arabique, d'au moins 78 % en poids, en particulier d'au moins 80 % en poids, de préférence d'au moins 82 % en poids, de manière particulièrement préférée d'au moins 84 % en poids, et/ou
où la gomme arabique présente une teneur en polysaccharides, par rapport à la gomme arabique, d'au plus 95,5 % en poids, en particulier d'au plus 95 % en poids, de préférence d'au plus 94,5 % en poids, de manière particulièrement préférée d'au plus 94 % en poids.

6. Composition selon l'une quelconque des revendications précédentes,
où le triglycéride à chaîne moyenne (MCT) est un triglycéride d'un acide carboxylique en C₈, en particulier d'un acide carboxylique saturé linéaire en C₈; et/ou
où le triglycéride à chaîne moyenne (MCT) est la tricapryline; et/ou
où le triglycéride à chaîne moyenne (MCT) est un triglycéride mixte d'acide carboxylique en C₈ et C₁₀, en particulier un triglycéride mixte d'acide carboxylique saturé linéaire en C₈ et C₁₀;
en particulier où le triglycéride à chaîne moyenne (MCT) présente un rapport de teneur en C₈ : C₁₀ dans l'intervalle de 30 % - 90 % : 70 % - 10 %, en particulier dans l'intervalle de 50 % - 80 % : 50 % - 20 %, de préférence dans l'intervalle de 55 % - 75 % : 45 % - 25 %.

7. Composition selon l'une quelconque des revendications précédentes,
où la composition présente un indice de corps cétoniques (indice cétogène), calculé en tant que nombre théorique de millimoles (mmol) d'acide 3-hydroxybutyrique (3-BHB) par un gramme de la composition, d'au moins 0,5 mmol/g; et/ou
où la composition présente un indice de corps cétoniques (indice cétogène), calculé en tant que nombre théorique de millimoles (mmol) d'acide 3-hydroxybutyrique (3-BHB) par un gramme de la composition, d'au moins 1 mmol/g, en particulier d'au moins 2 mmol/g, de préférence d'au moins 3 mmol/g, de manière particulièrement préférée d'au moins 4 mmol/g; et/ou
où la composition présente un indice de corps cétoniques (indice cétogène), calculé en tant que nombre théorique de millimoles (mmol) d'acide 3-hydroxybutyrique (3-BHB) par un gramme de la composition, dans l'intervalle de 0,5 mmol/g à 50 mmol/g; et/ou
où la composition présente un indice de corps cétoniques (indice cétogène), calculé en tant que nombre théorique de millimoles (mmol) d'acide 3-hydroxybutyrique (3-BHB) par un gramme de la composition, dans l'intervalle de 1 mmol/g à 45 mmol/g, en particulier dans l'intervalle de 2 mmol/g à 40 mmol/g, de préférence dans l'intervalle de 3 mmol/g à 30 mmol/g, de manière particulièrement préférée dans l'intervalle de 4 mmol/g à 25 mmol/g.

8. Composition selon l'une quelconque des revendications précédentes,
où la composition contient au moins un émulsifiant;
en particulier où la composition contient l'émulsifiant en une quantité d'au moins 0,0001 % en poids, en particulier d'au moins 0,001 % en poids, de préférence d'au moins 0,01 % en poids, par rapport à la composition; et/ou
en particulier où la composition contient l'émulsifiant en une quantité d'au plus 5 % en poids, en particulier d'au plus 2,5 % en poids, de préférence d'au plus 1 % en poids, par rapport à la composition; et/ou
en particulier où la composition contient l'émulsifiant en une quantité dans l'intervalle de 0,0001 % en poids à 5 % en poids, en particulier dans l'intervalle de 0,001 % en poids à 2,5 % en poids, de préférence dans l'intervalle de 0,01 % en poids à 1 % en poids, par rapport à la composition.

9. Composition selon la revendication 8,
où l'émulsifiant est un émulsifiant amphotère, anionique, cationique ou non ionique; et/ou
où l'émulsifiant est sélectionné dans le groupe de glycérides d'acides gras, en particulier de mono- et diglycérides d'acides gras, et leurs sels; d'esters d'acide citrique de glycérides d'acides gras, en particulier d'esters d'acide citrique de mono- et diglycérides d'acides gras, et leurs sels; d'esters d'acide tartrique et d'esters d'acide mono- et diacétyltartrique de glycérides d'acides gras, en particulier d'esters d'acide tartrique et d'esters d'acide diacétyltartrique de mono- et diglycérides d'acides gras, et leurs sels; d'esters d'acide acétique de glycérides d'acides gras, en particulier d'esters d'acide acétique de mono- et diglycérides d'acides gras, et leurs sels; d'esters d'acide lactique de glycérides d'acides gras, en particulier d'esters d'acide lactique de mono- et diglycérides d'acides gras, et leurs sels; d'esters d'acide acétique et tartrique mixtes de glycérides d'acides gras, en particulier d'esters d'acide acétique et tartrique mixtes de mono- et diglycérides d'acides gras, et leurs sels; d'esters d'acide gras de polyglycérol, en particulier de mono-, di- et poly-esters d'acide gras de polyglycérol, de préférence de polyricinoléate de polyglycérol; d'esters de sorbitane, en particulier d'esters de sorbitane de polyoxyéthylène, de préférence de monoesters de sorbitane de polyoxyéthylène, de manière particulièrement préférée d'acides gras, en particulier de monolaurate de sorbitane de polyoxyéthylène, de monooléate de sorbitane de polyoxyéthylène, de monopalmitate de sorbitane de polyoxyéthylène, de monostéarate de sorbitane de polyoxyéthylène et de tristéarate de sorbitane de polyoxyéthylène; d'alcools gras éthoxylés; de glycérides d'acides gras éthoxylés, en particulier de mono- et diglycérides d'acides gras éthoxylés, et leurs sels; d'alkylpolyglucosides et de glycérides de sucre, en particulier de cétéarylglucosides; d'esters de sucre d'acides gras, en particulier d'esters de saccharose d'acides gras; d'amidons modifiés; de celluloses modifiées; de composés d'ammonium quaternaires (ester-quats); de lécithines; de phospholipides; de sesquistéarates d'alkylglucose, en particulier de sesquistéarate de méthylglucose; d'alcools gras; d'acides gras et leurs sels et esters; de siloxanes et de silicones; de diméthicones et de diméthicones modifiés, en particulier de PEG/PPG-diméthicones; d'alkylsarcosinates de métaux alcalins, en particulier d'alkylsarcosinates de sodium, de préférence de laurylsarcosinates de métaux alcalins; d'esters d'acyle d'acides aminés et leurs sels; de copolymères de POE/PPG; d'esters de phosphate (esters d'acide phosphorique) et leurs sels, en particulier de phosphates de cétyle, de préférence de phosphates de cétyle de métaux alcalins, de préférence de phosphate de cétyle de potassium; de polyacrylates; d'huiles de ricin et d'huiles de ricin modifiées, en particulier d'huiles de ricin et d'esters d'acide gras et d'esters d'acide gras d'huiles de ricin; d'alkylsulfates éthoxylés; ainsi que leurs combinaisons et mélanges; et/ou
où l'émulsifiant est lié et/ou fixé aux particules; et/ou où la composition contient l'émulsifiant sous forme liée et/ou fixée aux particules; et/ou
où l'émulsifiant se présente sous forme de particules et/ou sous forme de particules d'émulsifiant; et/ou
où la composition contient l'émulsifiant sous forme de particules d'émulsifiant séparées, en particulier non liées et/ou libres.

10. Composition selon l'une quelconque des revendications précédentes,
où la composition est apte à s'écouler et/ou coulante; et/ou
où la composition est au moins essentiellement exempte d'agglomérats; et/ou
où la composition est au moins essentiellement exempte d'agglomérats ou d'agrégats de particules, en particulier de macroagglomérats de particules, ayant des tailles supérieures à 5 mm, en particulier supérieures à 4,5 mm, de préférence supérieures à 4 mm, de manière particulièrement préférée supérieures à 3,5 mm; et/ou
où la composition présente une teneur en humidité résiduelle d'au plus 5 % en poids, en particulier d'au plus 4 % en poids, de préférence d'au plus 3 % en poids, par rapport à la composition; et/ou
où la composition présente une teneur en humidité résiduelle d'au moins 0,1 % en poids, en particulier d'au moins 0,25 % en poids, de préférence d'au moins 0,5 % en poids, par rapport à la composition; et/ou
où la composition présente une teneur en humidité résiduelle dans l'intervalle de 0,1 % en poids à 5 % en poids, en particulier dans l'intervalle de 0,25 % en poids à 4 % en poids, de préférence dans l'intervalle de 0,5 % en poids à 3 % en poids, par rapport à la composition; et/ou
où les particules de la composition présentent une taille de particule D90, en particulier un diamètre de particule D90, dans l'intervalle de 0,01 mm à 5 mm, en particulier dans l'intervalle de 0,05 mm à 2 mm, de préférence dans l'intervalle de 0,1 mm à 1 mm, de manière particulièrement préférée dans l'intervalle de 0,2 mm à 0,8 mm; en particulier déterminée par diffraction de lumière, de préférence déterminée par diffractométrie laser selon la norme ISO 13320:2020; et/ou
où les particules de la composition présentent une taille de particule D50, en particulier un diamètre de particule D50, dans l'intervalle de 0,01 mm à 3 mm, en particulier dans l'intervalle de 0,05 mm à 2 mm, de préférence dans l'intervalle de 0,1 mm à 1 mm, de manière particulièrement préférée dans l'intervalle de 0,15 mm à 0,75 mm; en particulier déterminée par diffraction de lumière, de préférence déterminée par diffractométrie laser selon la norme ISO 13320:2020.

11. Produit alimentaire, cosmétique ou pharmaceutique, contenant une composition selon l'une quelconque des revendications 1 à 10.

12. Utilisation d'une composition selon l'une quelconque des revendications 1 à 10 dans un produit alimentaire, cosmétique ou pharmaceutique et/ou pour la production d'un produit alimentaire, cosmétique ou pharmaceutique.

13. Procédé de production d'une composition pulvérulente contenant des triglycérides à chaîne moyenne (MCT) selon l'une quelconque des revendications 1 à 10,
où le procédé comprend les étapes de procédé suivantes, en particulier dans l'ordre indiqué ci-après des étapes de procédé:
(i) préparation et/ou production d'une dispersion homogène de préférence aqueuse, en particulier d'une émulsion homogène de préférence aqueuse, qui contient de la gomme arabique, en particulier comme matériau de support à base de polysaccharide, d'une part, et au moins un triglycéride à chaîne moyenne (MCT), en particulier au moins un triglycéride d'au moins un acide carboxylique saturé linéaire en C₆, C₈, C₁₀ ou C₁₂, d'autre part, dans un milieu de support liquide de préférence aqueux,
où une gomme arabique avec une teneur en glycoprotéines, par rapport à la gomme arabique, dans l'intervalle de 4 % en poids à 25 % en poids est utilisée et où le triglycéride est utilisé en une quantité dans l'intervalle de 50 % en poids à 90 % en poids, par rapport au poids sec de la dispersion, en particulier de l'émulsion; ensuite
(ii) séchage, en particulier séchage par pulvérisation, de la dispersion obtenue à l'étape de procédé (i), en particulier de l'émulsion, avec élimination au moins essentiellement complète du milieu de support liquide de préférence aqueux, de sorte qu'il en résulte une composition pulvérulente contenant des triglycérides à chaîne moyenne (MCT) selon l'une quelconque des revendications 1 à 10.

14. Procédé de contrôle et/ou d'ajustement de la charge, en particulier de la quantité de charge, d'un matériau de support à base de polysaccharide comprenant de la gomme arabique ou formé à partir de celle-ci avec au moins un triglycéride à chaîne moyenne (MCT), en particulier avec au moins un triglycéride d'au moins un acide carboxylique saturé linéaire en C₆, C₈, C₁₀ ou C₁₂, en particulier pour la production d'une composition particulaire et/ou pulvérulente contenant des triglycérides à chaîne moyenne (MCT), de préférence pour la production d'une composition selon l'une quelconque des revendications 1 à 10,
où la charge, en particulier la quantité de charge, est contrôlée et/ou ajustée en fonction de la teneur en glycoprotéines du matériau de support à base de polysaccharide,
où la gomme arabique présente une teneur en glycoprotéines, par rapport à la gomme arabique, dans l'intervalle de 4 % en poids à 25 % en poids, où la teneur en glycoprotéines se rapporte à la teneur totale de toutes les glycoprotéines présentes dans la gomme arabique, et
où la charge, en particulier la quantité de charge, de triglycéride(s) se situe dans l'intervalle de 50 % en poids à 90 % en poids, par rapport à la totalité du matériau de support à base de polysaccharide et du/des triglycéride(s), en particulier par rapport à la composition.

15. Utilisation de la teneur en glycoprotéines d'un matériau de support à base de polysaccharide comprenant de la gomme arabique ou formé à partir de celle-ci pour le contrôle et/ou l'ajustement de la charge, en particulier de la quantité de charge, du matériau de support à base de polysaccharide avec au moins un triglycéride à chaîne moyenne (MCT), en particulier avec au moins un triglycéride d'au moins un acide carboxylique saturé linéaire en C₆, C₈, C₁₀ ou C₁₂, en particulier aux fins de la production d'une composition particulaire et/ou pulvérulente contenant des triglycérides à chaîne moyenne (MCT), de préférence aux fins de la production d'une composition selon l'une quelconque des revendications 1 à 10,
où la gomme arabique présente une teneur en glycoprotéines, par rapport à la gomme arabique, dans l'intervalle de 4 % en poids à 25 % en poids, où la teneur en glycoprotéines se rapporte à la teneur totale de toutes les glycoprotéines présentes dans la gomme arabique, et
où la charge, en particulier la quantité de charge, de triglycéride(s) se situe dans l'intervalle de 50 % en poids à 90 % en poids, par rapport à la totalité du matériau de support à base de polysaccharide et du/des triglycéride(s), en particulier par rapport à la composition.
